# EUROPEAN PATENT APPLICATION

(11) **EP 4 708 327 A2**
(43) Date of publication of application: **11.03.2026**
(21) Application number: 25217439.6
(22) Date of filing: 30.03.2021
(51) Int. Cl.: G16H 80/00

(54) **SYSTEMS AND METHODS OF INTEGRATING MEDICAL DEVICE CASE FILES WITH CORRESPONDING PATIENT CARE RECORDS**

(30) Priority: 31.03.2020 US 202063002821 P
(62) Divisional of application: 21722603.4
(71) Applicant: ZOLL Medical Corporation, Chelmsford, MA 01824-4105 (US)
(72) Inventor: HART, Aaron R., Arvada, 80002 (US); GAFFIELD, David J., Broomfield, 80020 (US)
(74) Representative: Potter Clarkson

(57) **Abstract**

A system for integrating medical device case files with corresponding electronic patient care records (ePCRs) is provided. The system includes a database storing a plurality of medical device case files and at least one processor. The at least one processor receives, from a patient charting device, charting data documented in an ePCR for an encounter with a particular patient, generates a search criterion based at least in part on the charting data, the search criterion including a predetermined relationship between at least one first parameter from the charting data and at least one second parameter associated with a medical device case file, identifies the medical device case file based on the generated search criterion, and generates an integrated patient encounter record including at least a portion of medical device data from the medical device case file and the charting data.

## Description

### CROSS-REFERENCE TO RELATED APPLICATIONS

This application claims the benefit of U.S. Provisional Patent Application 63/002,821 (filed 31 March 2020). The entire disclosure of this related application is hereby incorporated herein by reference in its entirety.

### BACKGROUND

The present disclosure is directed to systems and methods for documenting medical care of a patient.

Electronic medical record systems enable medical and/or emergency care providers to document patient encounters. For example, an emergency medical services (EMS) crew may document a patient encounter via an electronic patient care record (PCR). The electronic PCR may include data collected from a caregiver via a computer implemented user interface provided at an endpoint device. Accurate and comprehensive documentation in the electronic PCR may improve, for example, patient care during the encounter, the continuity of care between EMS and hospitals, and overall patient health outcomes. For example, protocol adherence required by the electronic PCR may improve the care during the encounter. Continuity of care may benefit from a record that thoroughly describes symptoms, physiological metrics, and treatments provided. Other post-encounter services that may benefit from a high-quality electronic PCR include, for example, rehabilitative care, insurance reimbursement and claims, quality assurance/quality improvement processes, etc.

Medical device case files are a helpful byproduct of providing patient care with computerized medical devices. Medical device case files can document medical device operation during a patient encounter, treatments delivered during the patient encounter, and values of patient parameters measured by the medical device during the patient encounter. Medical device case files may be reviewed by healthcare providers during or after patient encounters to gain insight into patient ailments and improve patient outcomes.

### SUMMARY

In at least one example, a medical records server system for integrating medical device case files with electronic patient care records is provided. The system includes at least one database storing a plurality of medical device case files from a plurality of medical devices including medical device data recorded during patient encounters; a network interface; and at least one processor coupled to the at least one database and the network interface. The at least one processor is configured to receive, from a patient charting device via the network interface, charting data documented in an electronic patient care record (ePCR) for an encounter with a particular patient, generate a search criterion based at least in part on the charting data to identify a medical device case file for the encounter with the particular patient, the search criterion including a predetermined relationship between at least one first parameter from the charting data and at least one second parameter associated with the medical device case file, search for the medical device case file using the generated search criterion, identify the medical device case file based on the generated search criterion, generate an integrated patient encounter record including at least a portion of the medical device data from the medical device case file and the charting data from the ePCR, and store the integrated patient encounter record in the at least one database.

In the system, to receive the charting data can include to receive the ePCR and to generate the integrated patient encounter record can include to import the at least the portion of the medical device data into the ePCR to generate a supplemented ePCR, and store the supplemented ePCR in the at least one database. The at least one processor can be configured to transmit the supplemented ePCR to the patient charting device. The at least one processor can be configured to transmit the at least the portion of the medical device data to the patient charting device via the network interface. The at least one processor can be configured to transmit at least a portion of the charting data to a medical device via the network interface.

In the system, the network interface can be configured to communicate with the patient charting device and a medical device via one or more of a cellular connection and a transmission control protocol/internet protocol connection. The at least one database can include a cloud database associated with multiple tenants. The at least one processor can be configured to transmit the integrated patient encounter record to one or more of a medical facility computing device and a health records database via the network interface. The network interface can be configured to communicatively couple to the patient charting device via one or more of a computer network and a cellular network. The network interface can be configured to communicatively couple to the plurality of medical devices via one or more of a computer network and a cellular network. The system can be configured to receive the plurality of medical device case files from the plurality of medical devices via the network interface. The system can be located remotely from the patient charting device and the plurality of medical devices.

In the system, the at least one processor can be configured to transmit, to the patient charting device via the network interface, an identifier of the medical device case file; receive, via the network interface from the patient charting device, a confirmation of the medical device case file; and store the integrated patient encounter record in response to the received confirmation. The at least one second parameter can include medical device data stored in the medical device case file. The at least one second parameter can include metadata for the medical device case file. The medical device case file can be a defibrillator case file. The at least one first parameter and the at least one second parameter can be a same parameter and the predetermined relationship can include a match between a value of the at least one first parameter and a value of the at least one second parameter. The at least one first parameter and the at least one second parameter can include one or more of a case start time, patient identification information, and a medical device identifier. The patient identification information can include biometric information. The biometric information can include one or more of facial recognition information, fingerprint information, and retinal scan information. The medical device identifier can be one or more of an identifier manually entered into the ePCR, an identifier electronically transmitted to the patient charting device, and an identifier scanned from a bar code or quick response (QR) code associated with a medical device.

In the system, the predetermined relationship can include a match between two or more first parameters from the charting data and two or more second parameters associated with the medical device case file, and the two or more first parameters and the two or more second parameters can be same parameters. The two or more first parameters and the two or more second parameters can include two or more of a case start time, patient identification information, a medical device identifier, a time-of-transfer, healthcare provider identification information, patient demographic information, and patient medical information. The two or more first parameters and the two or more second parameters can include the patient medical information and one or more of the case start time, the patient identification information, and the medical device identifier. The patient medical information can include one or more physiological measurements from the particular patient. The one or more physiological measurements can include one or more of blood pressure, body temperature, respiratory rate, heart rate, and an ECG parameter.

In the system, the predetermined relationship can include one or more of: an overlapping range between a case start time and a case end time from the medical device case file and from the ePCR, and a match between a medical device identifier from the medical device case file and from the ePCR and one or more of: a match between a patient biometric identifier from the medical device case file and from the ePCR, and a match between a healthcare provider biometric identifier from the medical device case file and from the ePCR. The predetermined relationship can include the at least one second parameter from the medical device data falling within a range associated with the at least one first parameter from the charting data. The predetermined relationship can include one or more of a case start time and a case end time included in the medical device case file that are within a target time range defined based on case start and end times included in the charting data, wherein the case start and end times included in the charting data are the at least one first parameter and wherein the case start time and case end time included in the medical device case file are the at least one second parameter. The predetermined relationship can include (a) a match between a medical device identifier included in the charting data and a medical device identifier included in the medical device case file, and (b) one or more of a case start time and a case end time included in the medical device case file that are within a target time range defined based on case start and end times included in the charting data, wherein the medical device identifier and case start and end times included in the charting data are the at least one first parameter, and wherein the medical device identifier included in the medical device case file and the case start time and case end time included in the medical device case file are the at least one second parameter. The at least one first parameter and the at least one second parameter can include a case start time and the range associated with the first parameter can include a range of case start times. The range of case start times can be 0 to 10 minutes. The at least one first parameter and the at least one second parameter can include a case start time and the range associated with the at least one first parameter including a time span between the case start time from the charting data and a transfer-of-care time from the charting data.

In the system, to search for the medical device case file can include to repeat the search until a single medical device case file is identified or until a search iteration threshold is exceeded. The generated search criterion can include an initial search criterion and the predetermined relationship can include an initial predetermined relationship, and to identify the medical device case file can include to: identify multiple candidate medical device case files based on the initial search criterion; and identify the medical device case file from the multiple candidate medical device case files based on supplemental selection information. The at least one processor can be configured to: transmit, to the patient charting device via the network interface, identifiers of the multiple candidate medical device case files; and receive, via the network interface from the patient charting device, the supplemental selection information including a user selection of the medical device case file for the encounter with the particular patient, and to store the integrated patient encounter record can include to store the integrated patient encounter record in response to the received confirmation. The identifiers for the multiple candidate medical device case files can include file association indicators for each medical device case file. The identifiers of the multiple candidate medical device case files can include descriptive information about contents of the multiple candidate medical device case files.

In the system, the supplemental selection information can include a supplemental search criterion, and the at least one processor can be configured to: search for the medical device case file amongst the multiple candidate medical device case files using the supplemental search criterion; and automatically identify the medical device case file from the multiple candidate medical device case files based on the supplemental search criterion. The supplemental search criterion can include at least one additional predetermined relationship between at least one additional parameter from the charting data and at least one additional parameter associated with the medical device case file. The first parameter and the second parameter can both be a case start time and the at least one additional parameter from the charting data and the at least one additional parameter associated with the medical device case file can include a parameter other than the case start time. The first parameter and the second parameter can be within 0-1, 0-2, 0-5, or 0-10 minutes of one another. The at least one additional parameter from the charting data and the at least one additional parameter associated with the medical device case file can include one or more of a medical device identifier, patient identification information, healthcare provider identification information, and patient demographic information. The first parameter and the second parameter can be within a time range of one another and a length of the time range can be longer if the at least one additional parameter from the charting data and the at least one additional parameter associated with the medical device case file are the medical device identifier than if the at least one additional parameter from the charting data and the at least one additional parameter associated with the medical device case file are a parameter other than the medical device identifier.

In the system, the initial predetermined relationship can include the second parameter falling within a range for the first parameter, and the supplemental search criterion can include a match between the first parameter and the second parameter. The initial predetermined relationship can include a match and the at least one first parameter from the charting data and the at least one second parameter associated with the medical device case file can include non-medical parameters, and the supplemental search criterion can include at least one medical parameter from the charting data that is medically consistent and indicative of at least one medical parameter from the medical device case file. The at least one first parameter from the charting data and the at least one second parameter from the medical device data can include one or more of a case start time, a medical device identifier, and patient identification information. The at least one medical parameter from the charting data can include one or more of a patient complaint, a medic impression, a drug administration, a vital sign, a physiological measurement, and a treatment provided to the particular patient. The at least one medical parameter from the charting data can include one or more of a symptom of chest pain, an ECG with ST elevation, a STEMI diagnosis. The at least one medical parameter from the medical device case file can include one or more of a type of physiological measurement, a value of a physiological measurement, a medical treatment, and an alarm. The medical treatment can include a defibrillation shock. The at least one medical parameter from the charting data can include a patient complaint of chest pain and the at least one medical parameter from the medical device case file can include a record of a twelve-lead electrocardiogram. The at least one medical parameter from the charting data can include aspirin or nitroglycerin administration. The at least one medical parameter from the medical device case file can include shock administration. The at least one medical parameter from the charting data can include one or more of a patient complaint of difficulty breathing and a medic impression of respiratory distress and the at least one medical parameter from the medical device case file can include a pulse oximetry scan and/or a capnography scan. The at least one medical parameter from the charting data can include administration of albuterol, steroids, or epinephrine. The at least one medical parameter from the charting data can include airway placement. The at least one medical parameter from the medical device case file can include a twelve lead ECG. The at least one medical parameter from the medical device case file can include alarms for one or more of heart rate, respiratory rate, pulse oximetry, and blood pressure.

In at least one example, a non-transitory, processor-readable storage medium having stored thereon processor-executable instructions for integrating medical device case files with electronic patient care records is provided. The processor-executable instructions are configured to cause a processor to: access at least one database storing a plurality of medical device case files from a plurality of medical devices, each case file includes medical device data recorded by a medical device during an encounter with a patient; receive, from a patient charting device via a network interface, charting data documented in an electronic patient care record (ePCR) for the encounter with the patient; generate a search criterion based at least in part on the charting data to identify the medical device case file for the encounter with the patient, the search criterion includes a predetermined relationship between at least one first parameter from the charting data and at least one second parameter associated with the medical device case file; search for the medical device case file using the generated search criterion; identify the medical device case file based on the generated search criterion; generate an integrated patient encounter record includes at least a portion of the medical device data from the medical device case file and the charting data from the ePCR, and store the integrated patient encounter record in the at least one database.

In the medium, the instructions to receive the charting data can include instructions to receive the ePCR, and the instruction to store the integrated patient encounter record can include instructions to import the at least the portion of the medical device data into the ePCR to generate a supplemented ePCR, and store the supplemented ePCR in the at least one database. The instructions can be configured to cause the processor to transmit the supplemented ePCR to the patient charting device. The instructions can be configured to cause the processor to transmit the at least the portion of the medical device data to the patient charting device via the network interface. The instructions can be configured to cause the processor to transmit at least a portion of the charting data to a medical device via the network interface. The instructions can be configured to cause the processor to communicate with the patient charting device and a medical device via one or more of a cellular connection and a transmission control protocol/internet protocol connection.

In the medium, the instructions to access the at least one database can include instructions to access a cloud database associated with multiple tenants. The instructions can be configured to cause the processor to transmit the integrated patient encounter record to one or more of a medical facility computing device and a health records database via the network interface. The instructions can be configured to cause the processor to communicatively couple to the patient charting device via the network interface and one or more of a computer network and a cellular network. The instructions can be configured to cause the processor to communicatively couple to the plurality of medical devices via the network interface and one or more of a computer network and a cellular network. The instructions can be configured to cause the processor to receive the plurality of medical device case files from the plurality of medical devices via the network interface. The instructions can be configured to cause the processor to communicate remotely with the patient charting device and the plurality of medical devices. The instructions can be configured to cause the processor to: transmit, to the patient charting device via the network interface, an identifier of the medical device case file; receive, via the network interface from the patient charting device, a confirmation of the medical device case file; and store the integrated patient encounter record in response to the received confirmation.

In the medium, the at least one second parameter can include medical device data stored in the medical device case file. The at least one second parameter can include metadata for the medical device case file. The medical device case file can be a defibrillator case file. The at least one first parameter and the at least one second parameter can be a same parameter and the predetermined relationship can include a match between a value of the at least one first parameter and a value of the at least one second parameter. The at least one first parameter and the at least one second parameter can include one or more of a case start time, patient identification information, and a medical device identifier. The patient identification information can include biometric information. The biometric information can include one or more of facial recognition information, fingerprint information, and retinal scan information. The medical device identifier can be one or more of an identifier manually entered into the ePCR, an identifier electronically transmitted to the patient charting device, and an identifier scanned from a bar code or QR code associated with a medical device. The predetermined relationship can include a match between two or more first parameters from the charting data and two or more second parameters associated with the medical device case file, and the two or more first parameters and the two or more second parameters can be same parameters. The two or more first parameters and the two or more second parameters can include two or more of a case start time, patient identification information, a medical device identifier, a time-of-transfer, healthcare provider identification information, patient demographic information, and patient medical information. The two or more first parameters and the two or more second parameters can include the patient medical information and one or more of the case start time, the patient identification information, and the medical device identifier. The patient medical information can include one or more physiological measurements from the patient. The one or more physiological measurements can include one or more of blood pressure, body temperature, respiratory rate, heart rate, and an ECG parameter.

In the medium, the predetermined relationship can include one or more of: an overlapping range between a case start time and a case end time from the medical device case file and from the ePCR, and a match between a medical device identifier from the medical device case file and from the ePCR and one or more of: a match between a patient biometric identifier from the medical device case file and from the ePCR, and a match between a healthcare provider biometric identifier from the medical device case file and from the ePCR. The predetermined relationship can include the second parameter from the medical device data falling within a range associated with the first parameter from the charting data. The processor-executable instructions can be configured to cause the processor to calculate the range. The range can be hardcoded. The first parameter and the second parameter can include a case start time and the range associated with the first parameter can include a range of case start times. The range of case start times can be 0-10 minutes. The first parameter and the second parameter can include a case start time and the range associated with the first parameter can include a time span between the case start time from the charting data and a transfer-of-care time from the charting data.

In the medium, the instructions to search for the medical device case file can include instructions to repeat the search until a single medical device case file is identified or until a search iteration threshold is exceeded. The generated search criterion can include an initial search criterion and the predetermined relationship can include an initial predetermined relationship, and the instructions to identify the medical device case file can include instructions to: identify multiple candidate medical device case files based on the initial search criterion; and identify the medical device case file from the multiple candidate medical device case files based on supplemental selection information. The instructions can be configured to cause the processor to: transmit, to the patient charting device via the network interface, identifiers of the multiple candidate medical device case files; and receive, via the network interface from the patient charting device, the supplemental selection information can include a user selection of the medical device case file for the encounter with the patient and the instructions to store the integrated patient encounter record can include instructions to store the integrated patient encounter record in response to the received confirmation.

In the medium, the identifiers for the multiple candidate medical device case files can include file association indicators for each medical device case file. The identifiers of the multiple candidate medical device case files can include descriptive information about contents of the multiple candidate medical device case files. The supplemental selection information can include a supplemental search criterion, and the processor-executable instructions can be configured to cause the processor to: search for the medical device case file amongst the multiple candidate medical device case files using the supplemental search criterion; and automatically identify the medical device case file from the multiple candidate medical device case files based on the supplemental search criterion. The supplemental search criterion can include at least one additional predetermined relationship between at least one additional parameter from the charting data and at least one additional parameter associated with the medical device case file. The first parameter and the second parameter can both be a case start time and the at least one additional parameter from the charting data and the at least one additional parameter associated with the medical device case file can include a parameter other than the case start time. The first parameter and the second parameter can be within 0 to 1, 0 to 2, 0 to 5, or 0 to 10 minutes of one another.

In the medium, the at least one additional parameter from the charting data and the at least one additional parameter associated with the medical device case file can include one or more of a medical device identifier, patient identification information, healthcare provider identification information, and patient demographic information. The first parameter and the second parameter can be within a time range of one another and a length of the time range can be longer if the at least one additional parameter from the charting data and the at least one additional parameter associated with the medical device case file are the medical device identifier than if the at least one additional parameter from the charting data and the at least one additional parameter associated with the medical device case file are a parameter other than the medical device identifier. The initial predetermined relationship can include the second parameter falling within a range for the first parameter, and the supplemental search criterion can include a match between the first parameter and the second parameter.

In the medium, the initial predetermined relationship can include a match and the at least one first parameter from the charting data and the at least one second parameter associated with the medical device case file can include non-medical parameters, the supplemental search criterion can include at least one medical parameter from the charting data that is medically consistent and indicative of at least one medical parameter from the medical device case file. The at least one first parameter from the charting data and the at least one second parameter from the medical device data can include one or more of a case start time, a medical device identifier, and patient identification information. The at least one medical parameter from the charting data can include one or more of a patient complaint, a medic impression, a drug administration, a vital sign, a physiological measurement, and a treatment provided to the patient. The at least one medical parameter from the charting data can include one or more of a symptom of chest pain, an ECG with ST elevation, a STEMI diagnosis. The at least one medical parameter from the medical device case file can include one or more of a type of physiological measurement, a value of a physiological measurement, a medical treatment, and an alarm. The medical treatment can include a defibrillation shock. The at least one medical parameter from the charting data can include a patient complaint of chest pain and the at least one medical parameter from the medical device case file can include a record of a twelve-lead electrocardiogram. The at least one medical parameter from the charting data can include aspirin or nitroglycerin administration. The at least one medical parameter from the medical device case file can include shock administration. The at least one medical parameter from the charting data can include one or more of a patient complaint of difficulty breathing and a medic impression of respiratory distress and the at least one medical parameter from the medical device case file can include a pulse oximetry scan and/or a capnography scan. The at least one medical parameter from the charting data can include administration of albuterol, steroids, or epinephrine. The at least one medical parameter from the charting data can include airway placement. The at least one medical parameter from the medical device case file can include a twelve lead ECG. The at least one medical parameter from the medical device case file can include alarms for one or more of heart rate, respiratory rate, pulse oximetry, and blood pressure.

In at least one example, a patient charting device for creating an integrated medical device and patient care record is provided. The patient charting device includes a user interface; a communications interface; and a processor, memory, and associated circuitry communicatively coupled with the user interface and the communications interface. The processor is configured to receive, via the user interface during a patient encounter, input specifying charting data, transmit, via the communications interface, the charting data to a server, receive, via the communications interface from the server, at least one identifier of at least one medical device case file associated with the charting data, render, via the user interface, at least one prompt to confirm that the at least one medical device case file was generated during the patient encounter, receive, via the user interface, input confirming that the at least one medical device case file was generated during the patient encounter, and transmit, via the communications interface, a confirmation specifying that the at least one medical device case file was generated during the patient encounter.

In the patient charting device, the processor, memory, and associated circuitry can be further configured to render prompts to capture the charting data. The charting data can include all or a portion of the patient care record. The at least one identifier of the at least one medical device case file can include a single identifier of a single medical device case files and the at least one prompt to confirm can include a single prompt to confirm that the single medical device case file was generated during the patient encounter. The at least one identifier of the at least one medical device case file can include a plurality of identifiers of a plurality of medical device case files and the at least one prompt can include one or more user interface controls configured to receive a selection of a single medical device case file of the plurality of medical device case files to confirm the single medical device case file was generated during the patient encounter. Each identifier of the plurality of identifiers can be associated with a file association indicator of a plurality of file association indicators, each file association indicator of the plurality of file association indicators can indicate a degree of similarity between parameters associated with the medical device case file identified by the identifier and parameters of the charting data. The one or more user interface controls can include a plurality of user interface controls, each user interface control of the plurality of user interface controls can correspond to an identifier of the plurality of identifiers, and each user interface control can be positioned within the at least one prompt according the file association indicator associated the identifier corresponding to the user interface control. To receive the at least one identifier can include to receive the plurality of file association indicators. The processor, memory, and associated circuitry can be further configured to determine the plurality of file association indicators. Each user interface control can be labeled with the file association indicator associated the identifier corresponding to the user interface control. Each user interface control can be labeled with case data from the medical device case file identified by the identifier corresponding the user interface control, the case data can include one or more of case start time, medical device identifier, time-to-transfer, healthcare provider identifier, patient identifier, patient demographic information, patient medical information, and physiological measurements. The at least one prompt can include a user interface control configured to receive input refusing to confirm that the at least one medical device case file was generated during the patient encounter.

In the patient charting device, the processor, memory, and associated circuitry can be further configured to: receive the at least one medical device case file; parse the at least one medical device case file to retrieve case data; and import the case data into the patient care record to generate an integrated patient encounter record. The processor, memory, and associated circuitry can be further configured to store the integrated patient encounter record locally and/or transmit the integrated patient encounter record to a multi-tenant cloud server. The user interface can include one or more of a touch screen configured to receive tactile input, a microphone configured to receive audio input, and wearable glasses configured to receive gestural input. The patient charting device can be a tablet, a medical device, a watch, or another computing device. The communications interface can support wired and/or wireless connections. The processor, memory, and associated circuitry can be further configured to: receive a medical device identifier via a camera, a radio-frequency identification sensor, and/or a near-field communication sensor; import the medical device identifier into the patient care record; transmit the patient care record including the medical device identifier to the server; and receive, from the server, a medical device case file generated by a medical device identified by the medical device identifier.

In at least one example, a medical records server system for integrating medical device case files with corresponding electronic patient care records is provided. The system includes at least one database storing a plurality of medical device case files from a plurality of medical devices includes medical device data recorded during patient encounters, wherein each medical device case file includes a first case start time and a medical device identifier; a network interface; and at least one processor coupled to the at least one database and the network interface. The at least one processor is configured to receive, from a patient charting device via the network interface, charting data documented in an electronic patient care record (ePCR) for an encounter with a particular patient, wherein each ePCR includes a second case start time and the medical device identifier, identify the medical device case file based on the first and second case start times and the medical device identifier, generate an integrated patient encounter record including at least a portion of the medical device data from the medical device case file and the charting data from the ePCR, and store the integrated patient encounter record in the at least one database.

In the system, to receive the charting data can include to receive the ePCR, and to store the integrated patient encounter record can include to: import the at least the portion of the medical device data into the ePCR to generate a supplemented ePCR, and store the supplemented ePCR in the at least one database. The at least one processor can be configured to transmit the supplemented ePCR to the patient charting device. The at least one processor can be configured to transmit the at least the portion of the medical device data to the patient charting device via the network interface. The at least one processor can be configured to transmit at least a portion of the charting data to a medical device via the network interface. The network interface can be configured to communicate with the patient charting device and the medical device via one or more of a cellular connection and a transmission control protocol/internet protocol connection. The at least one database can include a cloud database associated with multiple tenants. The at least one processor can include configured to transmit the integrated patient encounter record to one or more of a medical facility computing device and a health records database via the network interface. The network interface can be configured to communicatively couple to the patient charting device via one or more of a computer network and a cellular network. The network interface can be configured to communicatively couple to the plurality of medical devices via one or more of a computer network and a cellular network. The system can be configured to receive the plurality of medical device case files from the plurality of medical devices via the network interface.

In the system, the medical records server system can be located remotely from the patient charting device and the plurality of medical devices. The at least one processor can be configured to: transmit, to the patient charting device via the network interface, an identifier of the medical device case file; receive, via the network interface from the patient charting device, a confirmation of the medical device case file; and store the integrated patient encounter record in response to the received confirmation. The medical device case file can be a defibrillator case file. The medical device identifier can be one or more of an identifier manually entered into the ePCR, an identifier electronically transmitted to the patient charting device, and an identifier scanned from a bar code or QR code associated with a medical device. The first case start time can fall within a range associated with the second case start time. The at least one processor can be configured to calculate the range. The range can be predetermined. The range can be 0-10 minutes. The range can include a time span between the second case start time from the ePCR and a transfer-of-care time from the ePCR.

In the system, to identify the medical device case file can include to identify multiple candidate medical device case files. The at least one processor can be configured to: transmit, to the patient charting device via the network interface, identifiers of the multiple candidate medical device case files; receive, via the network interface from the patient charting device, supplemental selection information including a user selection of the medical device case file for the encounter with the particular patient; and store the integrated patient encounter record in response to the received confirmation. The identifiers for the multiple candidate medical device case files can include file association indicators. The identifiers of the multiple candidate medical device case files can include descriptive information about contents of the multiple candidate medical device case files.

### BRIEF DESCRIPTION OF THE DRAWINGS

Various aspects of the disclosure are discussed below with reference to the accompanying figures, which are not intended to be drawn to scale. The figures are included to provide an illustration and a further understanding of various examples and are incorporated in and constitute a part of this specification but are not intended to limit the scope of the disclosure. The drawings, together with the remainder of the specification, serve to explain principles and operations of the described and claimed aspects and examples. In the figures, each identical or nearly identical component that is illustrated in various figures is represented by a like numeral. For purposes of clarity, not every component may be labeled in every figure. A quantity of each component in a particular figure is an example only and other quantities of each, or any, component could be used.
FIG. 1 is a schematic block diagram of a medical records system in accordance with at least one example disclosed herein.
FIG. 2A is a flow diagram of a patient encounter file integration process executed by a file integration service 130 in accordance with at least one example disclosed herein.
FIG. 2B is a flow diagram of a patient encounter file integration process executed by a file integration service 130 in accordance with an example implementation that identifies a medical device case file that includes a medical device identifier that exactly matches a medical device identifier included in the charting data.
FIG. 2C is a flow diagram of a patient encounter file integration process executed by a file integration service 130 in accordance with an example implementation that identifies a medical device case file that includes a case start time and a case end time that are within a target time range that is defined based on case start and end times included in ePCR charting data.
FIG. 2D is a flow diagram of a patient encounter file integration process executed by a file integration service 130 in accordance with an example implementation that identifies a medical device case file that includes (a) a medical device identifier that exactly matches a medical device identifier included in the charting data, and (b) a case start time and a case end time that are within a target time range that is defined based on case start and end times included in ePCR charting data.
FIG. 3 is a flow diagram of a patient encounter file integration process executed by a file integration service 130 in accordance with at least one example disclosed herein.
FIG. 4 is a flow diagram of a patient encounter file integration process executed by a file integration service 130 in accordance with at least one example disclosed herein.
FIG. 5 is a flow diagram of an integration process executed by a medical device, a charting device, and a file integration service 130 in accordance with at least one example disclosed herein.
FIG. 6 is a flow diagram of an integration process executed by a charting device and a file integration service 130 in accordance with at least one example disclosed herein.
FIG. 7 is a flow diagram of an integration process with user confirmation executed by a charting device and a file integration service 130 in accordance with at least one example disclosed herein.
FIG. 8 is a flow diagram of a patient encounter file integration a file integration process executed by a file integration service 130 in accordance with at least one example disclosed herein.
FIG. 9 is a flow diagram of a patient encounter file integration process executed by a file integration service 130 in accordance with at least one example disclosed herein.
FIG. 10 is a flow diagram of a patient encounter file integration process executed by a file integration service 130 in accordance with at least one example disclosed herein.
FIG. 11 is a flow diagram of a patient encounter file integration process executed by a file integration service 130 in accordance with at least one example disclosed herein.
FIG. 12 is a flow diagram of a patient encounter file integration process executed by a file integration service 130 in accordance with at least one example disclosed herein.
FIG. 13 is a flow diagram of a patient encounter file integration process executed by a file integration service 130 in accordance with at least one example disclosed herein.
FIG. 14 is a schematic block diagram of examples of computing and medical device components with which at least one example disclosed herein may be implemented.
FIGS. 15A and 15B are views of user interface screens provided by an ePCR application to generate the ePCR in accordance with at least one example disclosed herein.
FIG. 16 is a view of a user interface screen provided by an ePCR application to request confirmation of one or more medical device case files in accordance with at least one example disclosed herein.

### DETAILED DESCRIPTION

Complete and accurate patient information, including patient biographical information, medical condition, medications, allergies, and the like are critical for accurate diagnoses and treatments. Complete and accurate patient information may also be provided to third parties such as healthcare providers in a hospital to enable efficient and accurate treatment and patient family members to locate the whereabouts and/or to observe the condition of the patient.

For example, consider an illustrative scenario of a crew of EMS healthcare providers in an ambulance being called upon to treat a patient suffering from an emergency medical condition (e.g., cardiac arrest, trauma, respiratory distress, drug overdose, etc.) and to transport the patient to a hospital. During the course of this emergency encounter, the EMS healthcare providers may be required to generate a patient charting record, for example, an electronic patient care record (ePCR). This ePCR can comprise charting data regarding the patient, such as observed patient symptoms during the encounter, observed patient physiological parameters (such as heart rate, ECG traces, temperature, blood-oxygen data, and the like), and treatments or medications administered during the encounter. The patient charting data can include information, such as any known allergies to medication, relevant medical history, and/or additional patient medical conditions. The patient charting data can also include patient demographic information and/or information regarding the emergency medical event, such as type of service requested, response mode, and triage classification.

Patient charting data and medical device case file data include treatments, events, observations, etc. for a single patient or victim as documented and recorded over the course of a patient encounter. The patient encounter includes interactions between the patient or victim and one or more healthcare providers and one or more medical devices for the purpose of providing healthcare services or assessing the health status of the patient. The patient encounter may include one or more medical events for the patient or victim. In an EMS context, the patient encounter may start when the healthcare provider initiates documentation of the patient encounter. For example, the healthcare provide may initiate documentation, for example, generation of an ePCR, in response to a dispatch notification from an emergency call center (e.g., a 911 center). In another example, the patient encounter may begin when the EMS healthcare provider arrives on-scene with the patient/victim. In an additional example, the patient encounter may begin with a patient treatment that may be subsequently included in documentation. For example, a lay bystander may witness a cardiac arrest and begin CPR prior to the arrival of an EMS team. The EMS documentation may include the events witnessed by the bystander and the care provided by the bystander prior to EMS arrival and these events and care may be part of the patient encounter. The patient encounter may include interactions between the patient and one or more medical devices. Multiple medical devices may treat the patient concurrently and/or sequentially. In the example of EMS, the patient encounter may terminate when the EMS provider transfers care of the patient to a medical facility at a time recorded in the patient encounter documentation and/or at a time recorded by a medical device upon disconnection of the medical device from the patient and/or a power-off event of the medical device. In an implementation, the patient charting data may include a start and stop time for the patient encounter as documented by the ePCR and/or the medical device case file(s) may include start and stop times as recorded by the medical device(s). The integrated patient encounter record generated as described herein may include times associated with the patient encounter from the ePCR and the medical device(s).

The treatment provided by healthcare providers during a patient encounter, including, for example an emergency encounter, may include treatments provided by one or more medical devices. These medical devices may be configured to record and locally store medical device case files. The medical device case file includes case data that documents significant medical and/or device events within, and other relevant information regarding the encounter. This case data can include physiological parameters of the patient acquired by sensors accessible by the medical devices during the encounter, data regarding treatment administered to patients via therapeutic devices controllable by the medical devices during the encounter, data regarding performance of the EMS healthcare providers, protected health information (PHI), and data regarding important milestones (e.g., code/event markers) within the encounter. Examples of physiological parameters stored within case data include heart rate, electrocardiogram (ECG) traces, blood pressure data, capnographic data, temperature, blood-oxygen data, and the like. Examples of treatment data stored within case data include defibrillation data, drug infusion data, chest compression data, and ventilation data. Examples of performance data stored within case data include chest compression performance data, ventilation performance data, and drug administration information, amongst others. As this illustrative scenario makes clear, a wide variety of valuable case data can be generated by a variety of medical devices that may be involved in a patient encounter.

The medical device may transmit the medical device case file to a centralized storage location (e.g., a server). For some EMS agencies, the number of medical device case files may be large even within a relatively short time window. For example, an EMS agency in a major metropolitan area may have on the order of 100 defibrillators deployed with 10-20 or more concurrent or overlapping patient encounters.

Linking the charting data included within an ePCR for a patient encounter with the case data in a medical device case file to generate an integrated patient encounter record for the same patient encounter may provide caregivers with medical information that is more beneficial than either one of these records alone. Typically, EMS transitions a patient to a hospital and the efficacy and efficiency of the hospital care may depend on the accuracy and completeness of the EMS record of the patient encounter.

However, establishing this linkage or association between an ePCR and a corresponding medical device case file, for example, based on manual selection by a user from a computer drop down menu that lists patient charting files and medical device case files, poses potential problems of timeliness, accuracy, and efficiency. Paramedics generally need to keep hands and eyes on the patient and, therefore, time spent on file management is time taken away from patient care. This may be particularly true in a mass casualty or pandemic situation. Furthermore, as paramedics need to move from one patient to their next call, establishing this linkage post-case may be forgotten or, again, may divert attention away from patient care. Additionally, the linkage between records requires extreme accuracy as extreme harm may result from linking charting data for one patient with medical device data for another patient.

In contrast, automated sorting and integration for linkage of these files may provide the advantages of saving caregiver time and improving accuracy, all of which may benefit and enhance patient care or may be critical for patient survival. In some cases, automated sorting may be combined with manual confirmation which may still improve the accuracy of the file linkage and save time. For example, in the example of the large EMS agency, automated sorting of a large number of medical device case files to provide the caregiver with a handful of choices from which to confirm linkage improves the efficiency and accuracy of the file linkage.

As a further advantage, the automated sorting and integration for linkage of these files relies on computerized access and storage of these medical files (e.g., charting and medical device case files) which in turn enables transmission of files throughout a medical care network. As an example, such transmission may enable telemedicine applications. These applications may enable a higher level of medical expertise for a given situation and/or may enable efficient care during a highly contagious pandemic. As another example, such transmission may enable sending a linked patient care record ahead of EMS to the hospital so that the hospital can better prepare for patient treatment. Such transmission may also enable sending the linked patient care record ahead to billing or insurance providers to verify coverage or other payment information.

Thus, in accordance with at least some embodiments disclosed herein, a medical records server system is provided that includes at least one database, a network interface, and one or more processors coupled to the at least one database, the network interface, and the one or more processors. The database stores medical device case files accumulated from multiple medical devices. In some cases, a medical device case file may be an integrated data source encounter structure including case file data from multiple medical device sources, as described in more detail herein. The one or more processors are configured to receive a patient charting data file (e.g., an ePCR), generate at least one search criterion based on the charting data, and search for a medical device case file that satisfies the at least one search criteria. The one or more processors are further configured to identify the medical device case file that satisfies the at least one search criteria as a result of the search, integrate the medical device case file and patient charting data file to generate a patient encounter file integration record and store the patient encounter file integration record. The file integration record may include at least a portion of medical device data from the identified case file and at least a portion of the charting data.

The server system can employ a variety of search techniques to identify associated patient charting data files and medical device case files. For instance, in some examples, the server attempts to match one or more first parameters from the charting data to a second parameter associated with the medical device case file. What constitutes a "match" may vary depending on the medical device and charting data available to the server system. For instance, in some examples, the server system searches for exact matches where the data available includes values that can be compared with the requisite precision (e.g., patient identifiers, medical device identifiers, timestamps of events expected to be recorded in synchrony by a charting device that originates the patient charting data and a medical device that originates the medical device case file, etc.). In some implementations, an exact match for values of a data parameter from the case file and the same data parameter from the patient charting record may include an equality between these data parameter from the two files. In other instances, the server system searches for relative matches where the data available includes values that must be compared in a less exacting manner (e.g., through the use of ranges or iterative searches). In still other examples, the server system can search for medical device data that is medically consistent with and indicative of charting data stored in the patient charting data file. These and other search techniques are described in detail below.

In some examples, the server system provides the healthcare provider with multiple candidate medical device case files to associate with the patient charting record, where the server system is unable to identify a single match between a charting data file and a medical device case file according to a pre-determined acceptable degree of confidence. In these examples, the server system may transmit information descriptive of the candidate files to enable the healthcare provider to select a particular medical device case file to associate with the patient charting record. Upon receipt of the healthcare provider's selection, the server generates and stores an integrated patient encounter record, as described herein.

Various examples of the server system can include a variety of data storage and transmission options. For instance, in some examples, the integrated patient encounter record is stored as a supplemental patient data charting file that includes the charting data and at least a portion of the medical device data. The database storing this information can be configured to support multiple tenants. The server can transmit the supplemental file to a charting device that originated the charting data, a computing device at a remote medical facility, and/or a health records database. In some examples, the server can transmit the charting data to the medical device that originated the medical device data. The server can, in some examples, execute these transmissions wirelessly (e.g., via a cellular network) and/or via a computer network (e.g., an Internet Protocol network). These and other examples will now be described in detail with reference to the accompanying figures.

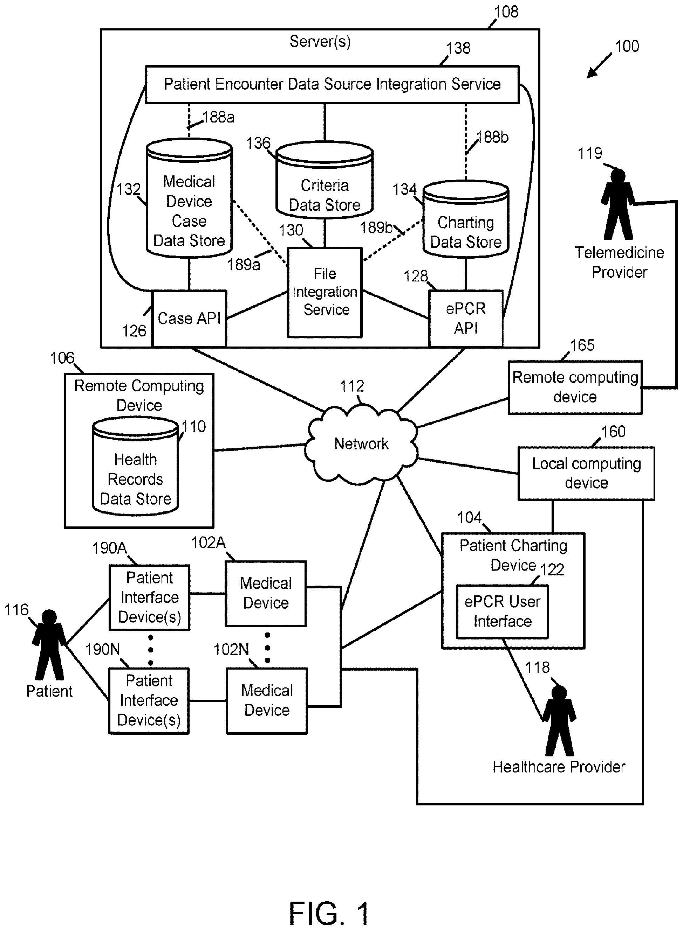

In an implementation, the medical device case file may be an integrated data source encounter structure. For example, the patient (e.g., the patient 116 in FIG. 1) may be associated with multiple medical devices (e.g., the medical devices 102A-102N in FIG. 1) and the medical device case files from these multiple devices may form an integrated data source encounter structure corresponding to a single ePCR. Example integrated data source encounter structures are described in U.S. Provisional Patent Application 63/002,678 (filed 31 March 2020) and U.S. Provisional Patent Application 63/111,234 (filed 9 November 2020), both of which are hereby incorporated herein by reference in their entirety. The integrated data source encounter structure includes medical device case files pertinent to the particular patient and the particular patient encounter that are associated or otherwise linked with one another and consolidated into a single data structure.

As an example of patient care that may generate an integrated data source encounter structure, when a victim, particularly one who is not currently in the hospital, suffers from a medical emergency such as sudden cardiac arrest, it is rarely the case that the large swath of appropriate medical devices are immediately present. In fact, it is more likely that only a bystander with very little medical experience is available to help. That bystander may have the presence of mind to find a public access AED, for example, stored in a nearby wall cabinet and to call EMS. The bystander may then apply defibrillation electrode pads to the victim and administer CPR, according to step-by-step instructions provided by the AED, examples of which may include the AED Plus^{®} or ZOLL AED 3^{®} public access AEDs, provided by ZOLL Medical Corporation. In some embodiments, the AED may collect data such as ECG waveforms of the victim, number of defibrillation shocks provided, ECG snapshots associated with the defibrillation shocks, and chest compression performance information, and may upload such information to the server(s) (e.g., the server(s) 108 with file association and integration capabilities from a file integration service 130 as shown in FIG. 1).

Once EMS arrives, the EMS healthcare providers may include in their patient charting data the events and procedures provided to the patient prior to their arrival and associated with the AED. Additionally, these EMS healthcare providers may employ a professional grade defibrillator/monitor and attach ECG electrodes (e.g., 12-lead, 3-lead), along with other sensors such as blood pressure and SpO2 sensors, for more advanced care and physiological monitoring. The more advanced defibrillator/monitor may also collect data such as, for example, heart rate, ECG waveforms of the victim, number of defibrillation shocks provided, ECG snapshots associated with the defibrillation shocks, blood pressure readings/trends, oxygen saturation readings/trends, drug infusions, chest compression and ventilation performance information, amongst others. Such information may be uploaded to the server(s) with information that enables consolidation of the medical device case files from the defibrillator/monitor with the medical device case files from the AED (e.g., the server(s) 108 may include a patient encounter data source integration service 138 as shown in FIG. 1). For example, a healthcare provider may use a mobile computing device to scan identifiers (e.g., QR codes) of the AED and the defibrillator/monitor, so as to associate the two devices and the corresponding medical device case files containing the relevant patient and treatment information with the specific emergency event.

EMS may decide that the victim requires immediate hospital care, and thus may begin transport via an ambulance that is further equipped with an automated chest compressor and ventilator unit for care on route to the hospital. The automated chest compressor may provide chest compressions according to pre-specified parameters, and the ventilator unit may monitor oxygen saturation and heart rate levels of the victim and administer automated ventilations, for example, with user assistance. The healthcare provider may further use a mobile computing device to scan identifiers (e.g., QR codes) of the automated chest compressor and the ventilator, so as to associate an additional two devices and the respective medical device case files containing patient and treatment information with this particular emergency event. The healthcare provider may further request that the mobile device link together the information generated by the four separate devices for the particular emergency victim/event, so that a server(s) configured to associate, consolidate, and integrate medical device case file data may consolidate and integrate the uploaded case files from the various medical devices used to treat the victim into an integrated data source encounter structure. The integrated data source encounter structure may then include all of the pertinent medical device case file data relating to the particular victim that was treated by the bystander and by EMS.

In addition to the scenario described above with an integrated data source encounter structure associated with EMS services, in an implementation, the medical devices (e.g., the devices 102A-102N in FIG. 1) may be associated with EMS services and additional medical devices (not shown) may be associated with services from a hospital or other medical facility as a follow-up to the EMS services. In this example, the integrated data source encounter structure may include medical device case files from the additional medical devices. For example, the system may parse the integrated data source encounter structure to isolate the EMS services portion of this structure and associate only this EMS services portion with the ePCR. This scenario may apply, for example, to a compilation of a patient's healthcare record for services provided by EMS only. This may be of benefit to EMS billing and/or EMS agency evaluations. As another example, the system may associate the integrated data source encounter structure with the ePCR. This scenario may apply, for example, to a compilation of a patient's healthcare record regardless of the source of services.

FIG. 1 illustrates a medical records system 100 that includes and implements a file integration service 130 in accordance with some examples. The system 100, according to examples of the present disclosure, can associate or integrate patient charting data files or records (e.g., electronic patient charting data records (ePCRs)) with medical device case files from one or more medical devices. In an implementation, the medical device case file may be an integrated data source encounter structure. In associating the case file with the charting data file, the file integration service 130 associates these files with one another based on their common association with the same patient during a same patient encounter with this patient. The files associated with one another were both generated during this same patient encounter and the file integration service 130 identifies this correspondence between these files. In an implementation, the file integration service 130 may generate integrated medical records of patient encounters based on the associated case file and charting data file. These integrated medical records not only document patient encounters but also provide healthcare providers with information needed to improve patient outcomes. As shown in FIG. 1, the medical records system 100 includes one or more medical device(s) 102A-102N, a patient charting device 104, a remote computing device 106, and server(s) 108 coupled to one another, for example, via a network 112. Each of the medical device(s) 102A-102N is coupled to one or more patient interface device(s) 190A-190N that are, in turn, coupled to a patient 116. For ease of reference, each of the medical device(s) 102A-102N and the patient interface device(s) 190A-190N may be referred to herein collectively as the medical devices 102 and the patient interface devices 190. Individual members of these collectives may be referred to generically as a medical device 102, a charting device 104, and patient interface devices 190.

The system 100 may further include one or more local computing device(s) 160 and/or one or more remote computing device(s) 165. The device(s) 160 and/or 165 may include a laptop computer, a mobile device (e.g., a computer tablet, smartphone, or wearable device such as a headset, glasses, or watch). The remote computing device(s) 165 may further include one or more servers. The device(s) 160 and/or 165 may provide a user interface on an integrated and/or connected user interface device (e.g., audio or visual device). The remote computing device(s) 165 may be configured for telemetry and provide telemedicine capabilities based on the patient charting data, the medical device data, and/or the associated files for these data sources. The remote computing device(s) 165 may be associated with a telemedicine provider 119. This may also facilitate participation in care by remotely located caregiver(s) 119, e.g., via telemedicine. The remote computing device(s) 165 enable a review of the patient data in real-time and/or as historical data. In a telemedicine application, the auxiliary device may be configured for telemetric reception, analysis, review, and/or transmission of the patient data. In an implementation, one or more of the local computing device(s) 160, the medical device(s) 102, and the patient charting device 104 may be proximately located to one another and communicate and/or transmit and receive charting data and/or medical device case file data via a short range wired or wireless connection. The wireless connection may include, for example, but not limited to Bluetooth^{®} and/or near-field communications.

As shown in FIG. 1, the charting device 104 includes an ePCR user interface 122 configured to interact with a healthcare provider 118. The server(s) 108 may be located remotely from the patient charting device 104 and the medical devices 102 and can host a medical device case application programming interface (API) 126, an ePCR API 128, patient encounter file integration service 130, a medical device case data store 132, a patient charting data store 134, and a criteria data store 136. The remote computing device 106 hosts a health records data store 110, which can receive and store charting and case data for a patient's medical record.

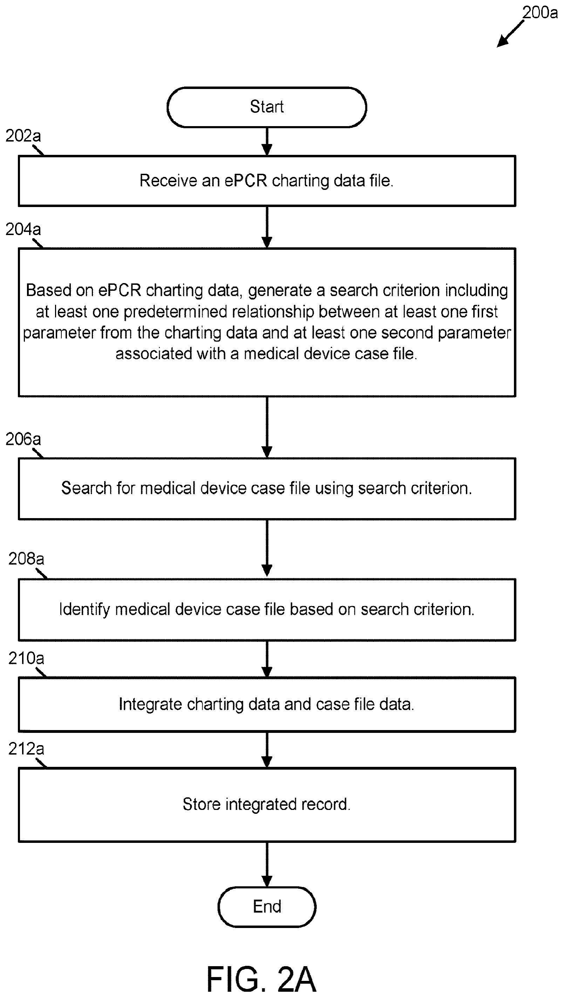

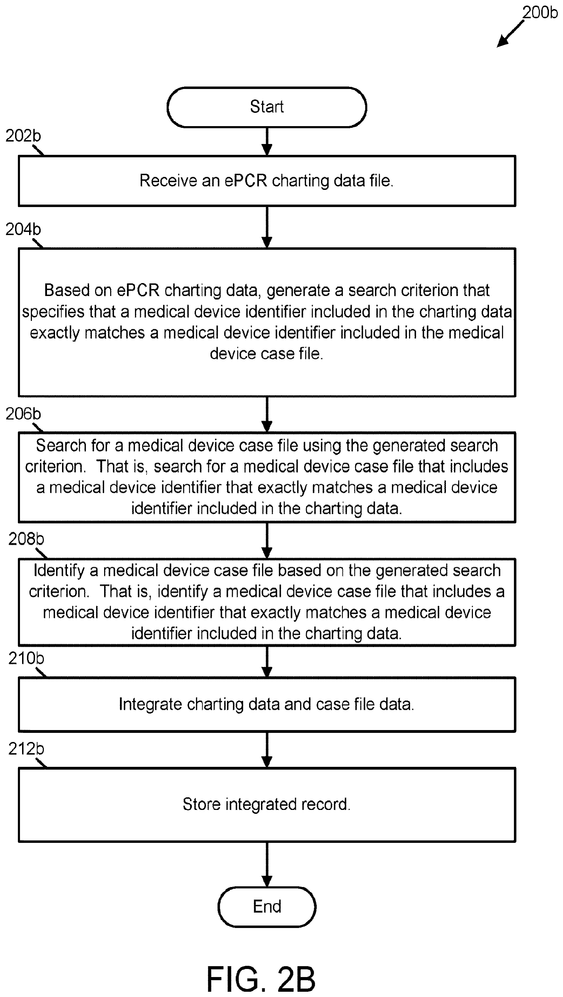

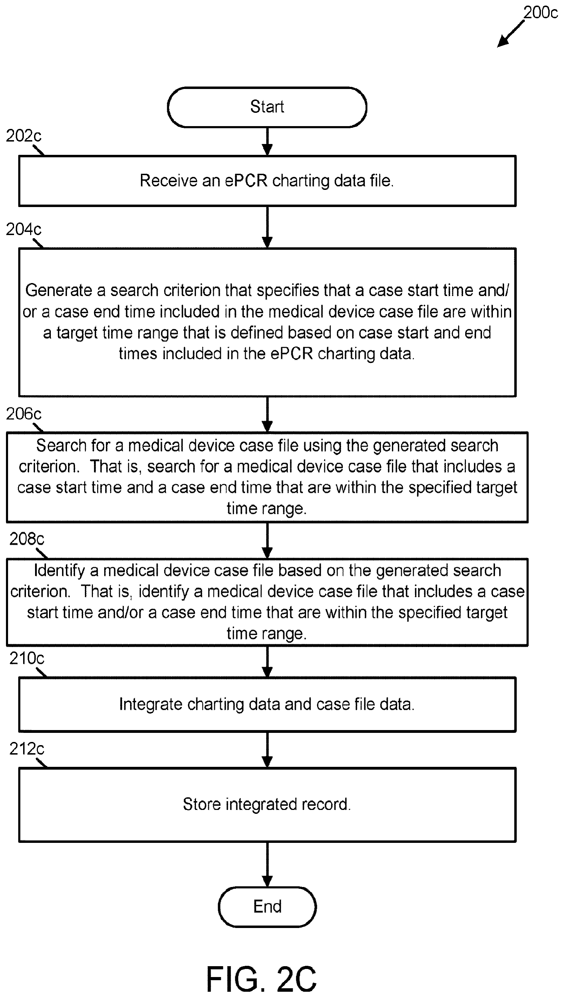

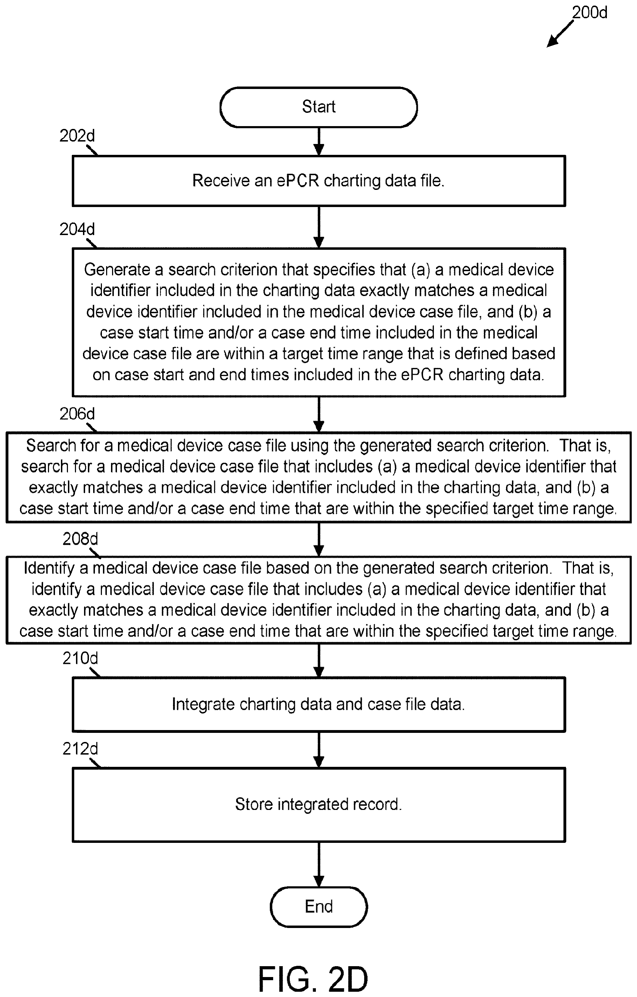

In some examples, the file integration service 130 is configured to monitor the charting data store 134 for, and/or receive messages from the ePCR API 128 descriptive of, inbound charting data received via the network 112. This inbound charting data can be, for example, sourced from one or more new or modified ePCRs generated and transmitted by the charting device 104. In these examples, to handle the inbound charting data, the file integration service 130 is further configured to execute one or more processes that attempt to identify case data within the case data store 132 that originated from a medical device case file generated during the patient encounter documented by the inbound charting data. Examples of these processes are described in detail below with reference to FIGS. 2-13. The file integration service 130 can be configured to take one or more of several actions in response to identifying case data generated by a medical device during the same patient encounter as inbound charting data in order to automatically map the medical device case file to the patient charting record for the same patient and the same patient encounter. These actions may include combining the case data with inbound charting data, storing the combined case and charting data (e.g., in the charting data store 134), transmitting messages including the combined case and charting data and/or transmitting messages including the case data or identifiers of the case data. These messages can be transmitted to the charting device 104, the medical devices 102, and/or the remote computing device 106 (e.g., for storage in the health records data store 110) via the ePCR API 128 and/or the case API 126 and the network 112. These and other actions that the file integration service 130 may be configured to take in response to identifying associated case data and charting data are also described in detail below with reference to FIGS. 2-13.

At least some of the processes described below with reference to FIGS. 2-13 advantageously leverage aspects of the charting data to identify case data. As such, to aid the reader's understanding, a description of charting data, case data, and the parts of the medical records system 100 that originate these data is provided prior to a description of the processes executed by the file integration service 130.

In some examples, the healthcare provider 118, who for example may be an EMS technician, can attach the patient interface devices 190 (e.g. sensors) coupled to the medical devices 102 to the patient 116 to monitor and/or treat the patient 116. This treatment may involve the medical devices 102 and may be documented by the healthcare provider 118 via the ePCR user interface 122 of the patient charting device 104. As illustrated in FIG. 1, in some examples the charting device 104 may be configured to communicably couple to the medical devices 102.

More specifically, in some examples, the charting device 104 may be a device used by the healthcare provider 118 to generate the ePCR and/or other records and/or notes about the condition of the patient 116 and/or treatments applied to the patient 116. The charting device 104 can include a combination of devices, according to some examples. For instance, the charting device 104 can include a processor coupled with memory configured to store data manipulated by the processor. The charting device 104 can have a clock, which can be synchronized with an external time source such as a network resource or a satellite to prevent the healthcare provider 118 from having to manually enter a time of treatment or observation (or having to attempt to estimate the time of treatment for charting purposes long after a caregiver and/or medical device administers treatment), according to examples of the present disclosure. The charting device 104 can include one or more system and/or network interfaces configured to receive and/or send data from and/or to other devices like the medical devices 102. In some examples, the charting device 104 can utilize these system and/or network interfaces to communicate with another device or system (e.g., the remote computing device 106 and/or the server(s) 108) that aggregates or otherwise receives data from other devices, such as the medical devices 102. Alternatively or additionally, the charting device 104 can communicate with devices, such as the medical devices 102, by establishing or joining a previous established local network including data entry devices as well as diagnostic and/or therapeutic medical devices. This local network can be established in an ad-hoc manner at the time of treatment of a patient or patients in the field and can include two or more proximally located devices.

In various implementations, the charting device 104 can receive, organize, store, share, distribute, and display data from the other devices (e.g., the medical devices 102) to further enhance the usefulness of the devices and to make it easier for the healthcare provider 118 to perform certain tasks that would normally require the healthcare provider 118 to divert visual and manual attention to the other devices separately, according to examples of the present disclosure. In other words, the charting device 104 can centralize, organize, and share information that might otherwise be de-centralized and disorganized, according to examples of the present disclosure.

In certain examples, the charting device 104 can share information received from the server(s) 108 with the other devices. For instance, in one example, the charting device 104 can receive information from the server(s) 108 and can share (e.g., transmit via a local network) such information with the medical devices 102. Alternatively or additionally, if a medical device 102 takes an ECG reading of the patient 116, or if a medical device 102 administers a treatment (such as medication, chest compression, ventilation, defibrillation shock, etc.), information descriptive of the ECG and/or the treatment may be shared, via charting device 104 or directly, with other devices (e.g., the remote computing device 106 and/or the server(s) 108) for storage in a patient record maintained therein. In another example, the charting device 104 can be configured to receive patient information, such as medical records, known medical conditions, and biographical information form the health records data store 110, and to share this information with the medical devices 102. This biographical information can be inserted into a patient record (e.g., an ePCR) being maintained and/or generated at the charting device 104.

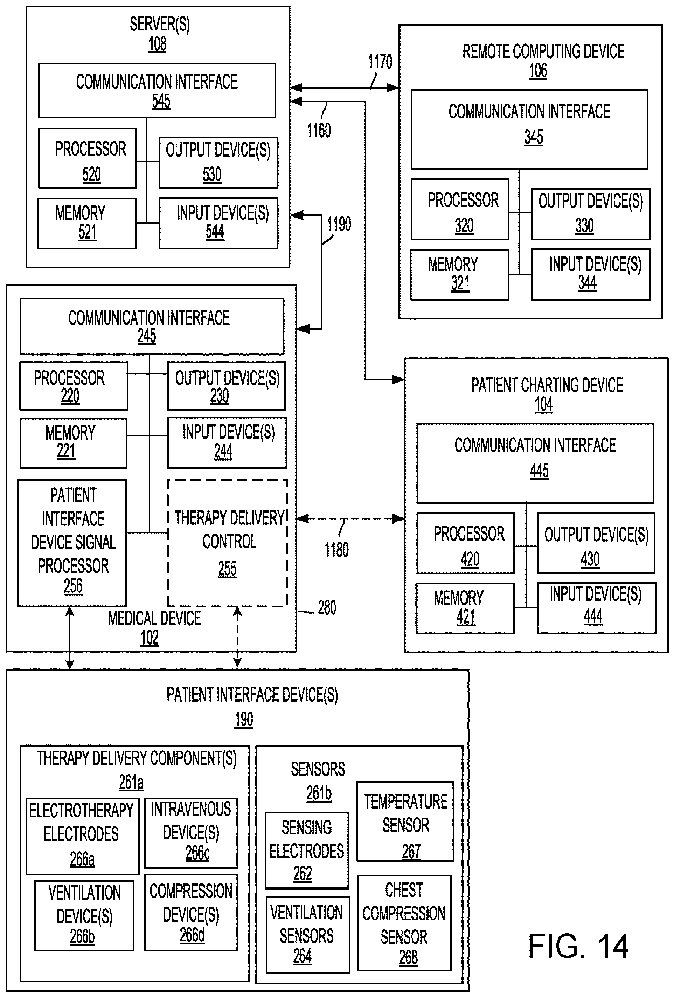

When the charting device 104 receives updated information from the other devices to which it is communicably coupled, and/or via input from the healthcare provider 118, the charting device 104 can send the updated information to the server(s) 108. Hence, information from one or more device(s) (e.g. the medical devices 102) may be stored locally at the charting device 104 (e.g., in the memory 421 of FIG. 14) and/or at the server(s) 108 (e.g., in the memory 521 of FIG. 14 and/or in the charting data store 134). Data from the charting device 104 (and, when present, data from the other devices that may be communicably coupled with the charting device 104) can be received by the server(s) 108 and stored in the charting data store 134 and/or the case data store 132 via the ePCR API 128 and/or the case API 126 as described further below. The computing device 106 can also access the stored information via these APIs to add the stored information to the health records data store 110.

According to some examples of the present disclosure, the charting device 104 can communicably couple (e.g. automatically, manually or selectively) to a medical device 102 that includes a wearable or implanted medical device, such as, for example, a LifeVest^{®} wearable defibrillator, a wearable patient monitor (e.g., a smart watch), a pacemaker, etc. In these examples, the charting device 104 receives and displays patient monitoring information generated by the wearable medical device. The charting device 104 can also be configured to receive patient-identifying information from the wearable medical device, thereby enabling the charting device 104 to query (e.g., across the network 112) an external database (e.g., the health records data stores 110) to retrieve additional information about the patient 116. The charting device 104 can also be configured to connect with an implantable cardioverter-defibrillator ("ICD") in a similar fashion, according to examples of the present disclosure.

In certain examples, the charting device 104 can be a tablet, smartphone, wearable device, and/or other mobile computing device and/or a combination of mobile devices that can access patient charting system capabilities described herein via a server or cloud interface, for example, an interface with the server(s) 108. According to some examples of the present disclosure, the charting device 104 can include a wristband and/or smart phone such as an Apple^{®} iPhone^{®} or iPad^{®} with an interactive data entry interface such as a touch screen or voice recognition data entry interface that can be communicably coupled to the tablet and tapped to indicate what a caregiver did with the patient 116 and when. Further, according to some examples of the present disclosure, the charting device 104 can be integrated with a medical device 102, such that a single device can be configured to monitor the patient, treat the patient, as well as to generate records and/or notes about the patient's condition and/or treatments applied to the patient 116. In these examples, the ePCR application can be embedded within the combination medical/computing device. Additional description of some components of the charting device 104 is provided further detail below with reference to FIG. 14.

The charting device 104 can include and implement the ePCR user interface 122 for an ePCR application. The ePCR user interface 122 can render visual, audio, haptic, and/or tactile content, including content relating to ePCR generation. Thus the ePCR user interface 122 can receive input or provide output, thereby enabling a user to interact with the charting device 104. The ePCR user interface 122 can be configured to receive charting data via various mechanisms, including, but not limited to, touchscreen, voice recognition, and scanner. For example, a patient may say his/her name and the ePCR user interface 122 can capture the patient name and store it as a portion of charting data. The ePCR user interface 122 can include a camera/scanner through which patient's driver license may be acquired/scanned and relevant information about the patient, such as name, address, and age, can be stored as charting data. The healthcare provider 118 may dictate data or findings when examining the patient 116 via the ePCR user interface 122. Such dictation can be captured and saved as charting data, according to some examples. In certain examples, the ePCR user interface 122 includes the input devices 444 and/or the output devices 430 of FIG. 14.

In some examples, the ePCR user interface 122 implemented by the charting device 104 can include a touchscreen and/or a flat panel PC or some other user interface hardware and a software stack configured to drive the hardware. Portions of the ePCR user interface 122 can be stored in the memory of the charting device 104 as an Android^{™} application, an Apple^{®} application, or other native application, and executed by the processor to interact with the healthcare provider 118. Alternatively or additionally, the memory of the charting device 104 may store a browser, or some other execution environment, configured to receive and render portions of the ePCR user interface 122 from one or more webserver(s).

In some examples, the ePCR user interface 122 can be utilized to access the ePCR application and to generate charting data to be stored in the charting data store 134. For instance, the ePCR user interface 122 can include a graphical user interface, which permits the user to select different subsets and/or display modes of the information gathered from and/or sent to other devices, according to examples of the present disclosure. In one example, the ePCR user interface 122 can be used to note a dosage of medicine, CPR compression depth, or other treatment parameters given to the patient at a particular time. The ePCR user interface 122 can also be used to record biographic and/or demographic and/or historical information about a patient, for example the patient's name, identification number, height, weight, and/or medical history, according to examples of the present disclosure. The types of charting data received via the ePCR user interface 122 can also include patient physiologic parameters, documented events, and the like. Charting data can include, but is not limited to, patient information (e.g., name, age, gender, weight, and/or other identification and/or demographic information), medical event specific information (e.g., type of service requested, disposition), and/or clinical information (e.g., patient assessment, patient blood pressure). Charting data can further include data from the patient care record, data from physician's chart, data from electronic health records, data from one or more health information exchanges, and data from hospital charts, in addition to data from ePCRs.

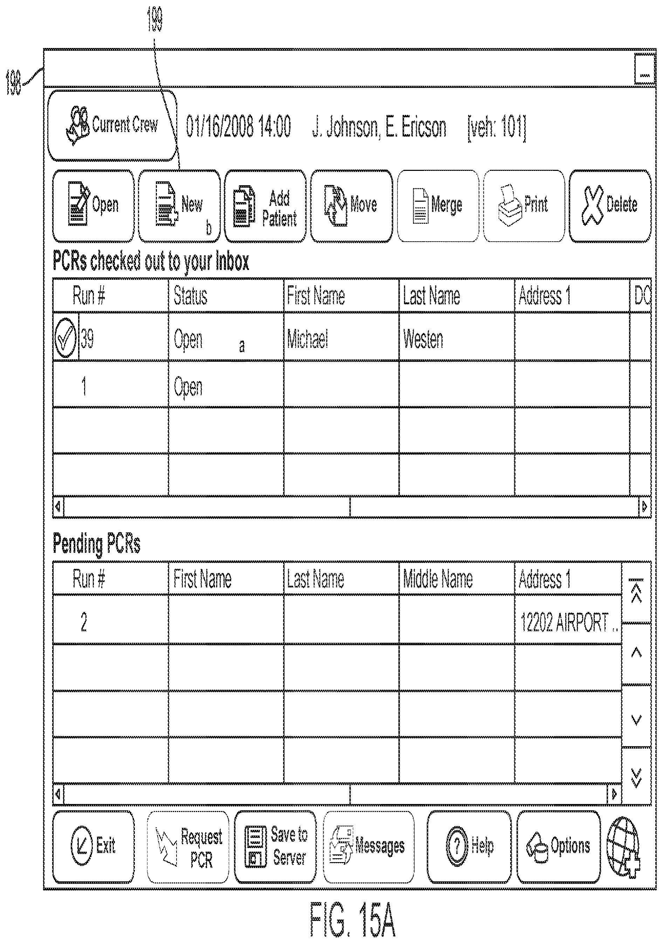

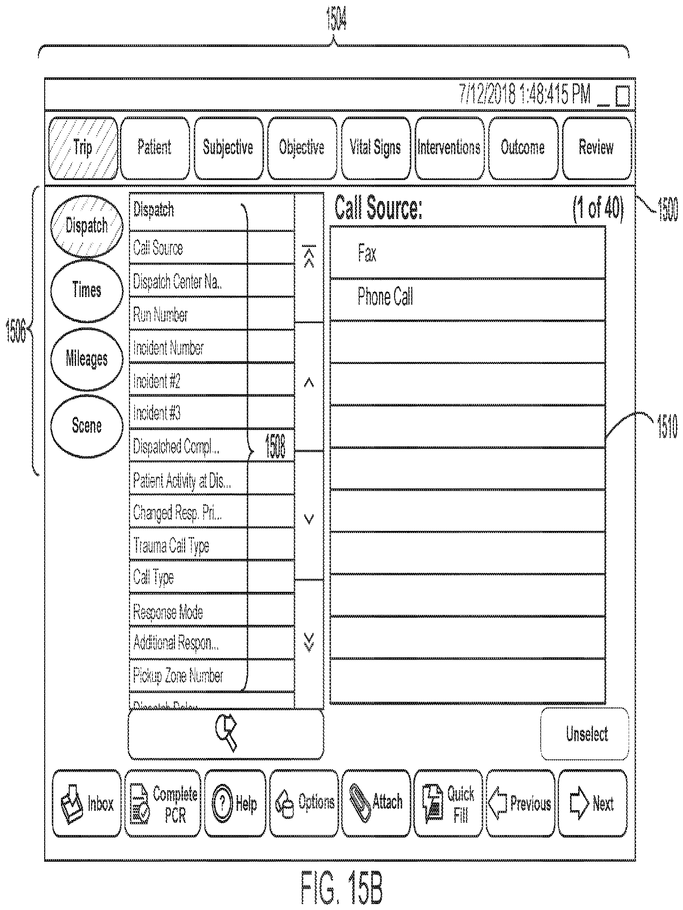

Referring to FIGS. 15A and 15B, examples of user interface screens provided by the ePCR user interface 122 to generate an ePCR are shown. The ePCR user interface 122 can include an ePCR initiation screen 198. A user (e.g., the healthcare provider 118) may select an ePCR generation control 199 to open one or more new ePCRs. Until the ePCR is completed or deleted, the "open ePCR" can be an ePCR in progress.

As shown in FIG. 15B, in one example implementation, selection of the ePCR generation control 199 can open the ePCR edit screen 1500. The ePCR edit screen 1500 can enable the user to generate the ePCR. The ePCR edit screen 1500 can provide primary controls 1504 and secondary controls 1506. In the example shown in FIG. 1B, the primary control "trip" is selected. The secondary controls 1506 that correspond to "trip" are dispatch, times, mileages, and scene. In this example, the secondary control "dispatch" is selected. Based on the selected primary and secondary controls, the ePCR application can provide a list 1508 of selectable fillable fields and a selectable menu 1510 of data entries for the selected fillable field. Via the selection of an item within the selectable menu 1510, the ePCR application can enable the user to fill fields of the ePCR in progress in order to complete the ePCR. Other types of charting data can be entered into various screens rendered by the ePCR user interface 122 in response to selection of, for example, the vital signs primary control, the patient primary control, the interventions primary control, and the outcome primary control.

In various implementations, the medical devices 102 can include a patient treatment device, or another kind of device that includes patient monitoring and/or patient treatment capabilities, according to examples of the present disclosure. For example, the medical devices 102 include a defibrillator and can be configured to deliver therapeutic electric shocks to the patient. In some examples, the medical devices 102 can deliver other types of treatments, such as providing ventilation, operating a respirator, and/or administering drugs or other medication.

More specifically, the medical devices 102 can include, for example, a defibrillator with patient interface devices 190 such as electrodes and/or sensors configured for attachment to the patient 116 to monitor heart rate and/or to generate electrocardiographs (ECGs), according to examples of the present disclosure. Each of the medical devices 102 can incorporate a clock, which can be synchronized with an external time source such as a network resource or a satellite to prevent the healthcare provider 118 from having to manually enter a time of treatment or observation (or having to attempt to estimate the time of treatment), according to examples of the present disclosure. The medical devices 102 can also include and/or couple to patient interface devices 190 such as sensors to detect and/or a processor to derive or calculate other patient parameters. In some examples, the medical devices 102 are configured to interoperate with the patient interfaces device(s) 190 to monitor, detect, treat, and/or derive or calculate blood pressure, temperature, respiration rate, blood oxygen level, end-tidal carbon dioxide level, pulmonary function, blood glucose level, and/or weight, according to examples of the present disclosure. Additional examples of the medical devices 102 and the patient interface devices 190 are described further below with reference to FIG. 14.

Although FIG. 1 depicts a single charting device 104, in certain examples more than one charting device 104 may be used. According to examples of the present disclosure, the server(s) 108 can receive patient charting data from charting device 104 and store it in the charting data store 134 along with an authenticated timestamp and an identifier associating the information with a particular patient charting device 104. In this way, data from multiple charting devices can be accessed by various users.

In some examples, the network 112 can include one or more communication networks through which the medical devices 102, the patient charting device 104, the remote computing device 106, and the server(s) 108 can send, receive, and/or exchange data. In various implementations, the network 112 can include a cellular communication network and/or a computer network. In some examples, the network 112 includes and supports wireless network and/or wired connections. For instance, in these examples, the network 112 may support one or more networking standards such as Global System for Mobile Communications (GSM), Code-Division Multiple Access (CDMA), Universal Serial Bus (USB), Bluetooth^{®}, Controller Area Network (CAN), ZigBee^{®}, Wireless Ethernet, Ethernet, and Transmission Control Protocol/Internet Protocol (TCP/IP), among others. The network 112 may include both private networks, such as local area networks, and public networks, such as the Internet. It should be noted that, in some examples, the network 112 may include one or more intermediate devices involved in the routing of packets from one endpoint to another. However, in other examples, the network 112 can involve only two endpoints that each have a network connection directly with the other.

The server(s) 108 can include one or more physical and/or virtual server computers configured to implement the case API 126, the ePCR API 128, the file integration service 130, the case data store 132, the criteria data store 136, and the charting data store 134. As such, the server(s) 108 can include one or more application servers, web servers, and/or data base servers. The server(s) 108 can communicate with the remote computing device 106, the medical devices 102, and the charting device 104 via the network 112. The server(s) 108 can include enterprise servers configured to support an organization as a single tenant and/or cloud servers configured to support multiple organizations as multiple tenants.

The server(s) 108 may be implemented in one or more clouds that may communicably couple to one another. For example, a single cloud including one or more servers may include the elements 126, 128, 130, 134, 132, 136, and 138 of FIG. 1. Alternatively, elements 126 and 132 may be implemented in a first cloud, elements 130, 136, and 138 may be implemented in a second cloud, and elements 128 and 124 may be implemented in a third cloud. As a further alternative, the elements 130, 136, and 138 may be implemented in a same cloud as elements 126 and 132 or may be implemented in a same cloud as elements 128 and 134. As yet another alternative, elements 126, 132, 128, and 134 may be implemented in a first cloud and elements 130, 136, and 138 may be implemented in a second cloud.

One or more of the communication links 188a and 189a, as illustrated in FIG. 1 between the file integration service 130 and the patient encounter data source integration service 138 and the medical device case data store 132, are optional links. Similarly, one or more of the communication links 188b and 189b, as illustrated in FIG 1. between the file integration service 130 and the patient encounter data source integration service 138 and the charting data store 134, are optional links. In an implementation, the file integration service 130 and/or the patient encounter data source integration service 138 may not communicate directly with these data stores for security purposes. In certain implementations, the file integration service 130 and/or the data source integration service 138 is conversant in multiple data formats and is able to interact with components of the medical device 102 and the patient charting device 104 via a public API which can serve as a gatekeeper to resources provided by such devices. For example, the medical device case data store 132 and the charting data store 134 may be owned by a first entity or separately owned by a first and second entity. The data source integration service 138 and the file integration service 130 may be owned by a third entity or separately owned by a third and fourth entity. Thus the API's (126 and/or 128) may provide security between these various entities in regard to access to the data stores 132 and 134.

For these and/or other reasons, in certain implementations it may be preferred that the patient encounter data source integration service 138 not communicate directly with the medical device case data store 132 and/or the charting data store 134, as indicated by the broken lines 188a and 188b shown in FIG. 1. In such case, the patient encounter data source integration service 138 may send the case API 126 a request for information that is stored in the medical device case data store 132. Likewise, the patient encounter data source integration service 138 may send the ePCR API 128 a request for information that is stored in the charting data store 134. Either or both of these requests may include appropriate security credentials. Based on the security credentials, the case API 126 and/or the ePCR API 128 may either grant or deny the request for information. If the respective API grants the request for information, then the API will retrieve the requested information from the appropriate data store. The patient encounter data source integration service 138 may perform one or more data merge operations according to one or more criteria identified by the service 138. When the patient encounter data source integration service 138 completes the one or more data merge operations, the patient encounter data source integration service 138 may return the merged file (for example, a file that includes multiple medical device data files merged into a single file) to the case API 126 for storage in the medical device case data store 132.

Similarly, for these and/or other reasons, in certain implementations it may be preferred that the file integration service 130 not communicate directly with the medical device case data store 132 and/or the charting data store 134, as indicated by the broken lines 189a and 189b shown in FIG. 1. In such case, the file integration service 130 may send the case API 126 a request for information that is stored in the medical device case data store 132. Likewise, the file integration service 130 may send the ePCR API 128 a request for information that is stored in the charting data store 134. Either or both of these requests may include appropriate security credentials. Based on the security credentials, the case API 126 and/or the ePCR API 128 may either grant or deny the request for information. If the respective API grants the request for information, then the API will retrieve the requested information from the appropriate data store. The file integration service 130 may perform one or more data merge operations according to one or more criteria identified by the file integration service 130. When the file integration service 130 completes the one or more data merge operations, the file integration service 130 may return the merged file (for example, a file that includes medical device data merged into patient charting data) to the ePCR API 128 for storage in the charting data store 134.

In some examples, the server(s) 108 can exchange data with remote devices such as the medical devices 102, the charting device 104, and the remote computing device 106 via the case API 126 and the ePCR API 128. These APIs are configured to receive, process, and respond to commands issued by processes implemented by the remote devices, such as the ePCR user interface 122 described herein. The APIs 126 and 128 may be implemented using a variety of interoperability standards and architectural styles. For instance, in one example, the APIs 126 and 128 are web services interfaces implemented using a representational state transfer (REST) architectural style. In this example, the APIs 126 and 128 communicate with a client process using Hypertext Transfer Protocol (HTTP) along with JavaScript Object Notation and/or extensible markup language. In some examples, portions of the HTTP communications can be encrypted to increase security. Alternatively or additionally, in some examples, the APIs 126 and 128 are implemented as a .NET web API that responds to HTTP posts to particular uniform resource locators with data descriptive of case data and charting data. Alternatively or additionally, in some examples, the APIs 126 and 128 are implemented using simple file transfer protocol commands and/or a proprietary application protocol accessible via a transmission control protocol socket. Thus, the APIs 126 and 128 as described herein are not limited to a particular implementation.

In some examples, the APIs 126 and 128 include a plurality of endpoints to enable reliable system performance. For instance, in at least one example, the each of the APIs 126 and 128 includes one or more first endpoints to receive and process requests for data previously stored in the case data store 132 or the charting data store 134 and one or more second endpoints to receive and process requests to store new data within the case data store 132 or the charting data store 134. This configuration can ensure that requests for data already stored in the case data store 132 or the charting data store 134 can be quickly serviced with minimal latency. This bifurcated architecture can be helpful because requests to upload ePCRs or medical device case files, parse the ePCRs or medical device case files, and store the resulting charting data or case data in the case data store 132 or the charting data store 134 can require more processing time and resources.

In some examples, the APIs 126 and 128 are configured to transmit messages to the file integration service 130 that notify the file integration service 130 of (and/or include) newly received case data and charting data. Where the messages merely notify the file integration service 130 of the presence of new data, the messages can include one or more identifiers of the new data that can be utilized by the file integration service 130 to retrieve the new data from the case data store 132 and/or the charting data store 134, although the inclusion of identifiers of the new data is not a requirement. In certain examples, the case API 126 is configured to receive case files from the medical devices 102, process the case files, and transmit messages to the medical devices 102 that indicate the result of the processing. This processing can include parsing the case files to retrieve values of case data stored therein and storing the case data in the case data store 132. Similarly, in some examples, the ePCR API 128 is configured to receive ePCRs from the charting device 104, process the ePCRs, and transmit a message to the charting device 104 that indicates the result of the processing. This processing can include parsing the ePCRs to retrieve values of charting data stored therein and storing the charting data and/or the ePCRs in the charting data store 134.

Continuing with FIG. 1, the charting data store 134 can be implemented by, for example, a database (e.g., a relational database) and stored on a non-transitory storage medium. In an implementation, the charting data store 134 includes a plurality of records that store charting data derived from a plurality of ePCRs. In at least one example, the charting data store 134 is organized into a set of relational database tables that includes an ePCR table and an ePCR fields table. In this example, the ePCR table includes rows of data that are each descriptive of an ePCR that documents a patient encounter in the charting database 134. Thus, each row in the ePCR table can include fields configured to store a unique identifier of the ePCR, a timestamp indicating when the patient charting device 104 created the ePCR, and metadata descriptive of the patient encounter documented by the ePCR (e.g., patient identification information that uniquely identifies the patient, healthcare providers involved in the patient encounter, unique identifiers of medical devices and supplies used in the patient encounter, overall issues that occurred during the patient encounter, and/or a type of dispatched EMS event associated with the ePCR).

Continuing with this example, the ePCR fields table includes rows of data that are each descriptive of a field stored within an ePCR. Thus, each row in the ePCR fields table includes fields configured to store a unique identifier of the ePCR to which the field belongs, a field that uniquely identifies the field among the fields associated with the ePCR, a date/time stamp associated with an ePCR data field value, a unique identifier of the source of the value (e.g., a particular medical device or a particular computing device), and a field that identifies (via a type identifier or textual information) one or more values associated with the field. Notably, each ePCR can have a large quantity of fields that each require entry of a specific data type. For instance, some example ePCRs may have 100 fields, 200 fields, 400 fields, 600 fields, or more. This quantity of fields may be mandated and, therefore, it is important that each ePCR be complete. Therefore, in some examples the charting data store 134 and/or other components, such as the ePCR user interface 122, are configured to prevent modification/deletion of ePCR fields by a user of the ePCR application. It should be noted that, in some examples, ePCRs can be serialized into files for transmission to the charting data store 134. In these examples, the charting data store 134 can store complete and distinct copies of the ePCR files themselves (e.g., as large binary objects).

Continuing with FIG. 1, the case data store 132 can be implemented by, for example, a database (e.g., a relational database) and stored on a non-transitory storage medium. In an implementation, the case data store 132 includes a plurality of records that store case data derived from case files from a plurality of medical devices used to treat patients during encounters. Moreover, in some examples, the case data store 132 can store complete copies of the case files themselves (e.g., as large binary objects). The case data stored in the case data store 132 can document patient encounters from the point of view of medical devices. As such, case data generated by a medical device during a patient encounter can include an identifier of the medical device, physiologic parameter values of the patient recorded by the medical device during the encounter, characteristics of treatment provided by the medical device to a patient during the encounter, actions taken by healthcare providers during the encounter, and timestamps associated with medical device case data. For instance, where the medical device is a defibrillator, the case data can include patient physiologic parameters such as ECG data for the patient, as well as characteristics of therapeutic shocks delivered by the defibrillator to the patient, CPR performance data, and timestamps reflecting a power-on time for the defibrillator and associated with recorded case data, among other information.

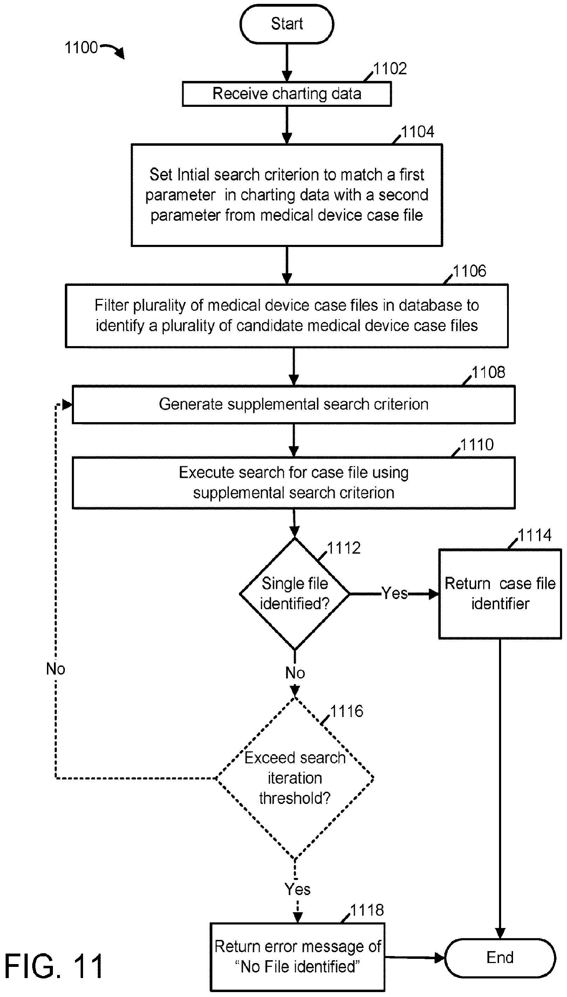

The criteria data store 136 can be implemented by, for example, a database (e.g., a relational database) and stored on a non-transitory storage medium. In one example, the criteria data store 136 includes a plurality of records that each specify a relationship between parameters of case and charting data. In this example, each of the plurality of records also includes information that ranks the relationship relative to other relationships within the criteria data store 136. This ranking can be accessed by the file integration service 130 during an iterative searching processes, as described further below with reference to FIGS. 11-13.

Criteria data store 136 optionally maintains an audit trail that tracks which data in an integrated record originated from an ePCR charting data file, and which data in the integrated record originated from a medical device case file. In certain embodiments where conflicting data exists, the most recently acquired data can take precedence over older data. In other embodiments where conflicting data exists, the configuration information can be used to specify a hierarchy of data sources that determine which data takes precedence (for example, retained) and which data is relegated (for example, discarded).

In one implementation an audit trail includes reference numbers for each file processed by the file integration service 130 along with an indication of the result of processing each file. For example, a medical device case file could be indicated as (i) having been merged with another medical device case file, or (ii) merged with an ePCR charting data file. Where multiple original medical device case files are merged and the resulting merged file is subsequently processed by the file integration service 130, the information in the audit file identifying the original files can be used to allow the file integration service 130 to (i) ignore the merged file if the original files have already been processed; (ii) select only a portion of the merged file for integration with an ePCR charting data file (for example, if the remainder of the merged file has already been integrated; or (iii) integrate the merged file with an ePCR charting data file with redundancy information that the ePCR API 128 can use to remove redundant information.

The case data store 132 and the charting data store 134 can be organized according to a variety of physical and/or logical structures. In at least one example, the case data store 132 and the charting data store 134 are implemented within a relational database having a highly normalized schema and accessible via a structured query language (SQL) engine, such as ORACLE or SQL-SERVER. This schema can, in some implementations, include columns and data that enable the case data store 132 and/or the charting data store 134 to house data for multiple tenants. In addition, although the description provided above illustrates the case data store 132 and the charting data store 134 as relational databases, the examples described herein are not limited to that particular physical form. Other databases may include flat files maintained by an operating system and including serialized, proprietary data structures, hierarchical database, xml files, NoSQL databases, document-oriented databases and the like. Thus, the case data store 132 and the charting data store 134 as described herein is not limited to a particular implementation.

The case data store 132 and the charting data store 134 can securely store the information received from the charting device 104 and/or the medical devices 102 for longer periods of time than the remote devices to permit later use of the information. For example, the charting device 104 may receive patient-identifying information such as name, address, and/or social security number via user input directly into the charting device 104, and then may convey some or all of the patient-identifying information to the server(s) 108 to query the charting data store 134 for past records involving the patient 116. In an implementation, the charting device 104 can convey some or all of the patient-identifying information to other servers via the network 112 to access patient records and/or information from various databases such as those provided by a medical facility, insurance company, medical billing service, financial record service, and/or a health information exchange. In other examples, the charting device 104 can be configured to receive information in other ways, including without limitation wired or wireless communication and/or messaging.

The server(s) 108 and/or other servers accessed via the network 112 can then forward any such records or portions of such records back to the charting device 104 (e.g. for display in a patient charting screen or past medical history screen) to assist the healthcare provider 118 with the current emergency encounter. Similarly, such past encounter information may also be accessed by other users, according to examples of the present disclosure.

The remote computing device 106 can include one or more physical and/or virtual computers configured to implement the health records data store 110. The health records data store 110 can include records descriptive of patient medical history. These records can include a variety of patient information, such as medical history prior to an encounter, symptoms that lead to an encounter, any diagnosis identified during the encounter, treatments prescribed as a result of the encounter, and outcomes resulting from the treatments. In at least one example, the remote computing device 106 and the health records data store 110 collectively act as a health information exchange (HIE) that is configured to expose one or more APIs that support health data exchange standards, such as state-wide health data exchange standards, regional health data exchange standard, HL7 message standards, and National Council for Prescription Drug Programs (NCPDP) script standards. It should be noted that the health records data store 110 can include data generated by one organization or by other organizations or networks or third-party sources, such as hospitals, clinics, doctors' offices, and pharmacies.

In some examples, the file integration service 130 is configured to identify case data (e.g., a case file) within the case data store 132 to associate with charting data (e.g., an ePCR) within the charting data store 134. FIG. 2A illustrates one example of an integration process 200a executed by the file integration service 130 in some implementations.

As shown in FIG. 2A, the process 200a starts with the file integration service 130 receiving 202a an ePCR charting data file uploaded by the patient charting device 104. The charting data file may constitute a completed ePCR that documents a patient encounter. For example, the file integration service 130 can receive 202a a message from an API (e.g., the ePCR API 128 of FIG. 1) indicating that the ePCR is available in a charting data store 134 and including a file identifier or may receive 202a the ePCR file including the charting data from the API. The message from the API may further include a request from the patient charting device 104 (e.g., based on input from the user 118 to the ePCR user interface 122) for an association and integration of the ePCR file with the medical device case file.

Continuing the process 200a, the file integration service 130 generates 204a at least one search criterion based on the charting data. In some examples, the at least one search criterion specifies at least one predetermined relationship between at least one first parameter from the charting data and at least one second parameter associated with a medical device case file. As discussed in further detail below, as examples, the first parameter and the second parameter may be a case start time or a medical device identifier (e.g., a QR code or serial number) entered into each of the charting data and the medical device case file. This example is not limiting of the disclosure and other examples, including examples discussed herein, are within the scope of the disclosure. The predetermined relationship can vary between examples. For instance, in some examples, the predetermined relationship can be a match between the at least one first parameter and the at least one second parameter. In an implementation, the match may be an equality. A predetermined relationship of equality can be particularly useful where the at least one first parameter and the at least one second parameter include numeric values or strings that can be matched with precision. Alternatively or additionally, the predetermined relationship can be a proximity or similarity between the at least one first parameter and the at least one second parameter. A predetermined relationship based on proximity or similarity can be particularly useful where the at least one first parameter and the at least one second parameter include timestamps, physiological measurements taken at different times or by different devices, or other values that can only be matched inexactly (e.g., by satisfying a threshold proximity or similarity). Alternatively or additionally, the predetermined relationship can be a combination of equality, proximity, and/or similarity between multiple parameters. For instance, in one example, the predetermined relationship requires at least one of an overlapping range between a case start time and a case end time as recorded in a case file and the ePCR and an equality between a medical device identifier from the case file and the ePCR and at least one of an equality between a patient biometric identifier from the case file and the ePCR and an equality between a healthcare provider biometric identifier from the case file and the ePCR. In an example, the medical device identifier may include information from a radio-frequency identification (RFID) tag, a barcode, or a QR code.

In some examples, the at least one predetermined relationship is a hardcoded part of the file integration service 130. In these examples, the file integration service 130 generates 204a the at least one search criterion by identifying the at least one first parameter within the charting data and associating the at least one first parameter with the predetermined relationship. For example, where the predetermined relationship is proximal and requires a second parameter be within a range of a first parameter and the first parameter is a timestamp indicating a case start time, the file integration service 130 generates 204a the at least one search criterion by identifying the timestamp within the charting data and associating the timestamp with the range of case start times for subsequent use in searching 206a. It should be noted that such a range can be a dynamic value calculated by the file integration service 130 and/or a predetermined value. For instance, a range can include a calculated range between a case start time and a transfer-of-care time from the charting data. Alternatively or additionally, a predetermined range of case start times can be, for example, between 0 to 1, 0 to 2, 0 to 5, and 1 to 10 minutes. Any given predetermined relationship can apply these ranges between time parameters indicating the same event (e.g., case start times) or between time parameters indicating different events (e.g., a case start time and a treatment time or a transfer time).

In some examples, the at least one predetermined relationship is soft-coded and stored within a criteria data store (e.g., the criteria data store 136 of FIG. 1). In these examples, the file integration service 130 can generate 204a the at least one search criterion by identifying a preferred predetermined relationship from the criteria data store prior to identifying, within the charting data as described above, the at least one first parameter specified by the predetermined relationship. For instance, in some examples, the file integration service 130 can identify the preferred predetermined relationship by finding the predetermined relationship with the highest rank that has not yet been used by the current instance of the process 200a.

It should be noted that each of the at least one first parameter and the at least one second parameter can be a single parameter or a plurality of parameters. For instance, in some examples, the at least one first parameter is a timestamp indicating a case start time for the patient encounter and the at least one second parameter is a timestamp indicating a time when the medical device started a case file for the patient encounter. In other examples, the at least one first parameter includes a plurality of first parameters and the at least one second parameter includes a plurality of second parameters. In some of these examples, the plurality of first parameters includes a timestamp indicating a case end time for a patient encounter (e.g., when the patient charting device 104 uploaded the ePCR to the server(s) 108) and an identifier of a charting device that generated the ePCR and the plurality of second parameters includes a timestamp indicating a time when the medical device 102A, ... , 102N uploaded a case file to the server(s) 108 and an identifier of a medical device that generated the case file. Other first parameters can include patient identifiers, healthcare provider identifiers, medical device identifiers, timestamps indicative of patient transfer, patient treatment information, patient medical information, patient demographic information, and physiological measurements specified in the charting data. Other second parameters can include patient identifiers, healthcare provider identifiers, medical device identifiers, timestamps indicative of patient transfer, patient treatment information, patient medical information, patient demographic information, and physiological measurements taken by a medical device specified in the case file. The patient identifiers can include biometric information (e.g., facial recognition information, fingerprint information, retinal scan information, and the like). The medical device identifiers can include identifiers manually entered into an ePCR, identifiers retrieved from memory of the medical device, identifiers electronically transmitted by the medical device to the charting device, and/or identifiers scanned from a bar or QR code associated with the medical device. The physiological measurements can include blood pressure, body temperature, respiratory rate, heart rate, and electrocardiogram (ECG) data.

Continuing the process 200a, the file integration service 130 searches 206a the case data for a case file that satisfies the at least one search criterion. During this searching 206a, the file integration service 130 can identify 208a a case file to associate with the charting data based on the at least one search criterion. In associating the case file with the charting data file, the file integration service 130 maps these files to one another based on their common association with the same patient during a same patient encounter with this patient. The files mapped to one another were both generated during this same patient encounter and the file integration service 130 identifies this correspondence between these files. For instance, the file integration service 130 can identify 208a a case file to associate with the charting data where a parameter in and/or associated with the medical device case file satisfies the predetermined relationship with a parameter in and/or associated with the patient charting record. For example, the parameter in and/or associated with the case file may match the parameter in and/or associated with the charting data (e.g., a case start time in the case file may match a case start time in the charting data).

Where the file integration service 130 identifies 208a a case file to associate with the charting data based on the at least one search criterion, the file integration service 130 integrates 210a and stores 212a an integrated patient encounter record and the process 200a ends. The integrated patient encounter record may be, for example, a supplemented case data file, a supplemented charting data file, a new file that includes charting data and case data, a file integration pointer, a case data file, and/or charting data file that includes integration information. For instance, in some examples, the file integration service 130 integrates 210a the charting data with the case data by importing the charting data into the case files. This integration 210a generates supplemented case files. Alternatively or additionally, in some examples, the file integration service 130 integrates 210a the charting data with the case data by importing the case data into the ePCR. This integration 210a generates a supplemented ePCR. In an implementation, the file integration service 130 integrates 210a the charting data and the case data to create a new file, in which case the charting data file and the case data files are left unchanged. In an implementation, the file integration service 130 stores integration information (e.g., in the charting data file and/or the case data file) that provides an association between the two files. Alternatively, the file integration service 130 may store the integration information as a linking pointer that provides both the case data file and the charting data file to a user in response to a query for one or the other or both. The association enables access to the case data file from the charting data file and/or access to the charting data file from the case data file. The integrated patient encounter record can include, for example, a copy of or a pointer to the case file, case data generated from the case file, and/or the charting data. Further, in some examples, the integrated patient encounter record can include a supplemented ePCR that includes the charting data and case data imported from and associated with the identified case file. The integrated patient encounter record can be stored, for example, in the charting data store and/or the case data store.

In some cases multiple medical device case files from a single patient encounter may exist and may satisfy the one or more search criteria. This may occur when multiple medical devices are used to diagnose and/or treat a patient during an encounter. Or it may occur when a single medical device generates multiple files, such as may happen if a battery is changed during an encounter. Regardless of the reason that multiple medical device case files exist, multiple of the components disclosed herein, including the patient encounter data source integration service 138 and/or the file integration service 130, can be used to link or merge such multiple files from a single patient encounter. In some cases one of these two components is configured to have higher priority than the other. In such case integrated data generated by the higher priority component will overwrite or otherwise supersede data generated by the lower priority component. One way of implementing this is to have the higher priority component process the medical device case files first and flag files which have been integrated. Unflagged files can then be subsequently processed by the lower priority component.

FIG. 2B illustrates an example of an integration process 200b that is executed by the file integration service 130 and that identifies a medical device case file that includes a medical device identifier that exactly matches a medical device identifier included in the charting data.

As shown in FIG. 2B, the process 200b starts with the file integration service 130 receiving 202b an ePCR charting data file uploaded by the patient charting device 104. The file integration service 130 can receive 202b within the process 200b by executing actions such as those executed by the file integration service 130 to receive 202a within the process 200a described above with reference to FIG. 2A.

The file integration service 130 generates 204b at least one search criterion that specifies that a medical device identifier included in the charting data must match exactly a medical device identifier included in the medical device case file. The medical device identifier may include information from, for example, an RFID tag, a barcode, or a QR code. The medical device identifiers can include identifiers manually entered into an ePCR, identifiers retrieved from memory of the medical device, identifiers electronically transmitted by the medical device to the charting device, and/or identifiers scanned from a bar or QR code associated with the medical device.

Continuing the process 200b, the file integration service 130 searches 206b the case data for a case file that satisfies the at least one search criterion. In particular, the file integration service 130 searches for a medical device case file that includes a medical device identifier that exactly matches a medical device identifier included in the charting data. Where the file integration service 130 identifies 208b a case file to associate with the charting data based on the at least one search criterion, the file integration service 130 integrates 210b and stores 212b an integrated patient encounter record and the process 200b ends. The file integration service 130 can integrate 210b and store 212b within the process 200b by executing actions such as those executed by the file integration service 130 to integrate 210a and store 212a within the process 200a described above with reference to FIG. 2A.

Another example of an integration process that is executed by the file integration service 130 identifies a medical device case file that includes a case start time and/or case end time that exactly matches a case start time and/or case end time included in the charting data. In such a process the file integration service 130. generates at least one search criterion that specifies that a case start time and/or case end time included in the charting data must match exactly a case start time and/or case end time included in the medical device case file. For example, in one implementation a case start time included in the charting data exactly matches a case start time included in the medical device case file. In another implementation, a case end time included in the charting data exactly matches a case end time included in the medical device case file. In another implementation, (a) a case start time included in the charting data exactly matches a case start time included in the medical device case file and (b) a case end time included in the charting data exactly matches a case end time included in the medical device case file. The file integration service 130 then searches the case data for a case file that satisfies the at least one search criterion. In particular, the file integration service 130 searches for a medical device case file that includes a case start time and/or case end time that exactly matches a case start time and/or case end time included in the charting data. Where the file integration service 130 identifies a case file to associate with the charting data based on the at least one search criterion, the file integration service 130 integrates and stores an integrated patient encounter record. , for example by executing actions such as those executed by the file integration service 130 to integrate 210a and store 212a within the process 200a described above with reference to FIG. 2A.

FIG. 2C illustrates an example of an integration process 200c that is executed by the file integration service 130 and that identifies a medical device case file that includes a case start time and/or a case end time that are within a "target time range" that is defined based on case start and end times included in the charting data. In some implementations the target time range begins at the case start time included in the charting data, and ends at the case end time included in the charting data. In other implementations the target time range begins slightly before or slightly after the case start time included in the charting data, and ends slightly before or slightly after the case end time included in the charting data. Thus the target time range may be defined by target range start and end times that are within a threshold proximity of respective case start and end times included in the charting data. For example, if the charting data indicates a case start time of 10:00am and a case end time of 10:15am, the target time range may be 9:55am to 10:20am, 9:55am to 10:16am, 9:59am to 10:20am, 10:01am to 10:15am, 10:00am to 10:14am, or any other suitable range. Thus it will be appreciated that the difference between the target range start time and the charting data case start time is not necessarily the same as the difference between the target range end time and the charting data case end time.

As shown in FIG. 2C, the process 200c starts with the file integration service 130 receiving 202c an ePCR charting data file uploaded by the patient charting device 104. The file integration service 130 can receive 202c within the process 200c by executing actions such as those executed by the file integration service 130 to receive 202a within the process 200a described above with reference to FIG. 2A.

The file integration service 130 generates 204c at least one search criterion that specifies that a case start time and a case end time included in the medical device case file are within a target time range. As noted above, the target time range is defined based on case start and end times included in the charting data. In some implementations the target time range begins at the case start time included in the charting data, and ends at the case end time included in the charting data. In other implementations the target time range begins slightly before or slightly after the case start time included in the charting data, and ends slightly before or slightly after the case end time included in the charting data.

Continuing the process 200c, the file integration service 130 searches 206c the case data for a case file that satisfies the at least one search criterion. In particular, the file integration service 130 searches for a medical device case file that includes a case start time and a case end time that are within the specified target time range. For example, in one implementation the file integration service 130 generates 204c the at least one search criterion by defining the target time range based on relevant timestamps within the charting data. In another implementation the file integration service 130 generates 204c the at least one search criterion by defining the target time range based on relevant timestamps within the charting data and applying a suitable threshold proximity to the target range start time and/or the target range end time. Thus the target time range is not necessarily defined by the exact start and end times included in the charting data. Where the file integration service 130 identifies 208c a case file to associate with the charting data based on the at least one search criterion, the file integration service 130 integrates 210c and stores 212c an integrated patient encounter record and the process 200c ends. The file integration service 130 can integrate 210c and store 212c within the process 200c by executing actions such as those executed by the file integration service 130 to integrate 210a and store 212a within the process 200a described above with reference to FIG. 2A.

FIG. 2D illustrates an example of an integration process 200d that is executed by the file integration service 130 and that identifies a medical device case file that includes (a) a medical device identifier that exactly matches a medical device identifier included in the charting data, and (b) a case start time and a case end time that are within a "target time range" that is defined based on case start and end times included in the charting data. As noted above, in some implementations the target time range begins at the case start time included in the charting data, and ends at the case end time included in the charting data. In other implementations the target time range begins slightly before or slightly after the case start time included in the charting data, and ends slightly before or slightly after the case end time included in the charting data. Thus the target time range may be defined by target range start and end times that are within a threshold proximity of respective case start and end times included in the charting data. The difference between the target range start time and the charting data case start time is not necessarily the same as the difference between the target range end time and the charting data case end time.

As shown in FIG. 2D, the process 200d starts with the file integration service 130 receiving 202d an ePCR charting data file uploaded by the patient charting device 104. The file integration service 130 can receive 202d within the process 200d by executing actions such as those executed by the file integration service 130 to receive 202a within the process 200a described above with reference to FIG. 2A.

The file integration service 130 generates 204d at least one search criterion that specifies that (a) a medical device identifier included in the charting data must match exactly a medical device identifier included in the medical device case file, and (b) a case start time and a case end time included in the medical device case file are within a target time range. As noted above, the target time range is defined based on case start and end times included in the charting data. In some implementations the target time range begins at the case start time included in the charting data, and ends at the case end time included in the charting data. In other implementations the target time range begins slightly before or slightly after the case start time included in the charting data, and ends slightly before or slightly after the case end time included in the charting data.

Continuing the process 200d, the file integration service 130 searches 206d the case data for a case file that satisfies the at least one search criterion. In particular, the file integration service 130 searches for a medical device case file that includes (a) a medical device identifier that exactly matches a medical device identifier included in the charting data, and (b) a case start time and a case end time that are within the specified time range. Where the file integration service 130 identifies 208d a case file to associate with the charting data based on the at least one search criterion, the file integration service 130 integrates 210d and stores 212d an integrated patient encounter record and the process 200d ends. The file integration service 130 can integrate 210d and store 212d within the process 200d by executing actions such as those executed by the file integration service 130 to integrate 210a and store 212a within the process 200a described above with reference to FIG. 2A.

As noted above, a search criterion specifies a relationship between parameters of case and charting data, and enables the identification of a medical device case file that is to be integrated with related charting data. FIGs. 2B through 2D illustrate example file integration processes, each of which uses one or more search criteria to search for and identify a medical device case file. Other search criteria can be used in these and other implementations, including one or more of the following: (i) a search criterion specifying that a case start time included in a medical device case file is within a threshold proximity of a case start time included in ePCR charting data; (ii) a search criterion specifying that a case end time included in a medical device case file is within a threshold proximity of a case end time included in ePCR charting data; (iii) a search criterion specifying that case start and end times included in a medical device case file are both within a target time range, wherein the target time range begins at a case start time included in ePCR charting data and ends at a case end time included in the ePCR charting data; and (iv) a search criterion specifying that case start and end times included in a medical device case file are both within a target time range, wherein the target time range begins at a specified duration before a case start time included in ePCR charting data and ends at the specified duration after a case end time included in the ePCR charting data. In implementations where a search criterion that relies on comparing timestamps included in ePCR charting data with timestamps included in a medical device case file, optional adjustments can be included to account for different time standards upon which the timestamps in the ePCR charting data and the timestamps in the medical device case file are based. Such differing time standards may be the result of using different time zones or using daylight savings time.

The various search criteria and methods described in FIGS. 2B, 2C, and 2D may depend upon various equipment configurations and capabilities. These configurations and capabilities may determine the manner in which the case start time, the case end time, and/or the medical device identifier are recorded in the patient charting file and/or the medical device case file. For example, these values may be automatically recorded in the patient charting files and/or medical device case files or manually entered or triggered. Further, these configurations and capabilities may determine whether one or more of the case start time, the case end time, and/or the medical device identifier are available as search criteria. For example, in a system that lacks the capability to automatically record or prompt for one or more of the case start time, the case end time, and/or the medical device identifier, one or more of these values may be unavailable for use as a search criteria. Finally, these configurations and capabilities may determine the relative reliability or accuracy of the case start time, the case end time, and/or the medical device identifier as search criteria. For example, automatically recorded values may be more accurate and reliable than manually entered values.

The ePCR charting data may include a case start time and a case end time. These times may be recorded automatically in the patient charting file. In an implementation, these times may correspond to clock times on the patient charting device. For example, the case start time may be the clock time at which a patient charting application opens and initiates a new charting file and the case end time may be the clock time at which the patient charting application closes the charting file. As another example, the patient charting application may receive a case start time transmitted from a computer aided dispatch (CAD) or other dispatch server or device to the patient charting device 104 and record this time in the patient charting file. This case start time may correspond to a clock time at the CAD or dispatch server or device at the time the case is assigned to an EMS crew. As a further example, the time may correspond to a clock time on the patient charting device 104 or local computing device 160 at which the EMS crew accepts a case assignment from the CAD or other dispatch server or device. In this scenario, the patient charting device 104 or local computing device 160 may display the case assignment, prompt the user for an acceptance, and automatically record the acceptance time as the case start time in the patient charting file. If the acceptance occurs at the local computing device 160, this device may transmit the case start time to the patient charting device for recordation in the patient charting file. As yet another example, the patient charting device 122 may receive time at a patient transport destination (e.g., based on a WiFi or Bluetooth or other communicative coupling initiated at the transport destination) and automatically record this received time as the case end time. As yet a further example, the patient charting device 122 may transmit the case file to the charting data store 134 and/or to a remote computing device 165 (e.g., a hospital server or other computing device at a patient transport destination) and the patient charting device may record the time of transmission as the case end time.

In an implementation the patient charting device may receive and automatically record the case start time and/or the case end time from a geofencing application on the patient charting device 104 or the local computing device 106. For example, the geofencing application may provide a case start time to the patient charting device 104 based on the patient charting device 104 crossing a geofence around an EMS agency location or around a patient location. As another example, the geofencing application may provide a case end time to the patient charting device 104 based on the patient charting device 104 crossing a geofence around a patient location or around a patient transport destination (e.g., a hospital, a doctor's office, a dialysis center, a psychiatric center, or other care provider location). Similarly, the patient charting device may receive and automatically record the case start time and/or the case end time based on one or more GPS coordinates of the patient charting device 104.

In some implementations, the case start time may be a time of treatment (e.g., a time that defibrillation shock, drug, or other therapy was delivered to the patient by the medical device. The medical device also records times at which the device provides therapy (e.g., as a time-stamped event marker). Thus a correlation between "case start time" may include a correlation between therapy delivery times as recorded in the patient charting file and in the medical device case file. The patient charting file may receive this information from the medical device (e.g., as transmitted information) or may receive this information via an entry to the patient charting file.

In an implementation, patient charting device 104 may prompt the user to enter or confirm a case start time and/or a case end time at the ePCR user interface 122. The patient charting device 104 may record this user entered or confirmed time in the patient charting file. In this case, the recorded time may be a clock time on the patient charting device confirmed by the user or may be a time gathered from a watch or other time source separate from the patient charting device and entered into the patient charting file by the user.

For case times and other entries to the patient charting file as discussed herein, these entries may be manual entries (e.g., via a keyboard), audio entries (e.g., via a microphone and a speech recognition capability at the patient charting device 104), entries captured via an augmented reality device, and/or entries captured at a wearable device that either communicates with the patient charting device 104 or servers as the patient charting device 104.

In some scenarios, the case end time may lag behind the actual discharge of the patient from the care of EMS. For example, an EMS crew may transport a patient to a hospital, discharge the patient to the hospital, and then proceed to complete the patient chart. This lag time may be necessary operationally if the EMS crew does not have time to complete patient charting during the patient care because they do not have time to divert their attention from the patient care to the task of patient charting. Thus, even if a communicative coupling exists between the patient charting device 104 and the medical device(s) 102A-102N with a sufficient bandwidth and signal strength to transfer files between these devices, the file association may still need to occur in the cloud because the patient charting file may not be complete until sometime after the event. At this time, the relevant medical device(s) may be redeployed or powered off. Additionally, in some cases, for privacy and data protection reasons, a caregiver must manually initiate or confirm a file transfer from a medical device to a patient charting device. If the caregiver forgets to initiate this transfer, the file association can still occur in the cloud rather than based on a device to device transfer. Furthermore, the medical devices and the patient charting application may be from different vendors and may not be compatible with one another in terms of file formats. Therefore, even with an available communications channel, in the absence of an appropriate software development kit (SDK), file transfers between these devices may not be possible and may require a file association in the cloud as described herein.

The case end time may correspond to a completion of the patient charting file or a time entered by the user. In various implementations, the case start time in the patient charting file may be a time at which a caregiver arrives at the patient scene (e.g., an "at patient side time") and the case end time may be a time at which the patient is discharged from the care of EMS (e.g., a "time of patient care transfer"). The time at which the caregiver arrives at the patient scene may be the earliest time at which a medical device could be deployed for use on the patient. Thus this time may be the relevant case start time. Similarly, the time at which the patient care is transferred away from EMS is the latest time at which a medical device could be detached from the patient. Typically, upon arrival at a transport destination, such as a hospital, a patient is transferred from medical devices belonging to the EMS agency to those belonging to the hospital.

During care of the patient, the EMS crew may couple the patient to one or more medical devices. The medical device may automatically record a case start time or a case end time as a time of power on or power off or as a time at which a patient interface device is coupled to or removed from the patient. The medical device may recognize this coupling or removal based on the presence or absence of a physiologic signal from the patient or based on a sensor signal that indicates a patient connection (e.g., a closed circuit based on a proper attachment of a pair of electrodes to a patient) or based on a sensor signal indicating that a patient interface device has been removed from a package or otherwise deployed. In an implementation, a medical device and a patient charting device may communicatively couple and one or more both devices may record a case start time and/or a case end time based on a time associated with the initiation of communications. In an implementation, a medical device may request a user entry or confirmation of a case start time or a case end time.

In an implementation, the medical device(s) 102A-102N may automatically record a device identifier in the medical device case file. For example, this device identifier may be a code unique to the particular medical device (e.g., a serial number and/or model number and/or other identifying information) included as metadata with the medical device case file. In an implementation, the medical device(s) 102A-102N may communicatively couple with the patient charting device 104 and transmit the medical device identifier to the patient charting device 104. The patient charting device 104 may automatically record this identifier in the patient charting file. In an implementation, the medical device(s) 102A-102N may include the medical device identifier on an exterior housing, for example, on an affixed tag or sticker or as an embossed or engraved code. The caregiver may visually inspect this code and manually enter the code at the ePCR user interface 122. Alternatively, the caregiver may capture the code using a camera or other visual recording device and transmit the captured code to the patient charting device 104 for recordation in the patient charting file. As another option, the medical device identifier may be a bar code or QR code and the caregiver may capture the code using a scanner and transmit the captured code to the patient charting device 104 for recordation in the patient charting file. In an implementation, the patient charting device 104 and/or the local computing device 160 may include the camera and/or the scanner. As a further option, the caregiver may read and vocalize the code and the patient charting device 104 may include a microphone configured to capture the audible information and automatically record the code in the patient charting file. In an implementation, particular medical devices may be associated with a particular EMS crew and/or EMS vehicle. In such an implementation, the caregiver may provide a crew or vehicle identification to the patient charting file and the patient charting device may consult a look-up table or other reference to associated a medical device identifier with the patient charting file based on the crew or vehicle identification.

Given the various possible modes of time recordation on the patient charting device 104 and the medical device(s) 102A-102N, the reliability and accuracy of any correlation, or match, between the times in the patient charting file and the medical device case file may vary to differing degrees depending on how each device records these times. Similarly, the various possible modes of capturing and recording the medical device identifier by the patient charting device 104 may determine the reliability and accuracy of any correlation, or match between the information in the patient charting file and in the medical device case file. Therefore, a system may use one or other or both of these criteria, as exemplified in FIGS. 2B, 2C, and 2D, as search criteria for file associations.

Use of both of these criteria may be not be necessary for a small EMS agency that owns only one or a few medical devices, such as defibrillators, automated compression devices, ventilators, etc. and/or has only one or a few medical devices deployed simultaneously (e.g., for two concurrent emergencies or scheduled transports). In this case, the times may be sufficient to associate the medical device case files and the patient charting files. However, for a large EMS agency, such as an agency in a major metropolitan area, the agency may own 100-200 medical devices and may have 10-20 concurrent deployments. In this case, the times may be insufficient and the medical device identifiers may be needed in conjunction with the times for accuracy of the file associations. This situation may be particularly true for a mass casualty situation where the times would substantially overlap between patients and there may be confusion in the field regarding timely recordation of medical device identifiers. Therefore, in this case, the search criterion may require several parameters for reliable and accurate file associations. A multiple or mass casualty situation also provides another example of a situation in which a file association may need to occur in the cloud even if a communicative coupling exists between the patient charting device 104 and the medical device(s) 102A-102N with a sufficient bandwidth and signal strength to transfer files between these devices. For example, if a long range communicative coupling such as WiFi or cellular is unavailable (e.g., in an interior space, a parking garage, an urban canyon, a remote location, etc.), the devices may only be able to communicate via a short range connection such as Bluetooth^{®}. However, multiple devices within short range communications distance of one another may interfere with detection capabilities that enable an automatic transfer of files between the medical devices and the patient charting device.

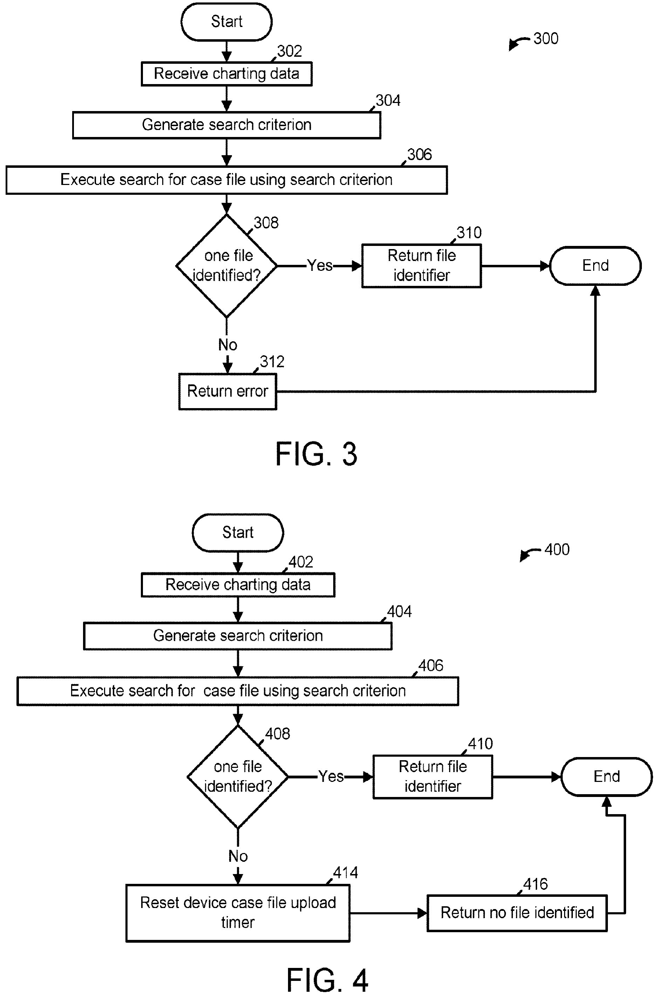

FIG. 3 illustrates one example of a file integration process 300 executed by a file integration service 130 (e.g., the file integration service 130 of FIG. 1) in some implementations. The file integration service 130 may execute the process 300 in response to a request from a calling process (e.g., the ePCR API 128 of FIG. 1) based on a file integration request from the patient charting device 104.

As shown in FIG. 3, the process 300 starts with the file integration service 130 receiving 302 charting data generated from an ePCR. This action is followed by the file integration service 130 generating 304 at least one search criterion based on the charting data and searching 306 case data stored in a case data store (e.g., the case data store 132 of FIG. 1) for a case file that satisfies the at least one search criterion. In some examples, the file integration service 130 can receive 302, generate 304, and search 306 within the process 300 by executing actions such as those executed by the file integration service 130 to receive 202a, generate 204a, and search 206a within the process 200a described above with reference to FIG. 2A.

Next, the file integration service 130 determines 308 whether the search 306 identified a single medical device case file as satisfying the search criterion. Where the file integration service 130 determines 308 that the file integration service 130 did not identify a single medical device case file, the file integration service 130 generates and returns 312 an error message to the calling process, and the process 300 ends. Where the file integration service 130 determines 308 that the file integration service 130 did identify a single medical device case file, the file integration service 130 returns 310 a message including the identified case file or an identifier thereof to the calling process, and the process 300 ends.

FIG. 4 illustrates one example of a file integration process 400 executed by a file integration service 130 (e.g., the file integration service 130 of FIG. 1) in some implementations. The file integration service 130 may execute the process 400 in response to a request from a calling process (e.g., the ePCR API 128 of FIG. 1) based on a file integration request from the patient charting device 104.

As shown in FIG. 4, the process 400 starts with the file integration service 130 receiving 402 charting data generated from an ePCR. This action is followed by the file integration service 130 generating 404 at least one search criterion based on the charting data and searching 406 case data stored a case data store (e.g., the case data store 132 of FIG. 1) for a medical device case file that satisfies the at least one search criterion. In some examples, the file integration service 130 can receive 402, generate 404, and search 406 within the process 400 by executing actions such as those executed by the file integration service 130 to receive 202a, generate 204a, and search 206a within the process 200a described above with reference to FIG. 2A.

Next, the file integration service 130 determines 408 whether the search 406 identified a single medical device case file as satisfying the search criterion. Where the file integration service 130 determines 408 that search did not identify a single medical device case file, the file integration service 130 resets 414 resets a timer and transmits 416 a return message to the calling process that indicates medical device case file was not identified. Where the file integration service 130 determines 408 that a single medical device case file was identified, the file integration service 130 returns 410 the identified medical device case file or an identifier thereof to the calling process, and the process 400 ends.

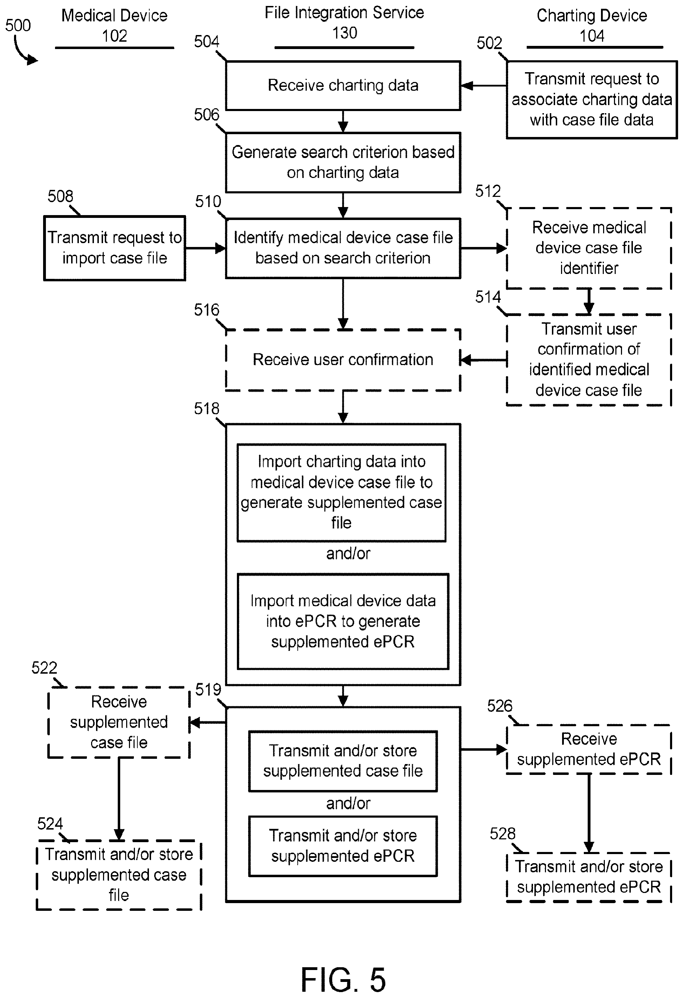

FIG. 5 illustrates one example of an integration process 500 executed by a medical device, a file integration service, and a charting device (e.g., a medical device 102, the file integration service 130 and the charting device 104 of FIG. 1) in some implementations. As shown in FIG. 5, operations rendered with dashed line borders may not present in some examples.

Within the process 500, the charting device transmits 502 a request message to the file integration service 130. This request message can include a request to associate charting data (e.g., an ePCR or a portion thereof) to case data (e.g., a medical device case file or a portion thereof). The request message can include the charting data and/or an identifier of charting data (e.g., an identifier of the charting data within a charting data store, such as the charting data store 134 of FIG. 1). In some examples, the charting device can transmit the request message and/or the charting data to the file integration service 130 via one or more messages to an ePCR API (e.g., the ePCR API 128 of FIG. 1) implemented by a server (e.g., a server of the server(s) 108 of FIG. 1). Alternatively or additionally, the charting device can store the charting data within the charting data store via the ePCR API as part of transmitting 502 the request message.

Continuing the process 500, the file integration service 130 receives 504 the charting data from the ePCR API or the charting data store. This action is followed by the file integration service 130 generating 506 at least one search criterion based on the charting data. In some examples, the file integration service 130 can receive 504 and generate 506 within the process 500 by executing actions such as those executed by the file integration service 130 to receive 202a and generate 204a within the process 200a described above with reference to FIG. 2A.

As shown in FIG. 5, the medical device transmits 508 one or more request messages to a case API (e.g., the case API 126) implemented by a server (e.g., a server of the server(s) 108 of FIG. 1). The request messages can include one or more requests to import one or more case files into a case data store (e.g., the case data store 132 of FIG. 1). The request messages can include the case files to be imported. In response to receiving the request messages, the case API can receive the case files, parse the case files to retrieve case data from the case files, store the case data and/or the case files in the case data store, and transmit one or more response messages to the medical device that indicate results of processing the request messages.

Continuing the process 500, the file integration service 130 identifies 510 case data associated with the imported case files as satisfying the at least one search criterion. In some examples, the file integration service 130 can identify 510 within the process 500 by executing actions such as those executed by the file integration service 130 in identifying 208a within the process 200a described above with reference to FIG. 2A. The file integration service 130 can further generate and transmit a confirmation request including one or more identifiers of case files and/or other information and/or metadata descriptive of the case files to the charting device as part of identifying 510 the case file. The one or more identifiers of the case files can include, for example, one or more timestamps indicating when the case files were created by the medical devices and/or identifiers of medical devices that generated the case files (e.g., an identifier associated with a bar code, QR code, serial number, IP address, etc.).

In an implementation, identifying 510 the medical device case file may include identifying an integrated data source encounter structure or identifying an integrated data source encounter structure and parsing the integrated data source encounter structure to extract an EMS portion of the integrated data source encounter structure. Identifying the integrated data source encounter structure may include utilizing a search criterion specifying a time range associated with the patient charting data and one or more medical device identifiers associated with the patient encounter. The search criterion may require that a portion or all of the medical device identifiers for the medical device case files be associated with the patient charting data. Parsing the integrated data source encounter structure may include identifying and accessing a portion of the integrated data source encounter structure based on a start time and a stop time of the EMS treatments and/or based on the medical device identifiers for the medical devices associated with the EMS treatment.

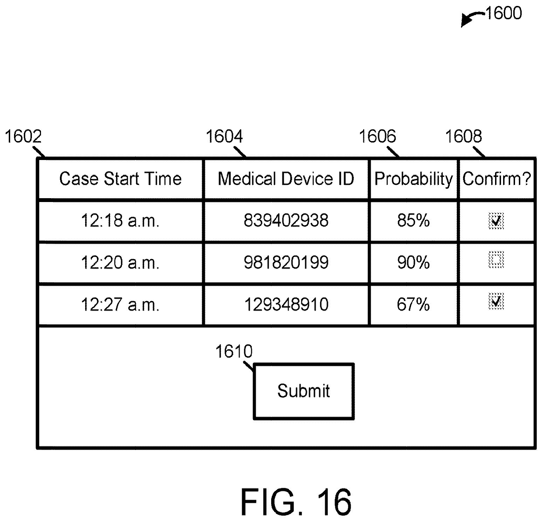

Continuing with the process 500, the charting device receives 512 the confirmation request including the identifiers and/or other metadata of the case files from the file integration service 130 and prompts a user (e.g. the healthcare provider 118 of FIG. 1) to confirm whether the case files are descriptive of the same patient encounter as the charting data (e.g., a manual confirmation of the association between the two files to enable a record integration). In some examples, the charting device prompts the user via an ePCR user interface (e.g., the ePCR user interface 122 of FIG. 1) using the identifiers. FIG. 16 illustrates one example of a user interface screen 1600 that the ePCR user interface is configured to render in response to receiving a confirmation request. As shown in FIG. 16, the screen 1600 includes columns of case controls 1602, 1604, and 1606; a column of confirmation controls 1608; and a submit control 1610. The case controls 1602, 1604, and 1606 are configured to display the identifiers of and information regarding the case files identified in the confirmation request. More specifically, each of the case controls 1602 is configured to display a case start time, each of the case controls 1604 is configured to display an identifier of the medical device that generated the case file, and each of the case controls 1606 is configured to display an indicator of a degree to which the case file satisfies a predetermined criterion for associating the case file with the currently selected ePCR. The file integration service 130 may calculate or determine these file association indicators. The file integration service 130 may provide the file association indicators to a user interface (e.g., the ePCR user interface 122) along with a file association confirmation prompt in order to aid the user in evaluating whether or not the identified files are associated with the same patient encounter. For instance, in some examples, the file integration service 130 may calculate file association indicators based on a calculated difference between a timestamp associated with the currently selected ePCR and a timestamp of each case file. To make the file association indicators easy to interpret, in some examples, the ePCR user interface 122 may map the calculated difference to one member of a set of colors for which each color is associated with a range of difference values. For instance, in one example green is associated with differences between 1 second and 4 minutes, yellow is associated with differences between 4 minutes and 10 minutes, and red is associated with differences greater than 10 minutes. In an implementation, the ePCR user interface 122 may use one or more file association indicators, such as, for example, but not limited to the color (e.g., as discussed above), a number, a word, a graphic or a combination thereof.

It should be noted that parameters other than timestamps can be used to calculate file association indicators. For instance, in some examples, the color of each file association indictor is determined from a calculated distance (e.g., cosine distance) between a first feature vector associated with the case file and a second feature vector associated with the currently selected ePCR. The members of these feature vectors can be based on, for example, the parameters of the case file and currently selected ePCR that are compared via the predetermined relationship specified with the search criterion that did not result in a single identified case file to associate with the charting file.

In some examples, the ePCR user interface orders the rows of case files presented in the screen 1600 by increasing or decreasing differences or distances. The confirmation controls 1608 are configured to receive input from a user 118 of the ePCR user interface 122 confirming or rejecting an association with the case file identified by the row in which the confirmation control 1608 resides with the currently selected ePCR (e.g., the case file and the currently selected ePCR are associated with a same patient for a same patient encounter). The submit control 1610 is configured to receive input indicating that the user has confirmed or not confirmed the case files as desired.

Returning to the process 500, where the ePCR user interface receives input confirming the case files (e.g., selection of the submit control 1610), the ePCR user interface transmits 514 a confirmation response to the file integration service 130 (e.g., via the ePCR API). The file integration service 130 receives 516 the confirmation response.

In some examples, the file integration service 130 integrates 518 the charting data with case data to generate the integrated patient encounter record (e.g., a supplemented case data file, a supplemented charting data file, a new file that includes charting data and case data, a file integration pointer, a case data file and/or charting data file that includes integration information, as described above with regard to FIG. 2A).

Continuing the process 500, the file integration service 130 stores and/or transmits 519 the integrated case and charting data to the medical device and/or the charting device. For instance, in some examples, the file integration service 130 stores the supplemented case files in the case data store and/or transmits 519 the supplemented case files to the medical device. Alternatively or additionally, in some examples, the file integration service 130 stores the supplemented ePCR in the charting data store and/or transmits 519 the supplemented ePCR to the charting device.

Where the file integration service 130 transmits 519 the supplemented case files to the medical device 102, the medical device 102 receives 522 the supplemented case files. In an implementation, the medical device 102 may locally store 524 and/or transmit the supplemented case files. In various implementations, the medical device 102 may transmit the supplemented case file to one or more of the patient charting device 104, one or more of the server(s) 108, the remote computing device 106 and/or 165, and the local computing device 160. Where the file integration service 130 transmits 519 the supplemented ePCR to the charting device, the charting device receives 526 the supplemented ePCR. In an implementation, the patient charting device 104 may locally store 528 and/or transmit the supplemented ePCR. In various implementations, the patient charting device 104 may transmit the supplemented case file to one or more of the medical device 102, one or more of the server(s) 108, the remote computing device 106 and/or 165, and the local computing device 160.

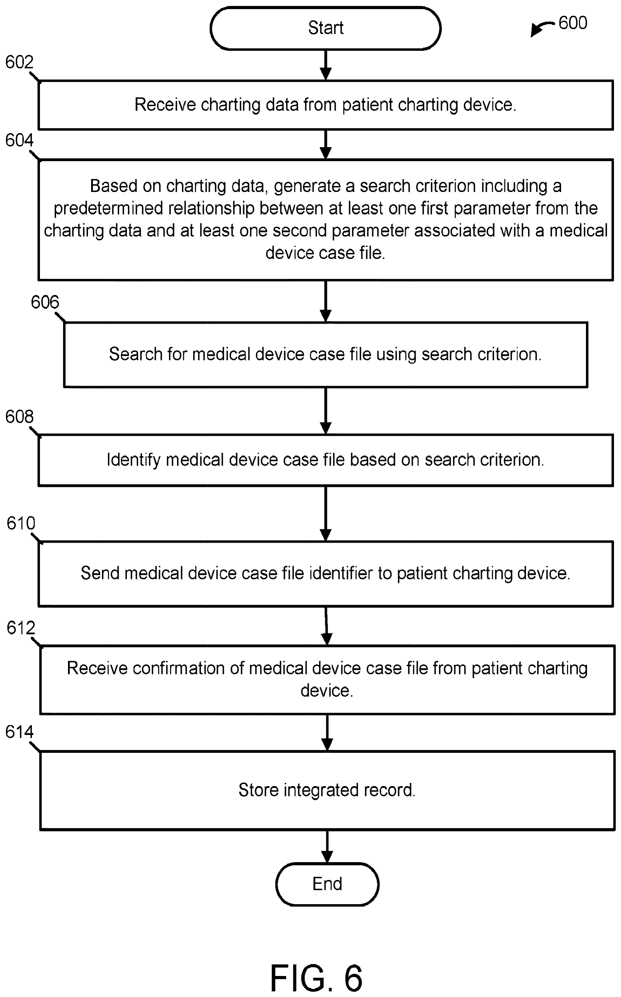

FIG. 6 illustrates one example of an integration process 600 executed by a file integration service and a charting device (e.g., the file integration service 130 and the charting device 104 of FIG. 1) in some implementations.

As shown in FIG. 6, the process 600 starts with the file integration service 130 receiving 602 charting data from the charting device. This action is followed by the file integration service 130 generating 604 at least one search criterion based on the charting data, searching 606 case data stored in a case data store (e.g., the case data store 132 of FIG. 1) for a medical device case file that satisfies the at least one search criterion, and identifying 608 the medical device case file as being associated with the charting data based on the at least one search criterion. In associating the case file with the charting data file, the file integration service 130 maps these files to one another based on their common association with the same patient during a same patient encounter with this patient. The files mapped to one another were both generated during this same patient encounter and the file integration service 130 identifies this correspondence between these files. In some examples, the file integration service 130 can receive 602, generate 604, search 606, and identify 608 within the process 600 by executing actions such as those executed by the file integration service 130 to receive 202a, generate 204a, search 206a, and identify 208a within the process 200a described above with reference to FIG. 2A.

Next, the file integration service 130 transmits 610 an identifier of the identified case file to the charting device. This identifier can include a timestamp indicating when the case file was generated by the medical device. In some examples, the file integration service 130 transmits 610 the timestamp to an ePCR user interface (e.g., the ePCR user interface of FIG. 1) hosted by the medical device. In certain examples, the charting device receives the identifier of the case file from the file integration service 130 and prompts a user (e.g. the healthcare provider 118 of FIG. 1) to confirm whether the case file is descriptive of the same patient encounter as the charting data. In some examples, the charting device prompts the user via the ePCR user interface. Where the ePCR user interface receives input confirming the case file, the ePCR user interface transmits a confirmation message to the file integration service 130 (e.g., via an ePCR API such as the ePCR API 128 of FIG. 1).

Continuing the process 600, the file integration service 130 receives 612 the confirmation message from the patient charting device, stores 614 an integrated patient encounter record that may include charting data and case data, and the process 600 ends. In some examples, the file integration service 130 can store 614 the integrated patient encounter record within the process 600 by executing actions such as those executed by the file integration service 130 to store 212a the integrated patient encounter record within the process 200a described above with reference to FIG. 2A.

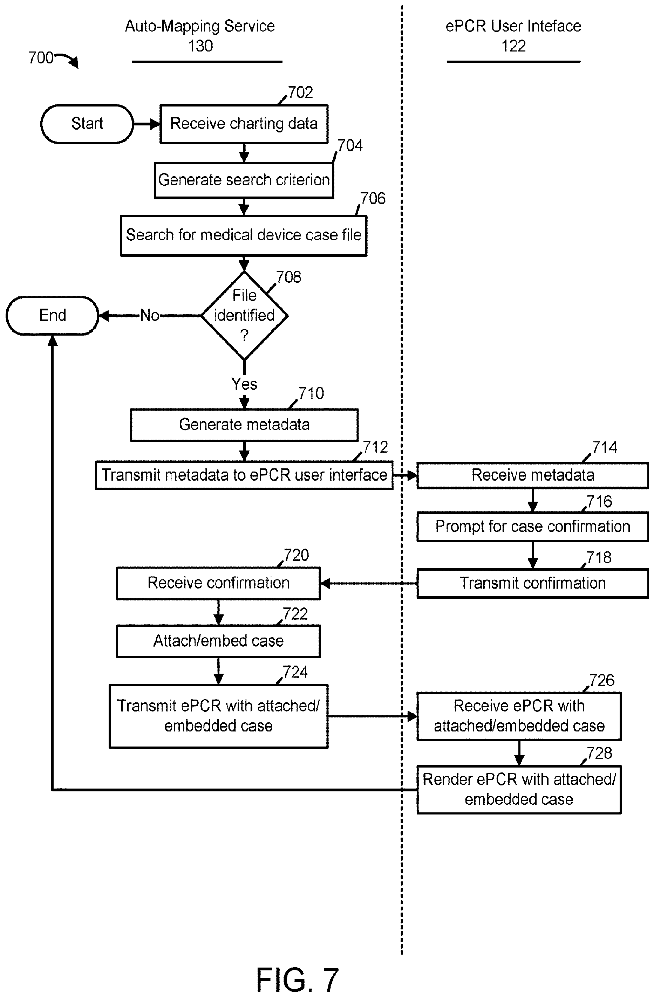

FIG. 7 illustrates one example of an integration process 700 executed by a file integration service and an ePCR user interface (e.g., the file integration service 130 and the ePCR user interface 122 of FIG. 1) in some implementations.

As shown in FIG. 7, the process 700 starts with the file integration service 130 receiving 702 charting data. This action is followed by the file integration service 130 generating 704 at least one search criterion based on the charting data and searching 706 case data stored in a case data store (e.g., the case data store 132 of FIG. 1) for a medical device case file that satisfies the at least one search criterion. In some examples, the file integration service 130 can receive 702, generate 704, and search 706 within the process 700 by executing actions such as those executed by the file integration service 130 to receive 202a, generate 204a, and search 206a within the process 200a described above with reference to FIG. 2A.

Next, the file integration service 130 determines 708 whether the search resulted in identification of one or more case files that satisfy the at least one search criterion. Where the file integration service 130 determines 708 that no medical device case files were identified that satisfy the at least one search criterion, the process 700 ends. Where the file integration service 130 determines 708 that one or more medical device case files were identified that satisfy the at least one search criterion, the file integration service 130 generates 710 metadata and/or other information descriptive of the identified medical device case files. This metadata and/or other information can include one or more identifiers of the identified case files, such as one or more timestamps indicating when the identified case files were created by one or more medical devices, one or more identifiers of the one or more medical devices that created the identified case files, and/or one or more identifiers of one or more charting devices coupled with the medical devices during creation of the identified case files, among other identifiers.

Continuing the process 700, the file integration service 130 transmits 712 the metadata and/or other information to the ePCR user interface over a network (e.g., the network 112 of FIG. 1). In some examples, the file integration service 130 transmits the metadata and/or other information via messages generated by one or more ePCR API calls (e.g., calls supported by the ePCR API 128 of FIG. 1). These messages can include a confirmation request.

The ePCR user interface receives 714 the metadata and/or other information and renders 716 a prompt for each case file described in the metadata and/or other information. For instance, the ePCR user interface can render a timestamp and/or a medical device identifier for each case file within its associated prompt. Each of the one or more prompts can be configured to receive input confirming that its associated case file is descriptive of operation of a medical device coupled to a patient (e.g., the patient 116 of FIG. 1) during an encounter documented by the charting data. In response to receiving input confirming a case file, the ePCR user interface transmits 718 a confirmation response to the file integration service 130 (e.g., via the ePCR API). The confirmation response can include an identifier of each confirmed case file.

Next, the file integration service 130 receives 720 the confirmation response and parses the confirmation response to retrieve identifiers of case files stored therein. The file integration service 130 attaches and/or embeds 722 the case files in the charting data. The case files can be embedded and/or attached in whole or in part. Where the case files are embedded, case data stored within the case files can be stored in fields of the ePCR containing the charting data.

Continuing the process 700, the file integration service 130 transmits 724 the ePCR containing the charting data and the attached/embedded case file to the ePCR user interface. The ePCR user interface receives 726 the ePCR with the attached/embedded case and renders 728 the ePCR for review and manipulation by the healthcare provider.

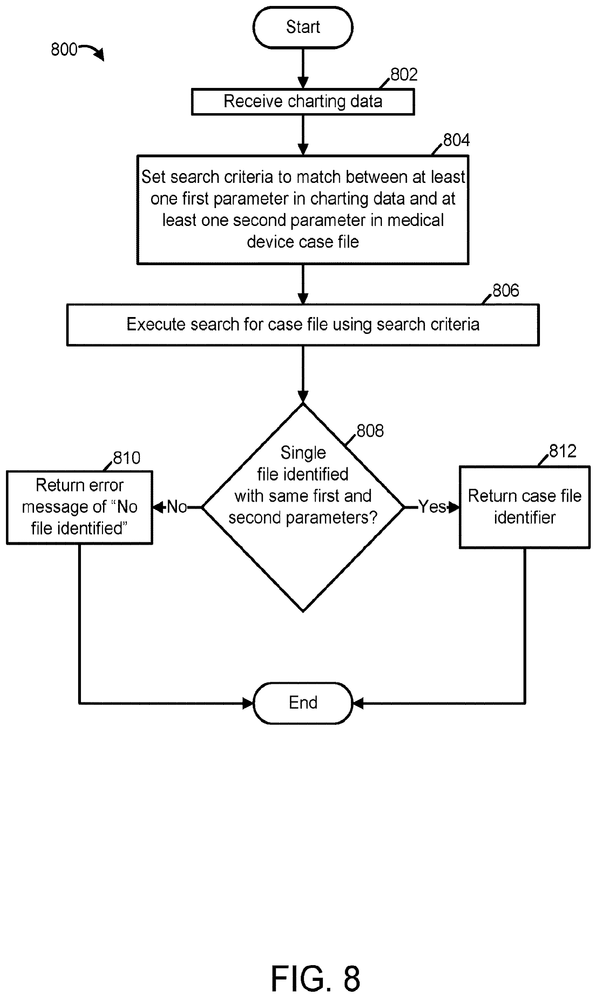

FIG. 8 illustrates one example of a file integration process 800 executed by a file integration service 130 (e.g., the file integration service 130 of FIG. 1) in some implementations. The file integration service 130 may execute the process 800 in response to a request from a calling process (e.g., the ePCR API 128 of FIG. 1) based on a file integration request from the patient charting device 104.

As shown in FIG. 8, the process 800 starts with the file integration service 130 receiving 802 charting data generated from an ePCR. This action is followed by the file integration service 130 generating 804 at least one search criterion based on the charting data and searching 806 case data stored in a case data store (e.g., the case data store 132 of FIG. 1) for a medical device case file that satisfies the at least one search criterion. In some examples, the file integration service 130 can receive 802, generate 804, and search 806 within the process 800 by executing actions such as those executed by the file integration service 130 to receive 202a, generate 204a, and search 206a within the process 200a described above with reference to FIG. 2A. However, within the process 800, the file integration service 130 generates 804 at least one search criterion that specifies a first, single parameter in the charting data match a second, single parameter associated with the case file and uses this search criterion to search 806.

Next, the file integration service 130 determines 808 whether the search 806 identified a single medical device case file as satisfying the search criterion. Where the file integration service 130 determines 808 that a single medical device case file was not identified, the file integration service 130 generates and returns 810 an error message indicating no file identified to the calling process, and the process 800 ends. Where the file integration service 130 determines 808 that the search identified a single medical device case file, the file integration service 130 generates and returns 812 the identified case file or an identifier thereof to the calling process, and the process 800 ends.

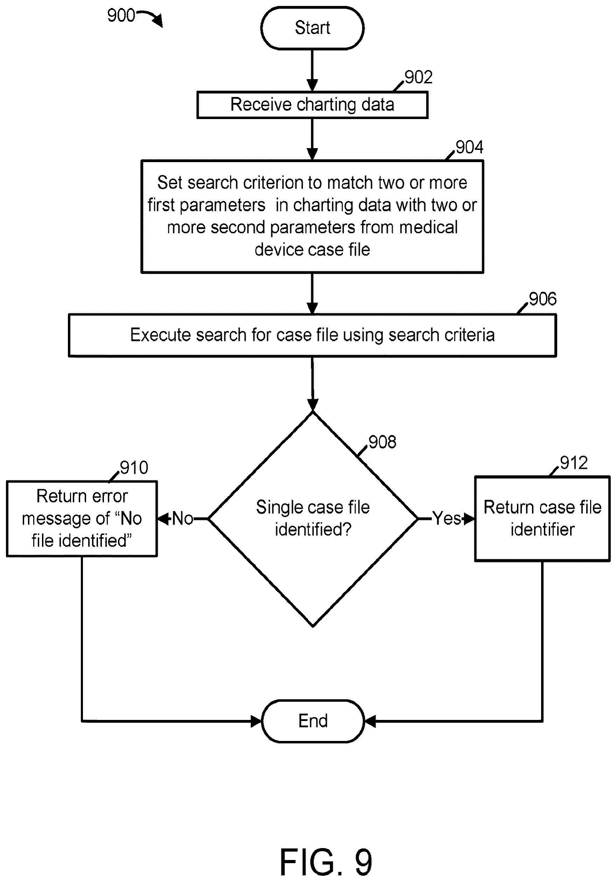

FIG. 9 illustrates one example of a file integration process 900 executed by a file integration service 130 (e.g., the file integration service 130 of FIG. 1) in some implementations. The file integration service 130 may execute the process 900 in response to a request from a calling process (e.g., the ePCR API 128 of FIG. 1) based on a file integration request from the patient charting device 104.

As shown in FIG. 9, the process 900 starts with the file integration service 130 receiving 902 charting data generated from an ePCR. This action is followed by the file integration service 130 generating 904 at least one search criterion based on the charting data and searching 906 case data stored in a case data store (e.g., the case data store 132 of FIG. 1) for a medical device case file that satisfies the at least one search criterion. In some examples, the file integration service 130 can receive 902, generate 904, and search 906 within the process 900 by executing actions such as those executed by the file integration service 130 to receive 202a, generate 204a, and search 206a within the process 200a described above with reference to FIG. 2A. However, within the process 900, the file integration service 130 generates 904 at least one search criterion that specifies a plurality of first parameters in the charting data match a plurality of second parameters associated with the case file and uses this search criterion to search 906.

Next, the file integration service 130 determines 908 whether the search 906 identified a single medical device case file as satisfying the search criterion. Where the file integration service 130 determines 908 that a single medical device case file was not identified, the file integration service 130 generates and returns 910 an error message to the calling process, and the process 900 ends. Where the file integration service 130 determines 908 that a single medical device case file was identified, the file integration service 130 generates and returns 912 the identified medical device case file or an identifier thereof to the calling process, and the process 900 ends.

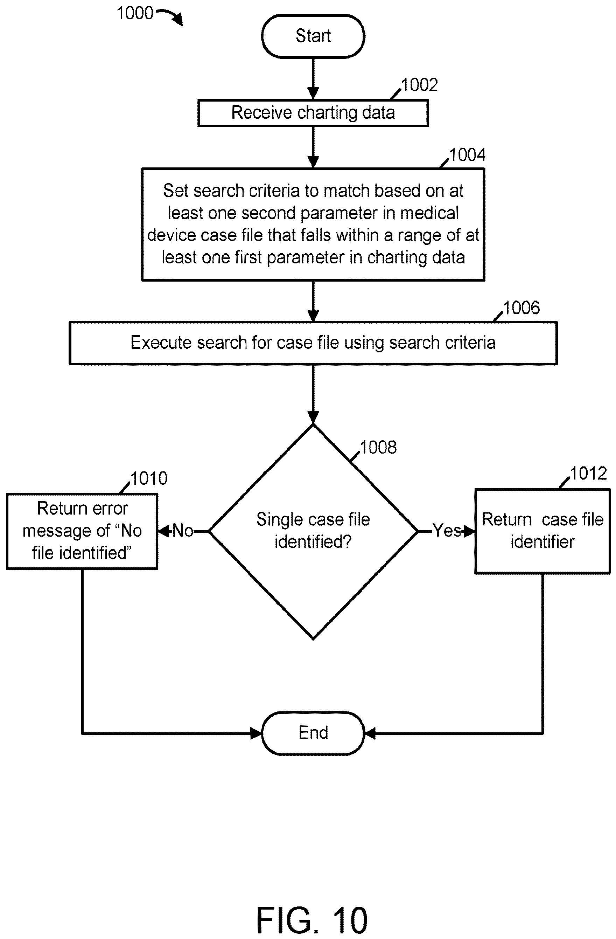

FIG. 10 illustrates one example of a file integration process 1000 executed by a file integration service 130 (e.g., the file integration service 130 of FIG. 1) in some implementations. The file integration service 130 may execute the process 1000 in response to a request from a calling process (e.g., the ePCR API 128 of FIG. 1) based on a file integration request from the patient charting device 104.

As shown in FIG. 10, the process 1000 starts with the file integration service 130 receiving 1002 charting data generated from an ePCR. This action is followed by the file integration service 130 generating 1004 at least one search criterion based on the charting data and searching 1006 case data stored in a case data store (e.g., the case data store 132 of FIG. 1) for a medical device case file that satisfies the at least one search criterion. In some examples, the file integration service 130 can receive 1002, generate 1004, and search 1006 within the process 1000 by executing actions such as those executed by the file integration service 130 to receive 202a, generate 204a, and search 206a within the process 200a described above with reference to FIG. 2A. However, within the process 1000, the file integration service 130 generates 1004 at least one search criterion that specifies at least one second parameter associated with the case file be within a range of at least one first parameter in the charting data and uses this search criterion to search 1006.

Next, the file integration service 130 determines 1008 whether the search 1006 identified a single medical device case file as satisfying the search criterion. Where the file integration service 130 determines 1008 that a single medical device case file was not identified, the file integration service 130 generates and returns 1010 an error message to the calling process, and the process 1000 ends. Where the file integration service 130 determines 1008 that a single medical device case file was identified, the file integration service 130 generates and returns 1012 the identified case file or an identifier thereof to the calling process, and the process 1000 ends.

FIG. 11 illustrates one example of a file integration process 1100 executed by a file integration service 130 (e.g., the file integration service 130 of FIG. 1) in some implementations. The file integration service 130 may execute the process 1100 in response to a request from a calling process (e.g., the ePCR API 128 of FIG. 1) based on a file integration request from the patient charting device 104. As shown in FIG. 11, operations rendered with dashed line borders may not present in some examples.

As shown in FIG. 11, the process 1100 starts with the file integration service 130 receiving 1102 charting data generated from an ePCR. This action is followed by the file integration service 130 generating 1104 at least one search criterion based on the charting data and filtering 1106 case data stored in a case data store (e.g., the case data store 132 of FIG. 1) for a medical device case file that satisfies the at least one search criterion. In some examples, the file integration service 130 can receive 1102, generate 1104, and filter 1106 within the process 1100 by executing actions such as those executed by the file integration service 130 to receive 202a, generate 204a, and search 206a within the process 200a described above with reference to FIG. 2A. However, within the process 1100, the file integration service 130 searches the case data for the case file by filtering 1106 (e.g., via a query) the case data store using an initial search criterion and, in so doing, identifies a plurality of candidate case files. In some examples, the initial search criterion employed by the filter specifies that a second parameter must fall within a range of a first parameter. In these examples, the file integration service 130 can be configured to generate 1108 a supplemental search criterion that specifies a match between the second parameter and the first parameter. In an implementation, the match may be an equality.

Next, the file integration service 130 generates 1108 at least one supplemental search criterion. In some examples, the at least one supplemental search criterion specifies at least one supplemental predetermined relationship between at least one first additional parameter from charting data and at least one second additional parameter associated with candidate case files. For example, where the at least one supplemental relationship is hardcoded, the file integration service 130 generates 1108 the at least one supplemental search criterion by identifying at least one first additional parameter from the charting data and associating the at least one first additional parameter with the at least one supplemental relationship. Alternatively or additionally, where the at least one supplemental relationship is soft-coded, the file integration service 130 generates 1108 the at least one supplemental search criterion by first identifying the next preferred predetermined relationship by rank within a criteria data store (e.g., the criteria data store 136 of FIG. 1) and next identifying the at least one first additional parameter as specified in the next preferred predetermined relationship from the charting data and associating the at least one first additional parameter with the at least one supplemental relationship.

Continuing the process 1100, the file integration service 130 searches 1110 the candidate case data for a medical device case file that matches the at least one supplemental search criterion. In some examples, the file integration service 130 can search 1110 within the process 1100 by executing actions such as those executed by the file integration service 130 to search 206a within the process 200a described above with reference to FIG. 2A. However, within the process 1100, the file integration service 130 can restrict its search 1110 to case data from the candidate case files. For instance, the file integration service 130 can identify a candidate case file as an associated file where an additional parameter associated with the candidate case file, stored in the case data, satisfies the supplemental relationship with the additional parameter of the charting data stored in the at least one supplemental search criterion.

Next, the file integration service 130 determines 1112 whether a single medical device case file was identified by the search. Where the file integration service 130 determines 1112 that a single medical device case file was identified, the file integration service 130 generates and returns 1114 the identified case file or an identifier thereof to the calling process, and the process 1100 ends. Where the file integration service 130 determines 1112 that a single medical device case file was not identified, the file integration service 130 iterates a counter and determines 1116 whether the counter has transgressed a configurable constraint to the number of search iterations acceptable in a single instance of the process 1100. Where the file integration service 130 determines 1116 that the counter has not transgressed the constraint, the file integration service 130 generates 1108 another supplemental search criterion and the process 1100 continues. Where the file integration service 130 determines 1116 that the counter has transgressed the constraint, the file integration service 130 returns 1118 an error message to the calling process, and the process 1100 ends.

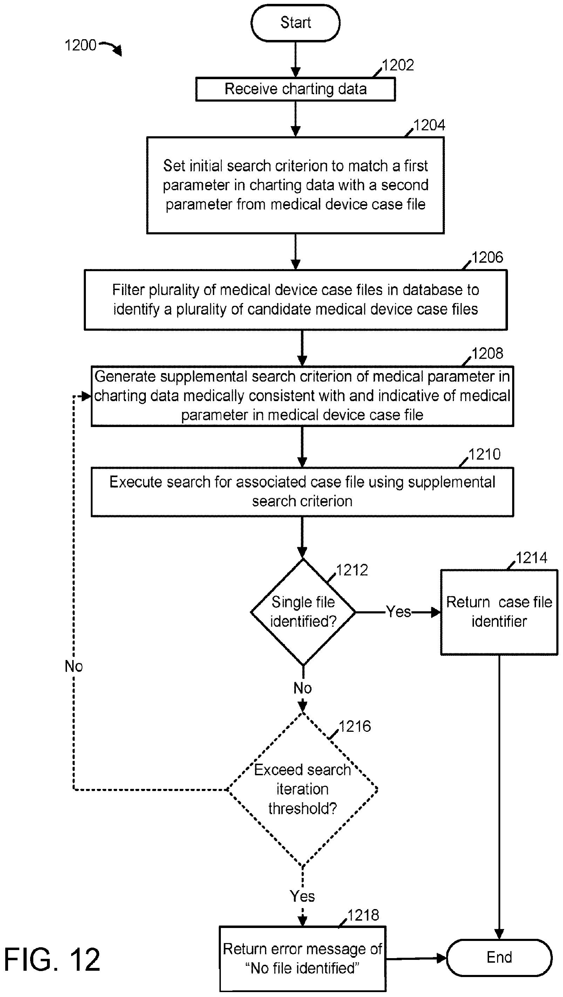

FIG. 12 illustrates one example of a file integration process 1200 executed by a file integration service 130 (e.g., the file integration service 130 of FIG. 1) in some implementations. The file integration service 130 may execute the process 1200 in response to a request from a calling process (e.g., the ePCR API 128 of FIG. 1) based on a file integration request from the patient charting device 104. As shown in FIG. 12, operations rendered with dashed line borders may not present in some examples.

As shown in FIG. 12, the process 1200 starts with the file integration service 130 receiving 1202 charting data generated from an ePCR. This action is followed by the file integration service 130 generating 1204 at least one search criterion based on the charting data and filtering 1206 (e.g., via a query) case data stored in a case data store (e.g., the case data store 132 of FIG. 1) using the at least one search criterion to identify a plurality of candidate case files. In some examples, the file integration service 130 can receive 1202, generate 1204, and filter 1206 within the process 1200 by executing actions such as those executed by the file integration service 130 to receive 1102, generate 1104, and filter 1106 within the process 1100 described above with reference to FIG. 11. However, within the process 1200, the initial search criterion employed by the filter includes an initial predetermined relationship that specifies a match between first and second non-medical parameters. These non-medical parameters can include case start time, medical device identifier, and/or patient identifiers. In an implementation, the match may be an equality.

Next, the file integration service 130 generates 1208 at least one supplemental search criterion. In this example, the at least one supplemental search criterion specifies that at least one first medical parameter in charting data be medically consistent with and indicative of at least one second medical parameter in a case file. In these examples, the at least one first medical parameter can include a patient complaint, a medic impression, a drug administration, a vital sign, a physiological measurement, a treatment provided to the patient, a symptom of chest pain, an ECG with ST elevation, and/or a STEMI diagnosis. Further, in these examples, the at least one second medical parameter can include a type of physiological measurement, a value of a physiological measurement, a medical treatment (e.g., a defibrillation shock), and an alarm.

Continuing the process 1200, the file integration service 130 searches 1210 the candidate case data, determines 1212 whether the search identified a single medical device case file, and returns 1214 the identified case file or an identifier thereof to the calling process where a single medical device case file was identified. Where a single medical device case file was not identified, the file integration service 130 determines 1216 whether a search limit has been exceeded, returns 1218 an error message indicating no identification of a single medical device case file to the calling process where the search limit has been exceeded, and returns to generate 1208 at least one supplemental search criterion where the search limit has not been exceeded. In some examples, the file integration service 130 can search 1210, determine 1212, return 1214, determine 1216, and return 1218 within the process 1200 by executing actions such as those executed by the file integration service 130 to search 1110, determine 1112, return 1114, determine 1116, and return 1118 within the process 1100 described above with reference to FIG. 11.

As explained above, the process 1200 generates 1208 at least one supplemental search criterion that specifies at least one medical parameter in the charting data be medically consistent with and indicative of at least one medical parameter in a case file. In this example, the at least one predetermined relationship specifies that the charting data be "medically consistent with and indicative of" at least one medical parameter in the case file. As such, in this example, the file integration service 130 evaluates the predetermined relationship while searching 1210 by executing one or more specialized heuristic, statistical, and/or machine learning processes that compare charting data and case data. Table 1 provides charting and case data that satisfy the "medically consistent with and indicative of" predetermined relationship according to some examples not limiting of the disclosure.

| TABLE 1 - MEDICAL DATA | | | |
|---|---|---|---|
| A | B | C | D |
| Primary Charting Data | Secondary Charting Data | Primary Medical Device Data | Secondary Medical Device Data |
| patient complaint of chest pain | drug administration (aspirin and/or nitroglycerin) | 12 lead ECG | shock administration |
| patient complaint of difficulty breathing and/or medic impression of respiratory distress | drug administration (albuterol, steroids, epinephrine) airway placement | pulse oximetry capnography | 12 lead ECG alarms (heart rate, respiratory rate, pulse oximetry, blood pressure) |
| unresponsive patient | airway administered | capnography | 12 lead ECG |

More specifically, as shown in Table 1 columns A and B list primary and secondary charting data that is medically consistent with and indicative of the primary and secondary medical device data listed on columns C and D. In some examples, the file integration service 130 can generate 1208 supplemental search criteria specifying that one or more case data parameters found while searching 1212 must be medically consistent with and indicative of the primary charting data and the secondary charting data. Alternatively, in some examples, the file integration service 130 can generate 1208 a first supplemental search criterion specifying that one or more case data parameters found while searching 1212 must be medically consistent with and indicative of the primary charting data. Where no single medical device case file is identified based on the first supplemental search criterion during searching 1212, the file integration service 130 can generate 1208 a second supplemental search criterion specifying that one or more case data parameters found while searching must be medically consistent with and indicative of the secondary charting data. In an implementation, this supplemental search criterion may improve or confirm an automated association of the medical device case file with the patient charting record and/or may be provided to a caregiver for manual confirmation of the association between the files. For example, if the charting data and the medical device data are medically inconsistent, the system may flag this inconsistency for the caregiver as a possible file identification error and require a manual confirmation or override to associate the files. An example of inconsistent data may be, for example, patient charting data indicating a responsive patient and medical device case file data indicating airway administration. Another example of inconsistent data may be, for example, patient charting data indicating an adult patient and medical device case file data indicating a pediatric defibrillation setting on a defibrillator. A further example of inconsistent data may be, for example, patient charting data indicating high blood pressure and/or high heart rate and medical device case file data indicating a blood pressure and/or heart rate within a normal range according to a medical protocol.

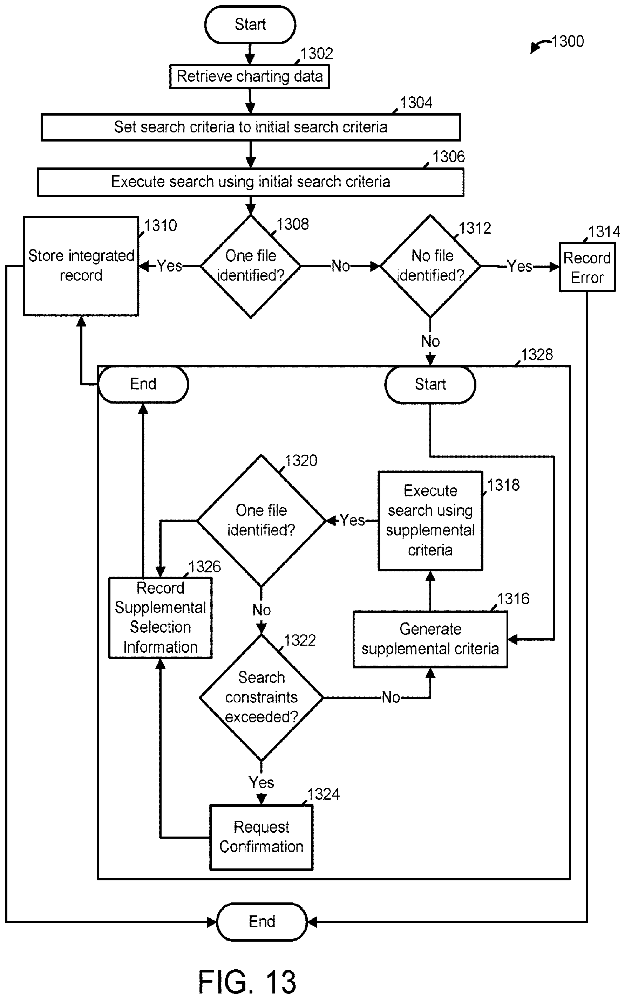

FIG. 13 illustrates one example of a file integration process 1300 executed by a file integration service 130 (e.g., the file integration service 130 of FIG. 1) in some implementations.

As shown in FIG. 13, the process 1300 starts with the file integration service 130 receiving 1302 charting data generated from an ePCR. This action is followed by the file integration service 130 generating 1304 at least one search criterion based on the charting data and searching 1306 case data stored in a case data store (e.g., the case data store 132 of FIG. 1) for a medical device case file that satisfies the at least one search criterion. In some examples, the file integration service 130 can receive 1302, generate 1304, and search 1306 within the process 1300 by executing actions such as those executed by the file integration service 130 to receive 202a, generate 204a, and search 206a within the process 200a described above with reference to FIG. 2A. However, within the process 1300, the file integration service 130 generates 1304 at least one initial search criterion. In at least some examples, this initial search criterion includes a predetermined relationship that specifies a time range between a first and second parameter. In these examples, the time range can be longer where the file integration service 130 is configured to generate 1316 supplemental search criteria within the process 1328 to include an additional parameter other than a medical device identifier. In other words, the time range can be shorter where the file integration service 130 is configured to generate 1316 supplemental search criteria with a medical device identifier as an additional parameter.

Next, the file integration service 130 determines 1308 whether the search 1306 identified a single medical device case file as satisfying the search criterion. Where the file integration service 130 determines 1308 that a single medical device case file was identified, the file integration service 130 stores 1310 an integrated patient encounter record (e.g., a supplemented case data file, a supplemented charting data file, a new file that includes charting data and case data, a file integration pointer, a case data file and/or charting data file that includes integration information) and the process 1300 ends. Where the file integration service 130 determines 1308 that no single medical device case file was identified, the file integration service 130 determines 1312 whether no identification of a single device case file. Where the file integration service 130 determines 1312 no identification of a single device case file, it records 1314 an error indicating no identification of a single device case file, and the process 1300 ends.

Where the file integration service 130 determines 1312 that multiple candidate case files were identified, the file integration service 130 identifies 1328 a single case file based on supplemental selection information. For instance, in some examples, the file integration service 130 identifies 1328 the single case file by executing an identification process that generates 1316 supplemental search criteria, searches 1318 for a medical device case file using the supplemental search criteria, and determines 1320 whether the search 1318 identified a single case file as satisfying the search criterion. In some examples, the file integration service 130 can generate 1316, search 1318, and determine 1320 within the process 1300 by executing actions such as those executed by the file integration service 130 to generate 1108, search 1110, and determine 1112 within the process 1100 described above with reference to FIG. 11.

Continuing the identification process, where the file integration service 130 determines 1320 that a single medical device case file was identified, the file integration service 130 records 1326 supplemental selection information identifying the medical device case file, and the identification process ends. Where the file integration service 130 determines 1320 that a single medical device case file was not identified, the file integration service 130 iterates a counter and determines 1322 whether the counter has transgressed a configurable constraint to the number of search iterations acceptable in a single instance of the identification process. Where the file integration service 130 determines 1322 that the counter has not transgressed the constraint, the file integration service 130 generates 1316 another supplemental search criterion, and the identification process continues. Where the file integration service 130 determines 1322 that the counter has transgressed the constraint, the file integration service 130 requests 1324 confirmation of a single medical device case file by transmitting a confirmation request to a charting device (e.g., the charting device 104 of FIG. 1). This confirmation request can include identifiers of the multiple, unresolved candidate case files. These identifiers can include descriptive information about the contents of the candidate case files (e.g., timestamps, identifiers of medical devices and charting devices, etc.) and/or a metric indicating the similarity between parameters associated with the case file and parameters of charting data within the ePCR. The charting device and the file integration service 130 can interoperate with one another to resolve the candidate case files to a single case file using the actions 512-516 described above with reference to FIG. 5. Upon receipt of the confirmation response, the file integration service 130 records 1326 supplemental selection information identifying the confirmed, identified case file, and the identification process ends.

Upon termination of the identification process, the file integration service 130 returns to the process 1300 by storing 1310 an integrated patient encounter record that may include charting and case data, and the process 1300 ends. In some examples, the file integration service 130 can store 1310 within the process 1300 by executing actions such as those executed by the file integration service 130 to store 212a within the process 200a described above with reference to FIG. 2A.

Referring to FIG. 14, a block diagram of examples of computing and medical device components are shown schematically.

The medical device 102 can include a processor 220, a memory 221, one or more output devices 230, one or more user input devices 244, and a communication interface 245. The communication interface 245 can include any of a variety of transmitters and/or receivers. For instance, in some examples, the communication interface 245 includes one or more of an NFC tag, an RFID tag, a barcode, and a QR code.

In various implementations, the medical device 102 can include a defibrillator, a patient monitor, a defibrillator/monitor, an automated compression device, a therapeutic cooling device, an extracorporeal membrane oxygenation (ECMO) device, a ventilation device, combinations thereof, or another type of medical device configured to couple to one or more therapy delivery components to provide therapy to the patient. In an implementation, the medical devices 102 can include an integrated therapy delivery/monitoring device within a single housing 280. The single housing 280 can surround, at least in part, a patient interface device signal processor 256 and/or a therapy delivery control 255.

The one or more patient interface devices 190 can include one or more therapy delivery component(s) 261a and/or one or more sensor device(s) 261b. The medical device 102 can be configured to couple to the one or more therapy delivery component(s) 261a. In combination, the medical device 102 and the one or more therapy delivery components can provide therapeutic treatment to a patient (e.g., the patient 116 of FIG. 1). In an implementation, the medical device 102 can include or incorporate the therapy delivery component(s) 261a. The therapy delivery component(s) 261a are configured to deliver therapy to the patient and can be configured to couple to the patient. For example, the therapy delivery component(s) 261a can include one or more of electrotherapy electrodes including defibrillation electrodes and/or pacing electrodes, chest compression devices (e.g., one or more belts or a piston), ventilation devices (e.g., a mask and/or tubes), drug delivery devices, etc. The medical device 102 can include the one or more therapy delivery component(s) 261a and/or can be configured to couple to the one or more therapy delivery component(s) 261a in order to provide medical therapy to the patient. The therapy delivery component(s) 261a can be configured to couple to the patient. For example, a healthcare provider (e.g., the healthcare provider 118) may attach the electrodes to the patient, and the medical device 102 (e.g., a defibrillator or defibrillator/patient monitor) may provide electrotherapy to the patient via the defibrillation electrodes. These examples are not limiting of the disclosure as other types of medical devices, therapy delivery components, sensors, and therapy are within the scope of the disclosure.

The medical devices 102 can include, for example, a therapeutic medical device capable of delivering a medical therapy. For example, the medical therapy can be electrical therapy (e.g. defibrillation, cardiac pacing, synchronized cardioversion, diaphragmatic or phrenic nerve stimulation) and the medical devices 102 can include a defibrillator, a defibrillator/monitor and/or another medical device configured to provide electrotherapy. As another example, the medical therapy can be chest compression therapy for treatment of cardiac arrest and the medical device 102 can be a mechanical chest compression device such as a belt-based chest compression device or a piston-based chest compression device. As other examples, the medical therapy can be ventilation therapy, therapeutic cooling or other temperature management, invasive hemodynamic support therapy (e.g. ECMO), etc. and the medical device 102 can be a device configured to provide a respective therapy. In an implementation, the medical device 102 can be a combination of one or more of these examples. The therapeutic medical device can include patient monitoring capabilities via one or more sensors. These types of medical therapy and devices are examples only and not limiting of the disclosure.

The medical devices 102 can include, incorporate, and/or be configured to couple to the one or more sensor(s) 261b which can be configured to couple to the patient. The sensor(s) 261b are configured to provide signals indicative of sensor data to the medical device 102. The sensor(s) 261b can be configured to couple to the patient. For example, the sensor(s) 261b can include cardiac sensing electrodes, a chest compression sensor, and/or ventilation sensors. The one or more sensors 261b can generate signals indicative of physiological parameters of the patient. For example, the physiological parameters can include one or more of at least one vital sign, ECG, blood pressure, heart rate, pulse oxygen level, respiration rate, heart sounds, lung sounds, respiration sounds, tidal CO2, saturation of muscle oxygen (SMO2), arterial oxygen saturation (SpO2), cerebral blood flow, electroencephalogram (EEG) signals, brain oxygen level, tissue pH, tissue fluid levels, physical parameters as determined via ultrasound images, parameters determined via near-infrared reflectance spectroscopy, pneumography, and/or cardiography, etc. Additionally or alternatively, the one or more sensors 261b can generate signals indicative of chest compression parameters, ventilation parameters, drug delivery parameters, fluid delivery parameters, etc.

In addition to delivering therapy to the patient, the therapy delivery component(s) 261a can include, be coupled to, and/or function as sensors and provide signals indicative of sensor data (e.g., second sensor data) to the medical device 102. For example, the defibrillation electrodes can be configured as cardiac sensing electrodes as well as electrotherapy delivery devices and can provide signals indicative of transthoracic impedance, electrocardiogram (ECG), heart rate and/or other physiological parameters. As another example, a therapeutic cooling device can be an intravenous cooling device. Such a cooling device can include an intravenous (IV) device as a therapy delivery component configured to deliver cooling therapy and sense the patient's temperature. For example, the IV device can be a catheter that includes saline balloons configured to adjust the patient's temperature via circulation of temperature controlled saline solution. In addition, the catheter can include a temperature probe configured to sense the patient's temperature. As a further example, an IV device can provide therapy via drug delivery and/or fluid management. The IV device can also monitor and/or enable monitoring of a patient via blood sampling and/or venous pressure monitoring (e.g., central venous pressure (CVP) monitoring).

The medical devices 102 can be configured to receive the sensor signals (e.g., from the therapy delivery component(s) 261a and/or the sensor(s) 261b) and to process the sensor signals to determine and collect the patient data. The patient data can include patient data which can characterize a status and/or condition of the patient (e.g., physiological data such as ECG, heart rate, respiration rate, temperature, pulse oximetry, non-invasive hemoglobin parameters, capnography, oxygen saturation (SpO2), end tidal carbon dioxide (EtCO2), invasive blood pressure (IBP), non-invasive blood pressures (NIBP), tissue pH, tissue oxygenation, Near Infrared Spectroscopy (NIRS) measurements, etc.). Additionally or alternatively, the patient data can characterize the delivery of therapy (e.g., chest compression data such as compression depth, compression rate, etc.) and/or the patient data can characterize a status and/or condition of the medical equipment used to treat the patient (e.g., device data such as shock time, shock duration, attachment of electrodes, power-on, etc.).

In some examples, the components of 220, 221, 230, 244, 245, and 255 of the medical device 102 are communicatively coupled (directly and/or indirectly) to each other for bidirectional communication.

Although shown as separate entities in FIG. 14, the one or more of the components of the medical device 102 can be combined into one or more discrete components and/or can be part of the processor 220. The processor 220 and the memory 221 can include and/or be coupled to associated circuitry to perform the functions described herein.

In an implementation, the medical devices 102 can include a therapeutic medical device configured to deliver medical therapy to the patient. Thus, the medical devices 102 can optionally include the therapy delivery control module 255. For example, the therapy delivery control module 255 can be an electrotherapy delivery circuit that includes one or more capacitors configured to store electrical energy for a pacing pulse or a defibrillating pulse. The electrotherapy delivery circuit can further include resistors, additional capacitors, relays and/or switches, electrical bridges such as an H-bridge (e.g., including a plurality of insulated gate bipolar transistors (IGBTs), voltage measuring components, and/or current measuring components. As another example, the therapy delivery control module 255 can be a compression device such as an electro-mechanical controller configured to control a mechanical compression device. As a further example, the therapy delivery control module 255 can be an electro-mechanical controller configured to control drug delivery, temperature management, ventilation, and/or other type of therapy delivery. Alternatively, some examples of the medical devices 102 may not be configured to deliver medical therapy to the patient 116 but can be configured to provide patient monitoring and/or diagnostic care. As shown in FIG. 14, in some examples, the therapy delivery control 255 exchanges messages with a charting device (e.g., the patient charting device 104) via a communication link 1180. These messages can include patient data descriptive of therapy provided to the patient or other patient data stored on the medical devices 102. This patient data can be used by an ePCR application in generating an ePCR documenting a dispatched EMS event. In one embodiment communication link 1180 is implemented using Bluetooth^{®} and/or near-field communications technology.

In certain implementations the file matching and merging functionalities described herein as being associated with the file integration service 130 are alternatively invoked at the medical device 102 and/or the patient charting device 104. In such implementations the communication link 1180 between the medical device 102 and the patient charting device 104 can be used to support this functionality, thus allowing a medical device case file to be integrated with related charting data even when a connection to the server 108 is unavailable. In applications where the communication link 1180 is limited, for example due to limited bandwidth or limited duration, file matching and merging functionality is optionally limited to one or more specified search criteria, such as a search criterion that compares device identifiers. This may be particularly useful in applications where it is desired to throttle bandwidth provided by the communication link 1180 to reserve limited resources for patient treatment and EMS interactions.

The information shared via the communication link 1180 is optionally limited to increase security and/or to protect PHI. This can be accomplished by, for example, configuring the medical device 102 and the patient charting device 104 to only communicate public information (for example, a device identifier) using the communication link 1180. In implementations where PHI is to be shared between the medical device 102 and the patient charting device 104, the communications can be routed via a trusted cloud service where stronger authentication can be implemented. In certain embodiments the server(s) 108 provides such a trusted cloud service. Routing communications via the server(s) 108 may be preferred for other reasons as well, such as where the medical device 102 and the patient charting device 104 produce data in different formats, and/or where one or more of the communicating devices are not configured to communicate in a local network.

The medical devices 102 can incorporate and/or be configured to couple to one or more patient interface devices 190. The patient interface devices 190 can include one or more therapy delivery component(s) 261a and one or more sensor(s) 261b. The one or more therapy delivery component(s) 261a and the one or more sensor(s) 261b can provide one or more signals to the medical devices 102 via wired and/or wireless connection(s).

The one or more therapy delivery component(s) 261a can include electrotherapy electrodes (e.g., the electrotherapy electrodes 266a), ventilation device(s) (e.g., the ventilation device(s) 266b), intravenous device(s) (e.g., the intravenous device(s) 266c), compression device(s) (e.g., the compression device(s) 266d), etc. For example, the electrotherapy electrodes can include defibrillation electrodes, pacing electrodes, and/or combinations thereof. The ventilation devices can include a tube, a mask, an abdominal and/or chest compressor (e.g., a belt, a cuirass, etc.), etc. and combinations thereof. The intravenous devices can include drug delivery devices, fluid delivery devices, and combinations thereof. The compression devices can include mechanical compression devices such as abdominal compressors, chest compressors, belts, pistons, and combinations thereof. In various implementation, the therapy delivery component(s) 261a can be configured to provide sensor data and/or be coupled to and/or incorporate sensors. For example, the electrotherapy electrodes can provide sensor data such as transthoracic impedance, ECG, heart rate, etc. Further the electrotherapy electrodes can include and or be coupled to a chest compression sensor. As another example, the ventilation devices can be coupled to and/or incorporate flow sensors, gas species sensors (e.g., oxygen sensor, carbon dioxide sensor, etc.), etc. As a further example, the intravenous devices can be coupled to and/or incorporate temperature sensors, flow sensors, blood pressure sensors, etc. As yet another example, the compression devices can be coupled to and/or incorporate chest compression sensors, patient position sensors, etc. The therapy delivery control module 255 can be configured to couple to and control the therapy delivery component(s) 261a.

In various implementations, the sensor(s) 261b can include one or more sensor devices configured to provide sensor data that includes, for example, but not limited to electrocardiogram (ECG), blood pressure, heart rate, pulse oxygen level, respiration rate, heart sounds, lung sounds, respiration sounds, tidal CO2, saturation of muscle oxygen (SMO2), arterial oxygen saturation (SpO2), cerebral blood flow, electroencephalogram (EEG) signals, brain oxygen level, tissue pH, tissue fluid levels, images and/or videos via ultrasound, laryngoscopy, and/or other medical imaging techniques, near-infrared reflectance spectroscopy, pneumography, cardiography, and/or patient movement. Images and/or videos can be two-dimensional or three-dimensional.

The sensor(s) 261b can include sensing electrodes (e.g., the sensing electrodes 262), ventilation sensors (e.g., the ventilation sensors 264), temperature sensors (e.g., the temperature sensor 267), chest compression sensors (e.g., the chest compression sensor 268), etc. For example, the sensing electrodes can include cardiac sensing electrodes. The cardiac sensing electrodes can be conductive and/or capacitive electrodes configured to measure changes in a patient's electrophysiology, for example to measure the patient's ECG information. In an implementation, the sensing electrodes can be configured to measure the transthoracic impedance and/or a heart rate of the patient. The ventilation sensors can include spirometry sensors, flow sensors, pressure sensors, oxygen and/or carbon dioxide sensors such as, for example, one or more of pulse oximetry sensors, oxygenation sensors (e.g., muscle oxygenation/pH), O2 gas sensors and capnography sensors, and combinations thereof. The temperature sensors can include an infrared thermometer, a contact thermometer, a remote thermometer, a liquid crystal thermometer, a thermocouple, a thermistor, etc. and can measure patient temperature internally and/or externally. The chest compression sensor can include one or more motion sensors including, for example, one or more accelerometers, one or more force sensors, one or more magnetic sensors, one or more velocity sensors, one or more displacement sensors, etc. The chest compression sensor can be, for example, but not limited to, a compression puck, a smart phone, a hand-held device, a wearable device, etc. The chest compression sensor can be configured to detect chest motion imparted by a rescuer and/or an automated chest compression device (e.g., a belt system, a piston system, etc.). The chest compression sensor can provide signals indicative of chest compression data including displacement data, velocity data, release velocity data, acceleration data, compression rate data, dwell time data, hold time data, blood flow data, blood pressure data, etc. In an implementation, the sensing electrodes and/or the electrotherapy electrodes can include or be configured to couple to the chest compression sensor.

Continuing with FIG. 14, examples of components of the charting device 104 are shown schematically. In an implementation, the charting device 104 can be configured as a charting device. The charting device 104 can include a processor 420, a memory 421, one or more output devices 430, one or more user input devices 444, and a communication interface 445. FIG. 14 also illustrates schematically examples of components of the computing device 106. As shown in FIG. 14, the computing device 106 can include a processor 320, a memory 321, one or more output devices 330, one or more user input devices 344, and a communication interface 345. FIG. 14 further illustrates schematically examples of components of the server(s) 108. As shown in FIG. 14, the server(s) 108 can include a processor 520, a memory 521, one or more output devices 530, one or more user input devices 544, and a communication interface 545.

Each of the charting device 104 (e.g., the charting device) and the computing device 106 can be a computer system, such as a desktop, notebook, mobile, portable, or other type of computing system. Each of these devices 104 and 106 can include server(s) and/or access server(s) via a monitor and/or other connected user interface device. Although described as server(s), the server(s) 108 can be another type of computing system including for example a desktop, notebook, mobile, portable, or other type of computing system.

As shown in FIG. 14, each of the devices 104 and 106, along with the server(s) 108 and the medical devices 102, includes a bus or other interconnection mechanism that communicably couples the processor, memory, output devices, input devices, and communication interface included therein. The bus can include a PCI/PCI-X or SCSI based system bus depending on the storage devices used, for example.

The processors 220, 320, 420, and 520 can each include a processor, such as, but not limited to, Intel^{®} Itanium^{®} or Itanium 2^{®} processor(s), or AMD^{®} Opteron^{®} or Athlon MP^{®} processor(s), or Motorola^{®} lines of processors. The communication interfaces 245, 345, 445, and 545 can each be any of an RS-232 port for use with a modem-based dialup connection, a 10/100 Ethernet port, or a Gigabit port using copper or fiber, for example. The communication interfaces 245, 345, 445, and 545 may be chosen depending on a network(s) such a Local Area Network (LAN), Wide Area Network (WAN), or any network to which the medical devices 102, the charting device 104, the computing device 106, and/or the server(s) 108 may connect. The memories 221, 321, 421, and 521 can be Random Access Memory (RAM), Read Only Memory (ROM), Flash memory, and/or another dynamic volatile and/or non-volatile storage device(s). The memories 221, 321, 421, and 521 can be used to store information and instructions. For example, hard disks such as the Adaptec^{®} family of SCSI drives, an optical disc, an array of disks such as RAID (e.g. the Adaptec family of RAID drives), or any other mass storage devices may be used. The components described above are meant to exemplify some types of possibilities. In no way should the aforementioned examples limit the scope of the disclosure. The memories 221, 321, 421, and 521 can further include removable storage media such as external hard-drives, floppy drives, flash drives, IOMEGA^{®} Zip Drives, Compact Disc - Read Only Memory (CD-ROM), Compact Disc - Re-Writable (CD-RW), or Digital Video Disk - Read Only Memory (DVD-ROM), for example.

Continuing with FIG. 14, the server(s) 108 can include, for example, the one or more storage server(s) and one or more application server(s). In some examples, the server(s) 108 are configured to exchange messages 1170 with the computing device 106. These messages can include charting and/or case data as described above. In some examples, the server(s) 108 are configured to exchange messages 1160 with the charting device 104 via the ePCR API 128. These messages can include data descriptive of ePCRs generated by the charting device 104. In some examples, the server(s) 108 are configured to exchange messages 1190 with the medical devices 102. These messages can include data descriptive of a patient (e.g., the patient 116 of FIG. 1) being treated by the medical device and/or treatment being delivered by the medical devices 102.

Some examples of the present disclosure include various steps, some of which can be performed by hardware components or can be embodied in machine-executable instructions. These machine-executable instructions can be stored on a non-transitory data storage medium and can be used to cause a general-purpose or a special-purpose processor programmed with the instructions to perform the steps. The non-transitory data storage medium can further store an operating system and the machine-executable instructions can be included within one or more software applications or programs, such as the ePCR application. These programs can implement the features disclosed herein and the methods that they execute. Alternatively, the steps can be performed by a combination of hardware, software, and/or firmware, on one device and/or distributed across multiple devices and/or processors. In addition, some examples of the present disclosure can be performed or implemented, at least in part ( e.g., one or more modules), on one or more computer systems, mainframes (e.g., IBM mainframes such as the IBM zSeries, Unisys ClearPath Mainframes, HP Integrity NonStop server(s), NEC Express series, and others), or client-server type systems. In addition, specific hardware aspects of examples of the present disclosure can incorporate one or more of these systems, or portions thereof.

Having thus described several aspects of at least one example, it is to be appreciated that various alterations, modifications, and improvements will readily occur to those skilled in the art. For instance, examples disclosed herein can also be used in other contexts. Such alterations, modifications, and improvements are intended to be part of this disclosure and are intended to be within the scope of the examples discussed herein. Accordingly, the foregoing description and drawings are by way of example only.
Further embodiments are provided in the following numbered clauses:
1. A medical records server system for integrating medical device case files with electronic patient care records, the system comprising:
   at least one database storing a plurality of medical device case files from a plurality of medical devices comprising medical device data recorded during patient encounters;
   a network interface; and
   at least one processor coupled to the at least one database and the network interface and configured to:
      receive, from a patient charting device via the network interface, charting data documented in an electronic patient care record (ePCR) for an encounter with a particular patient,
      generate a search criterion based at least in part on the charting data to identify a medical device case file for the encounter with the particular patient, the search criterion comprising a predetermined relationship between at least one first parameter from the charting data and at least one second parameter associated with the medical device case file,
      search for the medical device case file using the generated search criterion,
      identify the medical device case file based on the generated search criterion,
      generate an integrated patient encounter record comprising at least a portion of the medical device data from the medical device case file and the charting data from the ePCR, and
      store the integrated patient encounter record in the at least one database.
2. The medical records server system of clause 1,
   wherein to receive the charting data comprises to receive the ePCR, and
   wherein to generate the integrated patient encounter record comprises to:
      import the at least the portion of the medical device data into the ePCR to generate a supplemented ePCR, and
      store the supplemented ePCR in the at least one database.
3. The medical records server system of clause 2, wherein the at least one processor is configured to transmit the supplemented ePCR to the patient charting device.
4. The medical records server system of clause 1, wherein the at least one processor is configured to transmit the at least the portion of the medical device data to the patient charting device via the network interface.
5. The medical records server system of clause 1, wherein the at least one processor is configured to transmit at least a portion of the charting data to a medical device via the network interface.
6. The medical records server system of clause 1, wherein the network interface is configured to communicate with the patient charting device and a medical device via one or more of a cellular connection and a transmission control protocol/internet protocol connection.
7. The medical records server system of clause 1, wherein the at least one database comprises a cloud database associated with multiple tenants.
8. The medical records server system of clause 1, wherein the at least one processor is configured to transmit the integrated patient encounter record to one or more of a medical facility computing device and a health records database via the network interface.
9. The medical records server system of clause 1, wherein the network interface is configured to communicatively couple to the patient charting device via one or more of a computer network and a cellular network.
10. The medical records server system of clause 1, wherein the network interface is configured to communicatively couple to the plurality of medical devices via one or more of a computer network and a cellular network.
11. The medical records server system of clause 10, wherein the system is configured to receive the plurality of medical device case files from the plurality of medical devices via the network interface.
12. The medical records server system of clause 1, wherein the medical records server system is located remotely from the patient charting device and the plurality of medical devices.
13. The medical records server system of clause 1, wherein the at least one processor is configured to:
   transmit, to the patient charting device via the network interface, an identifier of the medical device case file;
   receive, via the network interface from the patient charting device, a confirmation of the medical device case file; and
   store the integrated patient encounter record in response to the received confirmation.
14. The medical records server system of clause 1, wherein the at least one second parameter comprises medical device data stored in the medical device case file.
15. The medical records server system of clause 1, wherein the at least one second parameter comprises metadata for the medical device case file.
16. The medical records server system of clause 1, wherein the medical device case file is a defibrillator case file.
17. The medical records server system of clause 1, wherein the at least one first parameter and the at least one second parameter are a same parameter and the predetermined relationship comprises a match between a value of the at least one first parameter and a value of the at least one second parameter.
18. The medical records server system of clause 17, wherein the at least one first parameter and the at least one second parameter comprise one or more of a case start time, patient identification information, and a medical device identifier.
19. The medical records server system of clause 18, wherein the patient identification information comprises biometric information.
20. The medical records server system of clause 19, wherein the biometric information comprises one or more of facial recognition information, fingerprint information, and retinal scan information.
21. The medical records server system of clause 18, wherein the medical device identifier is one or more of an identifier manually entered into the ePCR, an identifier electronically transmitted to the patient charting device, and an identifier scanned from a bar code or quick response code associated with a medical device.
22. The medical records server system of clause 1,
   wherein the predetermined relationship comprises a match between two or more first parameters from the charting data and two or more second parameters associated with the medical device case file, and
   wherein the two or more first parameters and the two or more second parameters are same parameters.
23. The medical records server system of clause 22, wherein the two or more first parameters and the two or more second parameters comprise two or more of a case start time, patient identification information, a medical device identifier, a time-of-transfer, healthcare provider identification information, patient demographic information, and patient medical information.
24. The medical records server system of clause 23, wherein the two or more first parameters and the two or more second parameters comprise the patient medical information and one or more of the case start time, the patient identification information, and the medical device identifier.
25. The medical records server system of clause 24, wherein the patient medical information comprises one or more physiological measurements from the particular patient.
26. The medical records server system of clause 25, wherein the one or more physiological measurements comprise one or more of blood pressure, body temperature, respiratory rate, heart rate, and an ECG parameter.
27. The medical records server system of clause 1, wherein the predetermined relationship comprises one or more of:
   (a) an overlapping range between a case start time and a case end time from the medical device case file and from the ePCR, and
   (b) a match between a medical device identifier from the medical device case file and from the ePCR; and
   one or more of:
   (a) a match between a patient biometric identifier from the medical device case file and from the ePCR, and
   (b) a match between a healthcare provider biometric identifier from the medical device case file and from the ePCR.
28. The medical records server system of clause 1, wherein the predetermined relationship comprises the at least one second parameter from the medical device data falling within a range associated with the at least one first parameter from the charting data.
29. The medical records server system of clause 1,
   wherein the predetermined relationship comprises one or more of a case start time and a case end time included in the medical device case file that are within a target time range defined based on case start and end times included in the charting data,
   wherein the case start and end times included in the charting data are the at least one first parameter, and
   wherein the case start time and case end time included in the medical device case file are the at least one second parameter.
30. The medical records server system of clause 1,
   wherein the predetermined relationship comprises:
      (a) a match between a medical device identifier included in the charting data and a medical device identifier included in the medical device case file, and
      (b) one or more of a case start time and a case end time included in the medical device case file that are within a target time range defined based on case start and end times included in the charting data,
   wherein the medical device identifier and case start and end times included in the charting data are the at least one first parameter, and
   wherein the medical device identifier included in the medical device case file and the case start time and case end time included in the medical device case file are the at least one second parameter.
31. The medical records server system of clause 1, wherein the at least one first parameter and the at least one second parameter comprise a case start time and the range associated with the first parameter comprises a range of case start times.
32. The medical records server system of clause 31, wherein the range of case start times is 0 to 10 minutes.
33. The medical records server system of clause 28, wherein the at least one first parameter and the at least one second parameter comprise a case start time and the range associated with the at least one first parameter comprises a time span between the case start time from the charting data and a transfer-of-care time from the charting data.
34. The medical records server system of clause 1, wherein to search for the medical device case file comprises to repeat the search until a single medical device case file is identified or until a search iteration threshold is exceeded.
35. The medical records server system of clause 1,
   wherein the generated search criterion comprises an initial search criterion and the predetermined relationship comprises an initial predetermined relationship, and
   wherein to identify the medical device case file comprises to:
      identify multiple candidate medical device case files based on the initial search criterion; and
      identify the medical device case file from the multiple candidate medical device case files based on supplemental selection information.
36. The medical records server system of clause 35, wherein the at least one processor is configured to:
   transmit, to the patient charting device via the network interface, identifiers of the multiple candidate medical device case files; and
   receive, via the network interface from the patient charting device, the supplemental selection information comprising a user selection of the medical device case file for the encounter with the particular patient, wherein to store the integrated patient encounter record comprises to store the integrated patient encounter record in response to the received confirmation.
37. The medical records server system of clause 36, wherein the identifiers for the multiple candidate medical device case files comprise file association indicators for each medical device case file.
38. The medical records server system of clause 36, wherein the identifiers of the multiple candidate medical device case files comprise descriptive information about contents of the multiple candidate medical device case files.
39. The medical records server system of clause 35, wherein the supplemental selection information comprises a supplemental search criterion, and the at least one processor is configured to: search for the medical device case file amongst the multiple candidate medical device case files using the supplemental search criterion; and automatically identify the medical device case file from the multiple candidate medical device case files based on the supplemental search criterion.
40. The medical records server system of clause 39, the supplemental search criterion comprising at least one additional predetermined relationship between at least one additional parameter from the charting data and at least one additional parameter associated with the medical device case file.
41. The medical records server system of clause 40, wherein the first parameter and the second parameter are both a case start time and wherein the at least one additional parameter from the charting data and the at least one additional parameter associated with the medical device case file comprise a parameter other than the case start time.
42. The medical records server system of clause 41, wherein the first parameter and the second parameter are within 0 to 1, 0 to 2, 0 to 5, or 0 to 10 minutes of one another.
43. The medical records server system of clause 40, wherein the at least one additional parameter from the charting data and the at least one additional parameter associated with the medical device case file comprise one or more of a medical device identifier, patient identification information, healthcare provider identification information, and patient demographic information.
44. The medical records server system of clause 43,
   wherein the first parameter and the second parameter are within a time range of one another, and
   wherein a length of the time range is longer if the at least one additional parameter from the charting data and the at least one additional parameter associated with the medical device case file are the medical device identifier than if the at least one additional parameter from the charting data and the at least one additional parameter associated with the medical device case file are a parameter other than the medical device identifier.
45. The medical records server system of clause 39,
   wherein the initial predetermined relationship comprises the second parameter falling within a range for the first parameter, and
   wherein the supplemental search criterion comprises a match between the first parameter and the second parameter.
46. The medical records server system of clause 39,
   wherein the initial predetermined relationship comprises a match and the at least one first parameter from the charting data and the at least one second parameter associated with the medical device case file comprise non-medical parameters, and
   wherein the supplemental search criterion comprises at least one medical parameter from the charting data that is medically consistent and indicative of at least one medical parameter from the medical device case file.
47. The medical records server system of clause 46, wherein the at least one first parameter from the charting data and the at least one second parameter from the medical device data comprises one or more of a case start time, a medical device identifier, and patient identification information.
48. The medical records server system of clause 46, wherein the at least one medical parameter from the charting data comprises one or more of a patient complaint, a medic impression, a drug administration, a vital sign, a physiological measurement, and a treatment provided to the particular patient.
49. The medical records server system of clause 46, wherein the at least one medical parameter from the charting data comprises one or more of a symptom of chest pain, an ECG with ST elevation, a STEMI diagnosis.
50. The medical records server system of clause 46, wherein the at least one medical parameter from the medical device case file comprises one or more of a type of physiological measurement, a value of a physiological measurement, a medical treatment, and an alarm.
51. The medical records server system of clause 50, wherein the medical treatment comprises a defibrillation shock.
52. The medical records server system of clause 46, wherein the at least one medical parameter from the charting data comprises a patient complaint of chest pain and the at least one medical parameter from the medical device case file comprises a record of a twelve-lead electrocardiogram.
53. The medical records server system of clause 52, wherein the at least one medical parameter from the charting data comprises aspirin or nitroglycerin administration.
54. The medical records server system of clause 52, wherein the at least one medical parameter from the medical device case file comprises shock administration.
55. The medical records server system of clause 46, wherein the at least one medical parameter from the charting data comprises one or more of a patient complaint of difficulty breathing and a medic impression of respiratory distress and the at least one medical parameter from the medical device case file comprises a pulse oximetry scan and/or a capnography scan.
56. The medical records server system of clause 55, wherein the at least one medical parameter from the charting data comprises administration of albuterol, steroids, or epinephrine.
57. The medical records server system of clause 55, wherein the at least one medical parameter from the charting data comprises airway placement.
58. The medical records server system of clause 55, wherein the at least one medical parameter from the medical device case file comprises a twelve lead ECG.
59. The medical records server system of clause 55, wherein the at least one medical parameter from the medical device case file comprises alarms for one or more of heart rate, respiratory rate, pulse oximetry, and blood pressure.
60. A non-transitory, processor-readable storage medium having stored thereon processor-executable instructions for integrating medical device case files with electronic patient care records, the processor-executable instructions configured to cause a processor to:
   access at least one database storing a plurality of medical device case files from a plurality of medical devices, each case file comprising medical device data recorded by a medical device during an encounter with a patient;
   receive, from a patient charting device via a network interface, charting data documented in an electronic patient care record (ePCR) for the encounter with the patient;
   generate a search criterion based at least in part on the charting data to identify the medical device case file for the encounter with the patient, the search criterion comprising a predetermined relationship between at least one first parameter from the charting data and at least one second parameter associated with the medical device case file;
   search for the medical device case file using the generated search criterion;
   identify the medical device case file based on the generated search criterion;
   generate an integrated patient encounter record comprising at least a portion of the medical device data from the medical device case file and the charting data from the ePCR; and
   store the integrated patient encounter record in the at least one database.
61. The non-transitory, processor-readable storage medium of clause 60,
   wherein to receive the charting data comprises to receive the ePCR, and
   wherein to store the integrated patient encounter record comprises to:
      import the at least the portion of the medical device data into the ePCR to generate a supplemented ePCR, and
      store the supplemented ePCR in the at least one database.
62. The non-transitory, processor-readable storage medium of clause 61, wherein the processor-executable instructions are configured to cause the processor to transmit the supplemented ePCR to the patient charting device.
63. The non-transitory, processor-readable storage medium of clause 60, wherein the processor-executable instructions are configured to cause the processor to transmit the at least the portion of the medical device data to the patient charting device via the network interface.
64. The non-transitory, processor-readable storage medium of clause 60, wherein the processor-executable instructions are configured to cause the processor to transmit at least a portion of the charting data to a medical device via the network interface.
65. The non-transitory, processor-readable storage medium of clause 60, wherein the processor-executable instructions comprise processor-executable instructions configured to cause the processor to communicate with the patient charting device and a medical device via one or more of a cellular connection and a transmission control protocol/internet protocol connection.
66. The non-transitory, processor-readable storage medium of clause 60, wherein to access the at least one database comprises to access a cloud database associated with multiple tenants.
67. The non-transitory, processor-readable storage medium of clause 60, wherein the processor-executable instructions are configured to cause the processor to transmit the integrated patient encounter record to one or more of a medical facility computing device and a health records database via the network interface.
68. The non-transitory, processor-readable storage medium of clause 60, wherein the processor-executable instructions comprise processor-executable instructions configured to cause the processor to communicatively couple to the patient charting device via the network interface and one or more of a computer network and a cellular network.
69. The non-transitory, processor-readable storage medium of clause 60, wherein the processor-executable instructions comprise processor-executable instructions configured to cause the processor to communicatively couple to the plurality of medical devices via the network interface and one or more of a computer network and a cellular network.
70. The non-transitory, processor-readable storage medium of clause 69, wherein the processor-executable instructions comprise processor-executable instructions configured to cause the processor to receive the plurality of medical device case files from the plurality of medical devices via the network interface.
71. The non-transitory, processor-readable storage medium of clause 60, wherein the processor-executable instructions comprise processor-executable instructions configured to cause the processor to communicate remotely with the patient charting device and the plurality of medical devices.
72. The non-transitory, processor-readable storage medium of clause 60, wherein the processor-executable instructions are configured to cause the processor to:
   transmit, to the patient charting device via the network interface, an identifier of the medical device case file;
   receive, via the network interface from the patient charting device, a confirmation of the medical device case file; and
   store the integrated patient encounter record in response to the received confirmation.
73. The non-transitory, processor-readable storage medium of clause 60, wherein the at least one second parameter comprises medical device data stored in the medical device case file.
74. The non-transitory, processor-readable storage medium of clause 60, wherein the at least one second parameter comprises metadata for the medical device case file.
75. The non-transitory, processor-readable storage medium of clause 60, wherein the medical device case file is a defibrillator case file.
76. The non-transitory, processor-readable storage medium of clause 60, wherein the at least one first parameter and the at least one second parameter are a same parameter and the predetermined relationship comprises a match between a value of the at least one first parameter and a value of the at least one second parameter.
77. The non-transitory, processor-readable storage medium of clause 76, wherein the at least one first parameter and the at least one second parameter comprise one or more of a case start time, patient identification information, and a medical device identifier.
78. The non-transitory, processor-readable storage medium of clause 77, wherein the patient identification information comprises biometric information.
79. The non-transitory, processor-readable storage medium of clause 78, wherein the biometric information comprises one or more of facial recognition information, fingerprint information, and retinal scan information.
80. The non-transitory, processor-readable storage medium of clause 77, wherein the medical device identifier is one or more of an identifier manually entered into the ePCR, an identifier electronically transmitted to the patient charting device, and an identifier scanned from a bar code or quick response code associated with a medical device.
81. The non-transitory, processor-readable storage medium of clause 60,
   wherein the predetermined relationship comprises a match between two or more first parameters from the charting data and two or more second parameters associated with the medical device case file, and
   wherein the two or more first parameters and the two or more second parameters are same parameters.
82. The non-transitory, processor-readable storage medium of clause 81, wherein the two or more first parameters and the two or more second parameters comprise two or more of a case start time, patient identification information, a medical device identifier, a time-of-transfer, healthcare provider identification information, patient demographic information, and patient medical information.
83. The non-transitory, processor-readable storage medium of clause 82, wherein the two or more first parameters and the two or more second parameters comprise the patient medical information and one or more of the case start time, the patient identification information, and the medical device identifier.
84. The non-transitory, processor-readable storage medium of clause 83, wherein the patient medical information comprises one or more physiological measurements from the patient.
85. The non-transitory, processor-readable storage medium of clause 84, wherein the one or more physiological measurements comprise one or more of blood pressure, body temperature, respiratory rate, heart rate, and an ECG parameter.
86. The non-transitory, processor-readable storage medium of clause 60, wherein the predetermined relationship comprises
   one or more of:
      (a) an overlapping range between a case start time and a case end time from the medical device case file and from the ePCR, and
      (b) a match between a medical device identifier from the medical device case file and from the ePCR; and
   and one or more of:
      (a) a match between a patient biometric identifier from the medical device case file and from the ePCR, and
      (b) a match between a healthcare provider biometric identifier from the medical device case file and from the ePCR.
87. The non-transitory, processor-readable storage medium of clause 60, wherein the predetermined relationship comprises the second parameter from the medical device data falling within a range associated with the first parameter from the charting data.
88. The non-transitory, processor-readable storage medium of clause 87, wherein the processor-executable instructions are configured to cause the processor to calculate the range.
89. The non-transitory, processor-readable storage medium of clause 87, wherein the range is hardcoded.
90. The non-transitory, processor-readable storage medium of clause 87, wherein the first parameter and the second parameter comprise a case start time and the range associated with the first parameter comprises a range of case start times.
91. The non-transitory, processor-readable storage medium of clause 90, wherein the range of case start times is 0 to 10 minutes.
92. The non-transitory, processor-readable storage medium of clause 87, wherein the first parameter and the second parameter comprise a case start time and the range associated with the first parameter comprises a time span between the case start time from the charting data and a transfer-of-care time from the charting data.
93. The non-transitory, processor-readable storage medium of clause 60, wherein to search for the medical device case file comprises to repeat the search until a single medical device case file is identified or until a search iteration threshold is exceeded.
94. The non-transitory, processor-readable storage medium of clause 60,
   wherein the generated search criterion comprises an initial search criterion and the predetermined relationship comprises an initial predetermined relationship, and
   wherein to identify the medical device case file comprises to:
      identify multiple candidate medical device case files based on the initial search criterion; and
      identify the medical device case file from the multiple candidate medical device case files based on supplemental selection information.
95. The non-transitory, processor-readable storage medium of clause 94,wherein the processor-executable instructions are configured to cause the processor to:
   transmit, to the patient charting device via the network interface, identifiers of the multiple candidate medical device case files; and
   receive, via the network interface from the patient charting device, the supplemental selection information comprising a user selection of the medical device case file for the encounter with the patient, wherein to store the integrated patient encounter record comprises to store the integrated patient encounter record in response to the received confirmation.
96. The non-transitory, processor-readable storage medium of clause 95, wherein the identifiers for the multiple candidate medical device case files comprise file association indicators for each medical device case file.
97. The non-transitory, processor-readable storage medium of clause 95, wherein the identifiers of the multiple candidate medical device case files comprise descriptive information about contents of the multiple candidate medical device case files.
98. The non-transitory, processor-readable storage medium of clause 94, wherein the supplemental selection information comprises a supplemental search criterion, and the processor-executable instructions are configured to cause the processor to:
   search for the medical device case file amongst the multiple candidate medical device case files using the supplemental search criterion; and
   automatically identify the medical device case file from the multiple candidate medical device case files based on the supplemental search criterion.
99. The non-transitory, processor-readable storage medium of clause 98, the supplemental search criterion comprising at least one additional predetermined relationship between at least one additional parameter from the charting data and at least one additional parameter associated with the medical device case file.
100. The non-transitory, processor-readable storage medium of clause 99,
   wherein the first parameter and the second parameter are both a case start time and
   wherein the at least one additional parameter from the charting data and the at least one additional parameter associated with the medical device case file comprise a parameter other than the case start time.
101. The non-transitory, processor-readable storage medium of clause 100, wherein the first parameter and the second parameter are within 0 to 1, 0 to 2, 0 to 5, or 0 to 10 minutes of one another.
102. The non-transitory, processor-readable storage medium of clause 99, wherein the at least one additional parameter from the charting data and the at least one additional parameter associated with the medical device case file comprise one or more of a medical device identifier, patient identification information, healthcare provider identification information, and patient demographic information.
103. The non-transitory, processor-readable storage medium of clause 102,
   wherein the first parameter and the second parameter are within a time range of one another and
   wherein a length of the time range is longer if the at least one additional parameter from the charting data and the at least one additional parameter associated with the medical device case file are the medical device identifier than if the at least one additional parameter from the charting data and the at least one additional parameter associated with the medical device case file are a parameter other than the medical device identifier.
104. The non-transitory, processor-readable storage medium of clause 98,
   wherein the initial predetermined relationship comprises the second parameter falling within a range for the first parameter, and
   wherein the supplemental search criterion comprises a match between the first parameter and the second parameter.
105. The non-transitory, processor-readable storage medium of clause 98,
   wherein the initial predetermined relationship comprises a match and the at least one first parameter from the charting data and the at least one second parameter associated with the medical device case file comprise non-medical parameters, and
   wherein the supplemental search criterion comprises at least one medical parameter from the charting data that is medically consistent and indicative of at least one medical parameter from the medical device case file.
106. The non-transitory, processor-readable storage medium of clause 105, wherein the at least one first parameter from the charting data and the at least one second parameter from the medical device data comprises one or more of a case start time, a medical device identifier, and patient identification information.
107. The non-transitory, processor-readable storage medium of clause 105, wherein the at least one medical parameter from the charting data comprises one or more of a patient complaint, a medic impression, a drug administration, a vital sign, a physiological measurement, and a treatment provided to the patient.
108. The non-transitory, processor-readable storage medium of clause 105, wherein the at least one medical parameter from the charting data comprises one or more of a symptom of chest pain, an ECG with ST elevation, a STEMI diagnosis.
109. The non-transitory, processor-readable storage medium of clause 105, wherein the at least one medical parameter from the medical device case file comprises one or more of a type of physiological measurement, a value of a physiological measurement, a medical treatment, and an alarm.
110. The non-transitory, processor-readable storage medium of clause 109, wherein the medical treatment comprises a defibrillation shock.
111. The non-transitory, processor-readable storage medium of clause 105, wherein the at least one medical parameter from the charting data comprises a patient complaint of chest pain and the at least one medical parameter from the medical device case file comprises a record of a twelve-lead electrocardiogram.
112. The non-transitory, processor-readable storage medium of clause 111, wherein the at least one medical parameter from the charting data comprises aspirin or nitroglycerin administration.
113. The non-transitory, processor-readable storage medium of clause 111, wherein the at least one medical parameter from the medical device case file comprises shock administration.
114. The non-transitory, processor-readable storage medium of clause 105, wherein the at least one medical parameter from the charting data comprises one or more of a patient complaint of difficulty breathing and a medic impression of respiratory distress and the at least one medical parameter from the medical device case file comprises a pulse oximetry scan and/or a capnography scan.
115. The non-transitory, processor-readable storage medium of clause 114, wherein the at least one medical parameter from the charting data comprises administration of albuterol, steroids, or epinephrine.
116. The non-transitory, processor-readable storage medium of clause 114, wherein the at least one medical parameter from the charting data comprises airway placement.
117. The non-transitory, processor-readable storage medium of clause 114, wherein the at least one medical parameter from the medical device case file comprises a twelve lead ECG.
118. The non-transitory, processor-readable storage medium of clause 114, wherein the at least one medical parameter from the medical device case file comprises alarms for one or more of heart rate, respiratory rate, pulse oximetry, and blood pressure.
119. A patient charting device for creating an integrated medical device and patient care record, the patient charting device comprising:
   a user interface;
   a communications interface; and
   a processor, memory, and associated circuitry communicatively coupled with the user interface and the communications interface, and configured to
      receive, via the user interface during a patient encounter, input specifying charting data, transmit, via the communications interface, the charting data to a server,
      receive, via the communications interface from the server, at least one identifier of at least one medical device case file associated with the charting data,
      render, via the user interface, at least one prompt to confirm that the at least one medical device case file was generated during the patient encounter,
      receive, via the user interface, input confirming that the at least one medical device case file was generated during the patient encounter, and
      transmit, via the communications interface, a confirmation specifying that the at least one medical device case file was generated during the patient encounter.
120. The patient charting device of clause 119, wherein the processor, memory, and associated circuitry are further configured to render prompts to capture the charting data.
121. The patient charting device of clause 119, wherein the charting data comprises all or a portion of the patient care record.
122. The patient charting device of clause 119, wherein the at least one identifier of the at least one medical device case file comprises a single identifier of a single medical device case files and the at least one prompt to confirm comprises a single prompt to confirm that the single medical device case file was generated during the patient encounter.
123. The patient charting device of clause 119, wherein the at least one identifier of the at least one medical device case file comprises a plurality of identifiers of a plurality of medical device case files and the at least one prompt comprises one or more user interface controls configured to receive a selection of a single medical device case file of the plurality of medical device case files to confirm the single medical device case file was generated during the patient encounter.
124. The patient charting device of clause 123, wherein
   each identifier of the plurality of identifiers is associated with a file association indicator of a plurality of file association indicators, each file association indicator of the plurality of file association indicators indicating a degree of similarity between parameters associated with the medical device case file identified by the identifier and parameters of the charting data,
   the one or more user interface controls comprise a plurality of user interface controls,
   each user interface control of the plurality of user interface controls corresponds to an identifier of the plurality of identifiers, and
   each user interface control is positioned within the at least one prompt according the file association indicator associated the identifier corresponding to the user interface control.
125. The patient charting device of clause 124, wherein to receive the at least one identifier comprises to receive the plurality of file association indicators.
126. The patient charting device of clause 124, wherein the processor, memory, and associated circuitry are further configured to determine the plurality of file association indicators.
127. The patient charting device of clause 124, wherein each user interface control is labeled with the file association indicator associated the identifier corresponding to the user interface control.
128. The patient charting device of clause 123, wherein each user interface control is labeled with case data from the medical device case file identified by the identifier corresponding the user interface control, the case data comprising one or more of case start time, medical device identifier, time-to-transfer, healthcare provider identifier, patient identifier, patient demographic information, patient medical information, and physiological measurements.
129. The patient charting device of clause 119, wherein the at least one prompt comprises a user interface control configured to receive input refusing to confirm that the at least one medical device case file was generated during the patient encounter.
130. The patient charting device of clause 119, the processor, memory, and associated circuitry are further configured to:
   receive the at least one medical device case file;
   parse the at least one medical device case file to retrieve case data; and
   import the case data into the patient care record to generate an integrated patient encounter record.
131. The patient charting device of clause 130, wherein the processor, memory, and associated circuitry are further configured to store the integrated patient encounter record locally and/or transmit the integrated patient encounter record to a multi-tenant cloud server.
132. The patient charting device of clause 119, wherein the user interface comprises one or more of a touch screen configured to receive tactile input, a microphone configured to receive audio input, and wearable glasses configured to receive gestural input.
133. The patient charting device of clause 119, wherein the patient charting device is a tablet, a medical device, a watch, or another computing device.
134. The patient charting device of clause 119, wherein the communications interface supports wired and/or wireless connections.
135. The patient charting device of clause 119, the processor, memory, and associated circuitry are further configured to:
   receive a medical device identifier via a camera, a radio-frequency identification sensor, and/or a near-field communication sensor;
   import the medical device identifier into the patient care record;
   transmit the patient care record comprising the medical device identifier to the server; and
   receive, from the server, a medical device case file generated by a medical device identified by the medical device identifier.
136. A medical records server system for integrating medical device case files with corresponding electronic patient care records, the system comprising:
   at least one database storing a plurality of medical device case files from a plurality of medical devices comprising medical device data recorded during patient encounters, wherein each medical device case file comprises a first case start time and a medical device identifier;
   a network interface; and
   at least one processor coupled to the at least one database and the network interface and configured to
      receive, from a patient charting device via the network interface, charting data documented in an electronic patient care record (ePCR) for an encounter with a particular patient, wherein each ePCR comprises a second case start time and the medical device identifier,
      identify the medical device case file based on the first and second case start times and the medical device identifier,
      generate an integrated patient encounter record comprising at least a portion of the medical device data from the medical device case file and the charting data from the ePCR, and
      store the integrated patient encounter record in the at least one database.
137. The medical records server system of clause 136,
   wherein to receive the charting data comprises to receive the ePCR, and
   wherein to store the integrated patient encounter record comprises to:
      import the at least the portion of the medical device data into the ePCR to generate a supplemented ePCR, and
      store the supplemented ePCR in the at least one database.
138. The medical records server system of clause 137, wherein the at least one processor is configured to transmit the supplemented ePCR to the patient charting device.
139. The medical records server system of clause 136, wherein the at least one processor is configured to transmit the at least the portion of the medical device data to the patient charting device via the network interfacemedical records server system of clause 136, wherein the at least one processor is configured to transmit at least a portion of the charting data to a medical device via the network interface.
141. The medical records server system of clause 136, wherein the network interface is configured to communicate with the patient charting device and the medical device via one or more of a cellular connection and a transmission control protocol/internet protocol connection.
142. The medical records server system of clause 136, wherein the at least one database comprises a cloud database associated with multiple tenants.
143. The medical records server system of clause 136, wherein the at least one processor is configured to transmit the integrated patient encounter record to one or more of a medical facility computing device and a health records database via the network interface.
144. The medical records server system of clause 136, wherein the network interface is configured to communicatively couple to the patient charting device via one or more of a computer network and a cellular network.
145. The medical records server system of clause 136, wherein the network interface is configured to communicatively couple to the plurality of medical devices via one or more of a computer network and a cellular network.
146. The medical records server system of clause 145, wherein the system is configured to receive the plurality of medical device case files from the plurality of medical devices via the network interface. 147. The medical records server system of clause 136, wherein the medical records server system is located remotely from the patient charting device and the plurality of medical devices.
148. The medical records server system of clause 136, wherein the at least one processor is configured to:
   transmit, to the patient charting device via the network interface, an identifier of the medical device case file;
   receive, via the network interface from the patient charting device, a confirmation of the medical device case file; and
   store the integrated patient encounter record in response to the received confirmation.
149. The medical records server system of clause 136, wherein the medical device case file is a defibrillator case file.
150. The medical records server system of clause 136, wherein the medical device identifier is one or more of an identifier manually entered into the ePCR, an identifier electronically transmitted to the patient charting device, and an identifier scanned from a bar code or quick response code associated with a medical device.
151. The medical records server system of clause 136, wherein the first case start time falls within a range associated with the second case start time.
152. The medical records server system of clause 151, wherein the at least one processor is configured to calculate the range.
153. The medical records server system of clause 151, wherein the range is predetermined.
154. The medical records server system of clause 151, wherein the range is 0 to 10 minutes.
155. The medical records server system of clause 151, wherein the range comprises a time span between the second case start time from the ePCR and a transfer-of-care time from the ePCR.
156. The medical records server system of clause 136, wherein to identify the medical device case file comprises to identify multiple candidate medical device case files.
157. The medical records server system of clause 156, wherein the at least one processor is configured to:
   transmit, to the patient charting device via the network interface, identifiers of the multiple candidate medical device case files;
   receive, via the network interface from the patient charting device, supplemental selection information comprising a user selection of the medical device case file for the encounter with the particular patient; and
   store the integrated patient encounter record in response to the received confirmation.
158. The medical records server system of clause 157, wherein the identifiers for the multiple candidate medical device case files comprise file association indicators.
159. The medical records server system of clause 157, wherein the identifiers of the multiple candidate medical device case files comprise descriptive information about contents of the multiple candidate medical device case files.

## Claims

1. A patient charting device for creating an integrated medical device and patient care record, the patient charting device comprising:
a user interface;
a communications interface; and
a processor, memory, and associated circuitry communicatively coupled with the user interface and the communications interface, and configured to
receive, via the user interface during a patient encounter, input specifying charting data,
transmit, via the communications interface, an electronic patient care record comprising the charting data to a server,
receive, via the communications interface from the server, at least one identifier of at least one medical device case file associated with the charting data,
render, via the user interface, at least one prompt to confirm that the at least one medical device case file was generated during the patient encounter,
receive, via the user interface, input confirming that the at least one medical device case file was generated during the patient encounter, and
transmit, via the communications interface, a confirmation specifying that the at least one medical device case file was generated during the patient encounter.

2. The patient charting device of claim 1, wherein the processor, memory, and associated circuitry are further configured to render prompts to capture the charting data.

3. The patient charting device of claim 1 or 2, wherein the charting data comprises all or a portion of the patient care record.

4. The patient charting device of any preceding claim, wherein the at least one identifier of the at least one medical device case file comprises a plurality of identifiers of a plurality of medical device case files and the at least one prompt comprises one or more user interface controls configured to receive a selection of a single medical device case file of the plurality of medical device case files to confirm the single medical device case file was generated during the patient encounter.

5. The patient charting device of claim 4, wherein confirmation is configured to enable record integration at the server, the server is configured to store the integrated medical device and patient care record based on the confirmation, and the integrated medical device and patient care record comprises one of:
a) a new file comprising the charting data and the case data.
b) the single medical device case file with imported charting data from the electronic patient care record, or
c) the electronic patient care record with imported case data from the single medical device case file

6. The patient charting device of claim 4, wherein
each identifier of the plurality of identifiers is associated with a file association indicator of a plurality of file association indicators, each file association indicator of the plurality of file association indicators indicating a degree of similarity between parameters associated with the medical device case file identified by the identifier and parameters of the charting data,
the one or more user interface controls comprise a plurality of user interface controls,
each user interface control of the plurality of user interface controls corresponds to an identifier of the plurality of identifiers, and
each user interface control is positioned within the at least one prompt according the file association indicator associated the identifier corresponding to the user interface control.

7. The patient charting device of any preceding claim, wherein the at least one prompt comprises a user interface control configured to receive input refusing to confirm that the at least one medical device case file was generated during the patient encounter.

8. The patient charting device of any preceding claim, the processor, memory, and associated circuitry are further configured to:
receive the at least one medical device case file;
parse the at least one medical device case file to retrieve case data; and
import the case data into the patient care record to generate an integrated patient encounter record.

9. The patient charting device of claim 8, wherein the processor, memory, and associated circuitry are further configured to store the integrated patient encounter record locally and/or transmit the integrated patient encounter record to a multi-tenant cloud server.

10. The patient charting device of any preceding claim, wherein the user interface comprises one or more of a touch screen configured to receive tactile input, a microphone configured to receive audio input, and wearable glasses configured to receive gestural input.

11. The patient charting device of any preceding claim, wherein the patient charting device is a tablet, a medical device, a watch, or another computing device.

12. The patient charting device of any preceding claim, wherein the communications interface supports wired and/or wireless connections.

13. The patient charting device of any preceding claim, wherein the processor, memory, and associated circuitry are further configured to:
receive a medical device identifier scanned from a bar code or QR code and associated with the at least one medical device ;
import the medical device identifier into the patient care record;
transmit the patient care record comprising the medical device identifier to the server; and
receive, from the server, the at least one medical device case file that includes the medical device identifier and was generated by a medical device corresponding to the medical device identifier.

14. The patient charting device of any of claims 1 to 12, wherein said at least one identifier of the at least one medical device case file associated with the charting data comprises an EMS vehicle identification, and an association between particular medical devices and particular EMS vehicles is used by the patient charting device, such as in a look-up table or other reference, to associate the EMS vehicle identification with the medical device case file.

15. The patient charting device of any preceding claim, wherein the at least one medical device case file includes a case start time associated with the electronic patient care record.
